# EUROPEAN PATENT APPLICATION

(11) **EP 4 249 495 A1**
(43) Date of publication of application: **27.09.2023**
(21) Application number: 22163396.9
(22) Date of filing: 21.03.2022
(51) Int. Cl.: C07H 1/06, C07H 19/073, C07H 21/04

(54) **METHOD FOR THE PURIFICATION OF POLYNUCLEOTIDES AND ANALOGUES THEREOF**

(71) Applicant: ATDBio Limited, Hengoed CF81 7TS (GB)
(72) Inventor: Brown, Tom, Southampton (GB); Cox, Owen, Southampton (GB)
(74) Representative: J A Kemp LLP

(57) **Abstract**

Provided herein is a method for modifying a polynucleotide or an analogue or derivative thereof, comprising reacting the polynucleotide or analogue or derivative thereof with a compound of formula (I): 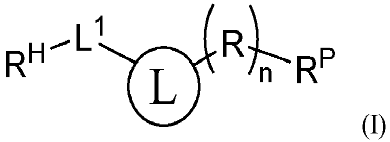 wherein R^{H}, L¹, Ⓛ, R, n and R^{P} are as defined herein. Also provided are associated methods for purifying a polynucleotide or analogue or derivative thereof, uses of a compound of formula (I), and such compounds per se.

## Description

### Field of the invention

The present invention relates to methods of modifying a polynucleotide or an analogue or derivative thereof. The present invention also relates to compounds for use in such methods and to modified polynucleotides thereby produced. The invention also relates to methods of purifying a polynucleotide using the modification methods disclosed herein, and to the use of a compound as defined herein to modify and/or purify a polynucleotide. The present invention also relates to polynucleotides or derivatives or analogues thereof obtainable by such methods.

### Background of the invention

Polynucleotides are biopolymers comprising nucleotide monomer units, and have applications in many different fields, including therapeutics, diagnostics and research. Polynucleotides with customised nucleotide sequences can be synthesised *in vitro,* for example using solution-phase or solid-phase synthetic methods.

Solid-phase chemical synthesis was developed in the 1960s and has a number of advantages over solution-phase synthesis. For example, large excesses of solution-phase reagents can be used to drive reactions quickly to completion; impurities and excess reagents can be washed away, facilitating easy isolation of the growing polynucleotide with no need for purification between reaction steps; and the process is amenable to automation on computer-controlled solid-phase synthesisers. Typically in solid-phase polynucleotide synthesis, a polynucleotide or an initial nucleoside/nucleotide monomer is attached to a solid support and a series of reactions are carried out to increase the chain length of the support-attached polynucleotide/initial monomer, such that synthesis is carried out on the solid support. The solid supports are typically held between filters within synthesis columns. Solution-phase reagents and solvents may pass freely through such columns while the solid support and its attached compounds are held in place. The polynucleotide or an initial nucleoside/nucleotide monomer is typically attached to the solid support using a linker.

Whilst solid-phase chemical synthesis of polynucleotides is well established and commercially used, significant problems remain.

One key problem encountered in conventional synthesis routes is ensuring a sufficiently high purity of the synthesized polymer. There are many sources of potential impurities in the polynucleotide synthesis pathway and it is typically not possible to eliminate these even with careful handling.

For example, one problem that can be encountered is inefficient coupling of nucleotide monomers to the growing monomer chain. The inefficiency of the coupling reaction can increase as the length of the chain increases. To address this capping of unreacted nucleotides at each step of the polymerisation cycle (typically using acetic anhydride in N-methyl imidazole) is common practice; this prevents further reaction. Capping techniques mean that failures early in the polymerisation reaction lead to significant alterations in mass of the final product: short oligonucleotides arising from failure after only a few monomers have been incorporated into the chain can be readily separated from much longer full length oligomers. However, as the chain gets longer it gets harder to separate capped truncated oligomers from the full length desired product. In particular, there may be an increasingly high chance that the terminal residue on a long polynucleotide chain is not the intended residue.

A further problem that can be encountered during polynucleotide synthesis is degradation. Degradation can occur as a result of various mechanisms, including the possible presence of degradating agents (whether chemical reagents or biological agents such as nucleases) in the reaction or product mixture.

A key source of degradation arises from the need to use protected functional groups in conventional synthesis. Such groups include for example DMT (4,4'-dimethoxytrityl). DMT is conventionally used in solid phase polynucleotide synthesis (which typically, although not universally, is approached in a 3'→5' direction) to protect the 5'-OH group of nucleotide monomers as they are added into the growing polynucleotide chain. However, harsh conditions are typically used to remove the DMT group - a typical reagent for detritylation is trichloroacetic acid in dichloromethane. These conditions invariably lead to some degradation of the polynucleotide product.

In attempts to overcome this problem, two basic approaches have been adopted. Both are problematic, however.

One approach involves removal of the DMT protecting group prior to final purification of the synthesized polynucleotide. This in principle means that potential degradation products are removed, but in practice it is very difficult to cleanly purify deprotected polynucleotides, particularly relatively long-chain oligonucleotides or polynucleotides which may have subtle damage. Even when methods such as reversed-phase or anion-exchange HPLC are used (such methods being the best typically available in a commercial setting), final purities are low.

An alternative approach has been termed "DMT-on" purification. In this approach, oligonucleotides or polynucleotides are synthesized with the terminal 5'-DMT group retained for purification. The presence of the 5'-DMT group increases the hydrophobicity of the full-length oligonucleotide relative to all failure sequences (which, as discussed above, are typically capped with acetic anhydride during synthesis and thus do not have a 5'-DMT group), allowing for more efficient purification (e.g. by reversed-phase HPLC). However, whilst the purification can be effective, the subsequent removal of the DMT group, which is required in most applications, can lead to further degradation of the desired product. For example, the acidic conditions used can lead to depurination.

Accordingly, there is a need for improved methods for purifying polynucleotides.

Another challenge in polynucleotide synthesis is effectively controlling the chemistry of the terminal polynucleotide in the synthesized polymer. Many different terminal modifications are needed, according to the application for the polynucleotide. Especially for applications which require extremely high purity products (e.g. therapeutic applications), conventional approaches are inadequate.

For example, some applications require a terminal (e.g. 5') OH group. Whilst such groups are readily achieved by conventional synthesis as discussed above, the available purity levels may be inadequate. Other applications such as gene editing require a terminal phosphate group. Whilst biological reagents such as kinases can be used to convert a terminal OH group to a terminal phosphate, the need for such further reaction steps after completion of the solid-phase synthesis is inefficient and can again lead to purity problems (e.g. if any contaminants are present in the kinase reagent mixture). Still further applications need "non-natural" terminal groups, such as alkyl and alkoxyl groups. For example, a common group used in many technical applications is the "Cₙ spacer" (wherein n is an integer such as 3, 9, 12 or 18), which is a nucleotide analog of structure: 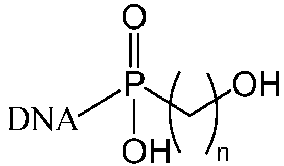 wherein "DNA" represents the remainder of the polynucleotide chain to which the C3 spacer is attached, e.g. via a 5' phosphate.

The need for a wide variety of chemistry at the terminus of a synthesized nucleotide is technically challenging. In particular, there is a need for methods of controlling terminus chemistry (e.g. -OH, -phosphate or -other, e.g. -C3 spacer) which is compatible with extremely high purity synthesis.

The present invention aims to address some or all of these needs.

### Summary of the invention

The inventors have recognised that current methods of solid phase polynucleotide synthesis need improvement. The inventors have found that technical problems such as those outlined above can be addressed by incorporation of a cleavable purification tag into the terminal monomer used in polynucleotide synthesis.

The inventors have appreciated that incorporation of a suitable purification tag is a significant challenge. To achieve sufficient purity e.g. by HPLC, tags incorporated need to be robustly attached to the polynucleotide, and typically need to be fairly large in order to have sufficient impact on the physical properties of the modified polynucleotide that separation of modified polynucleotides from unmodified polynucleotides can be effectively achieved.

Even if purification tags can be incorporated, however, problems remain. Such tags typically cannot be left on the purified polynucleotide without interfering with downstream applications, which is usually commercially and technically unacceptable. However, the removal of such tags by chemical or enzymatic means is also problematic as this process typically leads to degradation of the purified polynucleotide as outlined above. Accordingly, the problem of achieving satisfactory purification of controllably modified polynucleotides on a commercially-relevant scale has until now remained unsolved.

In the course of extensive research, the present inventors have recognised that certain photocleavable compounds can be used to modify polynucleotides. The inventors have designed compounds which allow a polynucleotide to be efficiently and effectively modified so as to both control the terminal chemistry of the polynucleotide and to also reversibly incorporate a hydrophobic group which can be used, for example, to improve purification by techniques such as HPLC. Whilst the invention has been made in the context of purification of polynucleotides, there are many other applications also as the modifications that can be made to polynucleotides in accordance with the invention are not limited.

Accordingly, as explained in more detail below, the invention provides a method for modifying a polynucleotide or an analogue or derivative thereof, comprising reacting the polynucleotide or analogue or derivative thereof with a compound of formula (I): 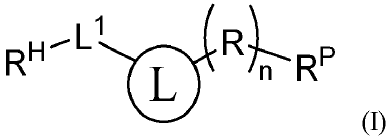 wherein:
- R^{H} is a hydrophobic group;
- L¹ is a linking group;
- Ⓛ is a photolabile group;
- R is a modifying group;
- n is an integer selected from 0 and 1; and
- R^{P} is a phosphorous-based group;
under conditions such that a reactive functional group of the polynucleotide or analogue or derivative thereof reacts with the phosphorous-based group R^{P}, thereby connecting the hydrophobic group R^{H} to the polynucleotide or analogue or derivative thereof.

In some embodiments, the method comprises reacting a free hydroxyl group at the 5' position of the polynucleotide or analogue or derivative thereof with the phosphorous-based group R^{P}. In some embodiments, the method comprises reacting a free hydroxyl group at the 3' position of the polynucleotide or analogue or derivative thereof with the phosphorous-based group R^{P}.

In some embodiments, R^{H} is selected from C₁ to C₂₀ alkyl, C₂ to C₂₀ alkenyl, C₂ to C₂₀ alkynyl, C₅ to C₁₀ carbocyclyl, C₆ to C₁₈ aryl, 5- to 10-membered heteroaryl and 5- to 10-membered heterocyclyl, wherein R^{H} is optionally substituted. In some embodiments, R^{H} is selected from C₄ to C₁₆ alkyl, C₄ to C₁₆ alkenyl, C₄ to C₁₆ alkynyl, C₅ to C₁₀ carbocyclyl and C₆ to C₁₀ aryl, preferably C₅ to C₁₂ alkyl; wherein R^{H} is optionally substituted and is preferably unsubstituted.

In some embodiments, L¹ is selected from: (i) a chemical bond; and (ii) a linker comprising a C₁ to C₂₀ alkylene group, a C₂ to C₂₀ alkenylene group and/or a C₂ to C₂₀ alkynylene group, wherein said alkylene, alkenylene or alkynylene group is optionally interrupted by and/or terminated in one or more groups selected from a heteroatom, a phosphite group, a phosphate group, a carbonyl group, a C₆ to C₁₀ aryl group, a C₅ to C₁₀ carbocyclyl group, a 5- to 10-membered heteroaryl group and a 5- to 10-membered saturated or partially unsaturated heterocyclic group, wherein the linker is optionally further substituted. In some embodiments, L¹ is a linker comprising a C₁ to C₆ alkylene group, a C₂ to C₆ alkenylene group and/or a C₂ to C₆ alkynylene group, wherein said alkylene, alkenylene or alkynylene group is optionally interrupted by and/or terminated in one or more groups selected from a heteroatom, a carbonyl group, a phenyl group, a cyclopentyl or cyclohexyl group, a 5- to 6-membered heteroaryl group and a 5- to 6-membered saturated or partially unsaturated heterocyclic group, wherein the linker is optionally further substituted.

In some embodiments, R^{P} is a phosphoramidite, a phosphoramidate, an alkyl phosphonamidite or an alkyl phosphonamidate. In some embodiments, R^{P} is a phosphoramidite or an alkyl phosphonamidite, preferably a phosphoramidite or a methyl phosphonamidite, more preferably 2-cyanoethyl N,N-diisopropyl phosphoramidite or N,N-diisopropyl methylphosphonamidite.

In some embodiments, R is selected from:
(i) a nucleoside or a derivative or analogue thereof;
(ii) a group comprising a C₁ to C₂₀ alkylene group, a C₂ to C₂₀ alkenylene group and/or a C₂ to C₂₀ alkynylene group, wherein said alkylene, alkenylene or alkynylene group is optionally interrupted by and/or terminated in one or more groups selected from: a heteroatom; a phosphite group; a phosphate group; a carbonyl group; a C₆ to Cio aryl group; a C₅ to C₁₀ carbocyclyl group; a 5- to 10-membered heteroaryl group; and a 5- to 10-membered heterocyclic group; and wherein R is optionally further substituted;
   preferably a group comprising a C₂ to C₆ alkylene group, a C₂ to C₆ alkenylene group and/or a C₂ to C₆ alkynylene group, wherein said alkylene, alkenylene or alkynylene group is optionally interrupted by and/or terminated in one or more groups selected from a heteroatom, a carbonyl group, a phenyl group, a cyclopentyl or cyclohexyl group, a 5- to 6-membered heteroaryl group and a 5- to 6-membered saturated or partially unsaturated heterocyclic group, wherein R is optionally further substituted;
   and
(iii) a second polynucleotide or a derivative or analogue thereof.

In some embodiments, the photolabile group Ⓛ is a moiety of formula -Ⓐ-CHR³-W-, such that the compound of formula (I) is a compound of formula (II): 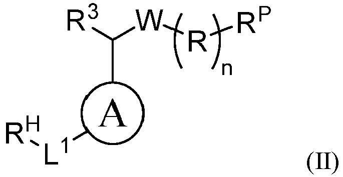 wherein R^{H}, L¹, and R are as defined herein, and wherein:
- Ⓐ is a 2-nitrobenzyl group, wherein said 2-nitrobenzyl group is optionally substituted with 1, 2 or 3 groups independently selected from halogen, optionally substituted C₁ to C₄ alkyl, -OR^{a}, -SR^{a}, -NR^{a}R^{a}, -C(O)OR^{a}, - C(O)NR^{a}R^{a}, -C(O)R^{b}, -OC(O)R^{b} and -NHC(O)R^{b}, wherein each R^{a} is independently selected from hydrogen, optionally substituted C₁ to C₂ alkyl and optionally substituted C₁ to C₂ alkoxyl and each R^{b} is independently selected from hydrogen and an optionally substituted C₁ to C₄ alkyl group;
- R³ is methyl, ethyl or C₁ to C₂ haloalkyl; and
- W is selected from a group of formula (W-2); an oxygen atom; and a group of formula (W-1): 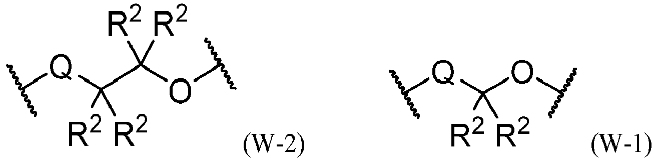 wherein:
- when n is 1, R^{P} is a phosphoramidite, a phosphoramidate, an alkyl phosphonamidite or an alkyl phosphonamidate; and when n is 0 R^{P} together with the oxygen atom to which it is attached forms a phosphoramidite, a phosphoramidate, an alkyl phosphonamidite or an alkyl phosphonamidate;
   wherein said phosphoramidite, a phosphoramidate, an alkyl phosphonamidite or an alkyl phosphonamidate is preferably a phosphoramidite or a methyl phosphonamidite, more preferably 2-cyanoethyl N,N-diisopropyl phosphoramidite or N,N-diisopropyl methylphosphonamidite;R^{P},
- Q is an oxygen atom or a sulphur atom; and
- each R² is independently selected from hydrogen, methyl, ethyl, C₁ to C₂ haloalkyl and a halogen group.

In some embodiments, W is a group of formula (W-2) and n is 0, such that the compound of formula (II) is a compound of formula (II-3): 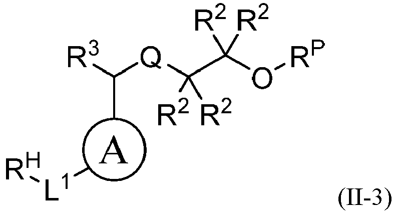 wherein R^{H}, L¹, Ⓐ, R², R³ and Q are as defined herein and R^{P}, together with the oxygen atom to which it is attached, forms an alkyl phosphonamidite, preferably a methyl phosphonamidite, and more preferably N,N-diisopropyl methylphosphonamidite.

In some embodiments, W is an oxygen atom and n is 0, such that the compound of formula (II) is a compound of formula (II-1): 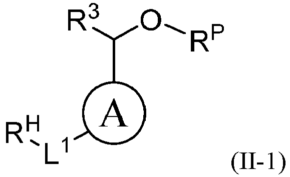 wherein R^{H}, L¹, Ⓐ, and R³ are as defined herein, and wherein R^{P} together with the oxygen atom to which it is attached forms a phosphoramidite, a phosphoramidate, an alkyl phosphonamidite or an alkyl phosphonamidate; preferably a phosphoramidite, more preferably 2-cyanoethyl N,N-diisopropyl phosphoramidite.

In some embodiments, W is a group of formula (W-1) and n is 1, such that the compound of formula (II) is a compound of formula (II-2): 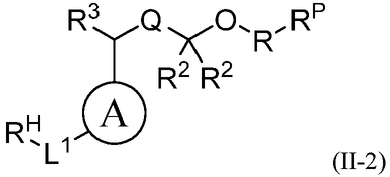 wherein R^{H}, L¹, Ⓐ, R², R³, Q, and R are as defined herein, and wherein R^{P} is a phosphoramidite, a phosphoramidate, an alkyl phosphonamidite or an alkyl phosphonamidate; preferably a phosphoramidite, more preferably 2-cyanoethyl N,N-diisopropyl phosphoramidite.

In some embodiments, Ⓐ, L¹ and R^{H} are together represented by formula (A-1) or formula (A-2): 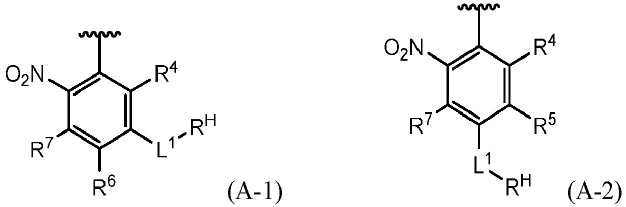 wherein L¹ and R^{H} are as defined herein, and wherein:
- R⁴, R⁵, R⁶ and R⁷ are each independently selected from hydrogen, halogen, optionally substituted C₁ to C₄ alkyl, -OR^{a}, -SR^{a}, -NR^{a}R^{a}, -C(O)OR^{a}, -C(O)NR^{a}R^{a}, -C(O)R^{b}, -OC(O)R^{b} and -NHC(O)R^{b}, preferably R⁴, R⁵, R⁶ and R⁷ are each independently selected from hydrogen, fluorine, optionally substituted C₁ to C₄ alkyl, -OR^{a}, -SR^{a}, -NR^{a}R^{a}, -OC(O)R^{b} and -NHC(O)R^{b}, more preferably R⁴, R⁵, R⁶ and R⁷ are each hydrogen or methoxy; wherein R^{a} and R^{b} are as defined herein.

Also provided is a method for purifying a polynucleotide or an analogue or derivative thereof, comprising:
(i) modifying a polynucleotide or analogue or derivative thereof in a reaction mixture as defined herein, thereby increasing the hydrophobicity of the polynucleotide or analogue or derivative thereof;
(ii) separating the modified polynucleotide or analogue or derivative thereof from other components in the reaction mixture according to the hydrophobicity of the modified polynucleotide or analogue or derivative thereof; and
(iii) optionally removing the hydrophobic group from the modified polynucleotide or analogue or derivative thereof by photo-illuminating the modified polynucleotide, analogue or derivative thereof, preferably by photo-illuminating the modified polynucleotide, analogue or derivative thereof using UV light at a wavelength of about 300 to about 500 nm.

In some embodiments, step (ii) comprises separating the modified polynucleotide or analogue or derivative thereof from other components in the reaction mixture using a chromatographic purification technique, preferably using high performance liquid chromatography, more preferably using reverse-phase high performance liquid chromatography.

Further provided is a compound of formula (I): 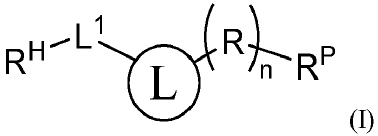 wherein R^{H}, L¹, Ⓛ, R, n and R^{P} are as defined herein.

Also provided is a modified polynucleotide or a derivative or analogue thereof, comprising a group of formula (I^{∗}): 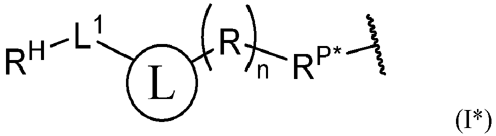 wherein R^{H}, L¹, Ⓛ, R and n are as defined herein, and wherein:
- R^{P∗} is a phosphorous-based linkage; and
- the wavy line indicates the point of attachment to the polynucleotide or derivative or analogue thereof.

In some embodiments, R^{P∗} is a phosphodiester linkage, a phosphotriester linkage, a phosphite-triester linkage, a phosphite-diester linkage, a phosphorothioate linkage, a phosphorodithioate linkage, an alkylphosphonate linkage, or an alkylphosphonite linkage, preferably phosphite-triester linkage or an alkylphosphonite linkage, and more preferably a phosphite-triester linkage or a methylphosphonite linkage; and/or the group of formula (I^{∗}) is attached at the 3' position or the 5' position of the polynucleotide or analogue or derivative thereof, preferably at the 5' position of the polynucleotide or analogue or derivative thereof.

Still further provided is the use of a compound of formula (I) as defined herein, for:
(i) modifying a polynucleotide or a derivative or analogue thereof, optionally thereby increasing the hydrophobicity of the polynucleotide or a derivative or analogue thereof; or
(ii) purifying a polynucleotide or a derivative or analogue thereof.

Further provided is a polynucleotide or a derivative or analogue thereof, obtainable by a method as defined herein.

### Brief description of the Figures

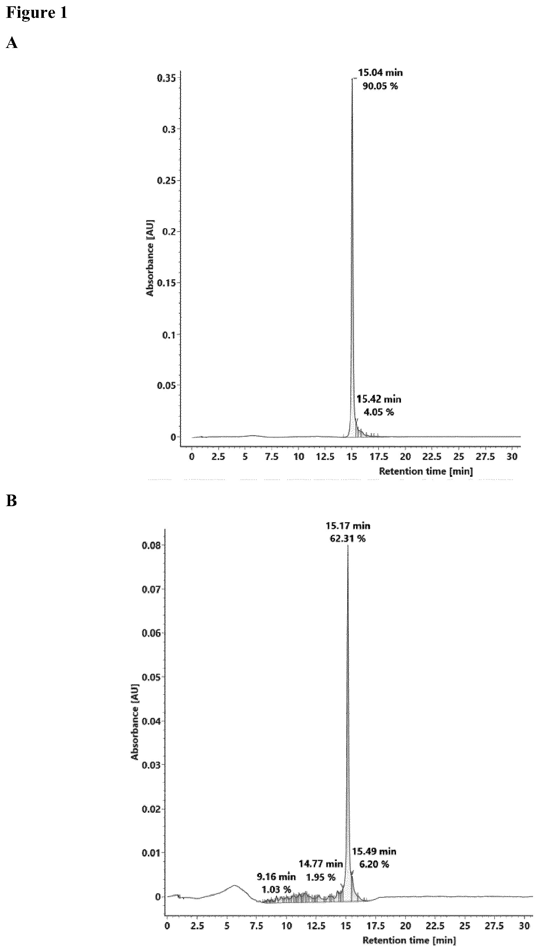

**Figure 1****:** Comparison of oligonucleotide purity by UPLC-MS following purification with RP-HPLC. **A:** Oligonucleotide synthesised with the terminal monomer unit consisting of the compound of example 2 (5'-PC C3). **B:** Oligonucleotide synthesized the terminal monomer unit consisting of a non-photocleavable C3 phosphoramidite. The figures show that significantly improved purity was obtained through use of the compound of example 2 as compared to the control. Results are described in the examples.

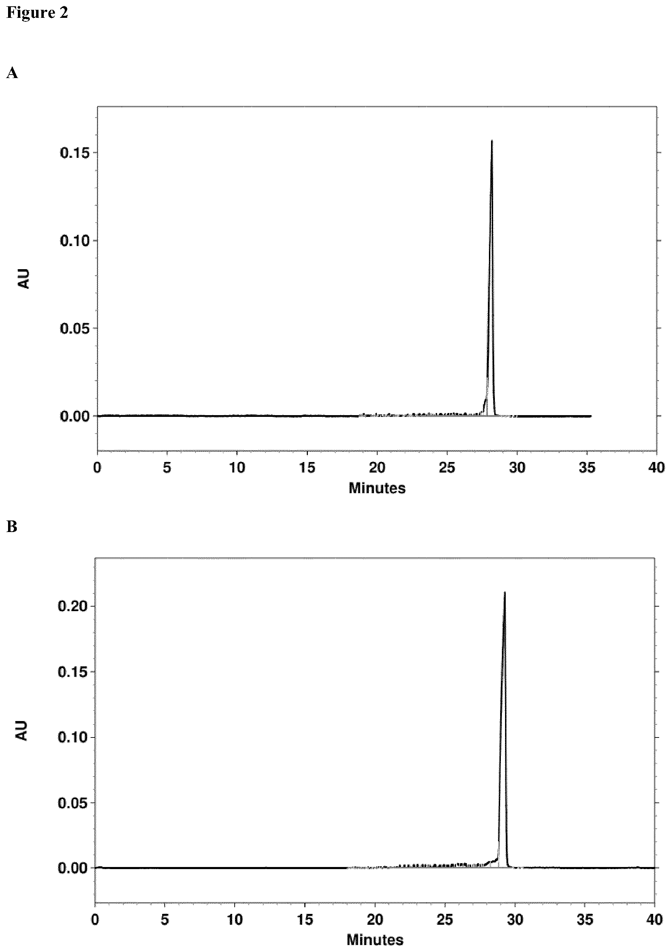

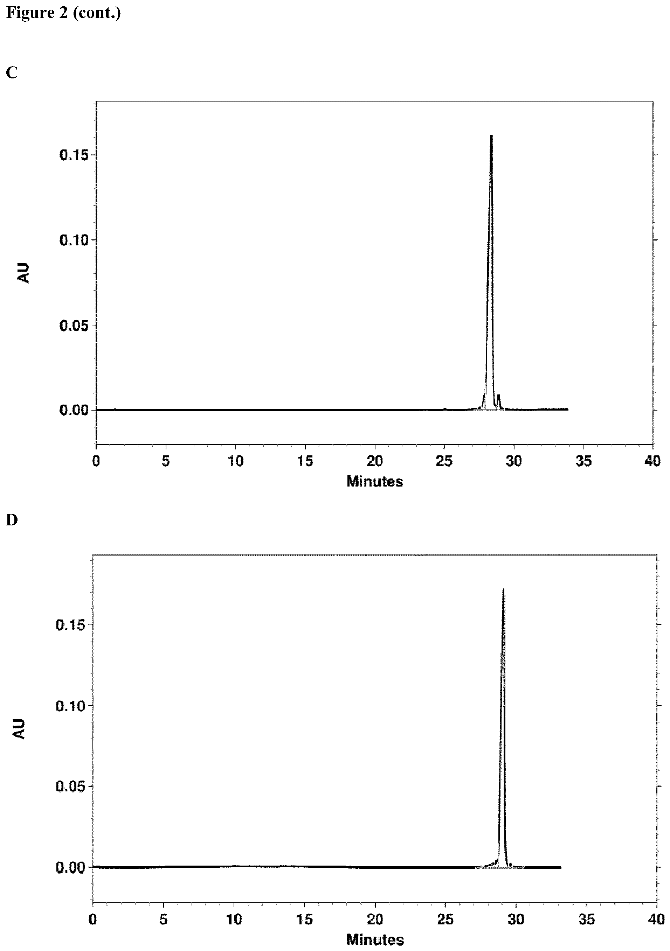

**Figure 2****:** Comparison of oligonucleotides purity following purification. **A:** Oligonucleotide of SEQ ID NO: 1. **B:** Oligonucleotide of SEQ ID NO: 2.
**C:** Oligonucleotide of SEQ ID NO: 2, corresponding to SEQ ID NO: 1 with terminal monomer unit consisting of the compound of example 1 (5'-PC PO₃). **D:** Oligonucleotide of SEQ ID NO: 4, corresponding to SEQ ID NO: 2 with terminal monomer unit consisting of the compound of example 1 (5'-PC PO₃). The figures show that significantly improved purity was observed for SEQ ID NO: 3 vs SEQ ID NO: 1; and SEQ ID NO: 4 vs SEQ ID NO: 2. The figures thus demonstrate that significantly improved purity was obtained through use of the compound of example 1 as compared to the control. Results are described in the examples.

### Detailed Description of the Invention

### Definitions

The term "nucleosides, nucleotides, polynucleotides and derivatives and analogues thereof' includes: nucleosides; derivatives of nucleosides; analogues of nucleosides; nucleotides; derivatives of nucleotides; analogues of nucleotides; polynucleotides; derivatives of polynucleotides; and analogues of polynucleotides.

As used herein, "derivatives" of nucleosides, nucleotides or polynucleotides are modified forms of nucleosides, nucleotides or polynucleotides which comprise one or more chemical modifications to the phosphate backbone, sugar ring or nucleobase of the DNA or RNA. A derivative of a nucleoside thus may be a chemically modified nucleoside as described in more detail herein.

As used herein "analogs" of nucleosides, nucleotides or polynucleotides are compounds which are structurally similar to nucleotides such as DNA and RNA but which include modifications at one or more positions such as at the phosphate backbone, sugar ring or nucleobase of the DNA or RNA. Nucleotide analogs include peptide nucleic acid (PNA), glycerol nucleic acid (GNA), threose nucleic acid (TNA), locked nucleic acid (LNA) and other synthetic polymers with nucleotide side chains.

Unless otherwise specified, the term "nucleoside" includes protected nucleosides - i.e. nucleosides that have been modified to include protecting groups in their chemical structure. Unless otherwise specified, the term "nucleotide" includes protected nucleotides - i.e. nucleotides that have been modified to include protecting groups in their chemical structure. Unless otherwise specified, the term "polynucleotide" includes protected polynucleotides - i.e. polynucleotides that have been modified to include protecting groups in their chemical structure. Such protecting groups are typically distinct from modifications that may be made in nucleotide derivatives for functional reasons e.g. to alter the properties of the nucleotide or polynucleotide.

As used herein and unless otherwise specified, the term "protecting group" refers to any suitable protecting group known in the field. Different protecting groups are suitable for protecting different chemical groups. For example, as used herein and unless otherwise specified, the term "hydroxyl protecting group" refers to any protecting group suitable for protecting hydroxyl groups that is known in the field. Similarly, as used herein and unless otherwise specified, the term "amine protecting group" refers to any protecting group suitable for protecting amine groups that is known in the field. Furthermore, as used herein and unless otherwise specified, the terms "phosphate protecting group" and "phosphite protecting group" refer to any protecting group suitable for protecting a phosphate or phosphite group, respectively, that is known in the field.

Suitable hydroxyl protecting groups include acetyl (Ac), benzoyl (Bz), benzyl (Bn), β-methoxyethoxymethyl ether (MEM), 4,4'-dimethoxytrityl (DMT), methoxymethyl ether (MOM), 4-monomethoxytrityl (MMT), levulinyl, 9-fluorenylmethyloxycarbonyl (Fmoc) *p*-methoxybenzyl ether (PMB), *p*-methoxyphenyl ether (PMP), methylthiomethyl ether, pivaloyl (Piv), tetrahydropyranyl (THP), tetrahydrofuran (THF), trityl (Tr), silyl ethers such as trimethylsilyl (TMS), *tert*-butyldimethylsilyl (TBDMS), tri-*iso-*propylsilyloxymethyl (TOM) and triisopropylsilyl (TIPS), methyl ether, ethoxyethyl ether, allyl ether and tert-butyl ether.

Suitable amine protecting groups include carbobenzyloxy (Cbz), *p*-methoxybenzyl carbonyl (Moz), *tert*-butyloxycarbonyl (BOC), 9-fluorenylmethyloxycarbonyl (Fmoc), acetyl (Ac), trifluoroacetyl, benzoyl (Bz), benzyl (Bn), benzylidene, trityl (Tr), carbamate, phthalimide, *p*-methoxybenzyl (PMB), 3,4-dimethoxybenzyl (DMPM), p-methoxyphenyl (PMP), trichloroethyl chloroformate (Troc), tosyl (Ts), nosyl and 2-nitrophenylsulfenyl (Nps).

Suitable phosphate protecting groups and phosphite protecting groups include: alkyl groups, such as C₁ to C₄ alkyl groups optionally substituted with 1, 2 or 3 groups independently selected from halogen, -OR^{a}, -SR^{a}, -NR^{a}R^{a}, -C(O)OR^{a}, -C(O)NR^{a}R^{a}, -C(O)R^{b}, -OC(O)R^{b}, -NHC(O)R^{b} and -CN, and in particular methyl, ethyl and 2-cyanoethyl, preferably 2-cyanoethyl; o-chlorophenyl; and p-chlorophenyl.

As used herein, an alkyl group can be linear, branched or cyclic but is preferably linear. A C₁ to C₂₀ alkyl group is a linear or branched alkyl group containing from 1 to 20 carbon atoms. A C₁ to C₂₀ alkyl group is typically a C₁ to C₁₀ alkyl group such as a C₁ to C₆ alkyl group, a C₁ to C₅ alkyl group or a C₁ to C₄ alkyl group. Suitable C₁ to C₄ alkyl groups include methyl, ethyl, n-propyl, i-propyl, n-butyl, sec-butyl and tert-butyl. A C₁ to C₄ alkyl group is preferably a C₁ to C₃ alkyl group, more preferably ethyl or methyl. Unless otherwise specified, an alkyl group can be unsubstituted or substituted as described herein. For the avoidance of doubt, where two alkyl groups are present, the alkyl groups may be the same or different.

As used herein, an alkenyl group can be linear, branched or cyclic but is preferably linear. An alkenyl group has one or more, e.g. one or two, typically one double bonds. A C₂ to C₂₀ alkenyl group is a linear or branched alkenyl group containing from 2 to 20 carbon atoms. A C₂ to C₂₀ alkenyl group is typically a C₂ to C₁₀ alkenyl group such as a C₂ to C₆ alkenyl group, a C₂ to C₅ alkenyl group or a C₂ to C₄ alkenyl group. A C₂ to C₄ alkenyl group is preferably a C₂ to C₃ alkenyl group. Examples of C₂-C₄ alkenyl groups include ethenyl, propenyl and butenyl. Unless otherwise specified, an alkenyl group can be unsubstituted or substituted as described herein. For the avoidance of doubt, where two alkenyl groups are present, the alkenyl groups may be the same or different.

As used herein, an alkynyl group can be linear, branched or cyclic but is preferably linear. An alkenyl group has one or more, e.g. one or two, typically one triple bonds. A C₂ to C₂₀ alkynyl group is a linear or branched alkynyl group containing from 2 to 20 carbon atoms. A C₂ to C₂₀ alkynyl group is typically a C₂ to C₁₀ alkynyl group such as a C₂ to C₆ alkynyl group, a C₂ to C₅ alkynyl group or a C₂ to C₄ alkynyl group. A C₂ to C₄ alkynyl group is preferably a C₂ to C₃ alkynyl group. Examples of C₂ to C₄ alkynyl groups include ethynyl, propynyl and butynyl. Unless otherwise specified, an alkynyl group can be unsubstituted or substituted as described herein. For the avoidance of doubt, where two alkynyl groups are present, the alkynyl groups may be the same or different.

As used herein, an alkylene group is a an unsubstituted or substituted bidentate moiety obtained by removing two hydrogen atoms from an alkane. The two hydrogen atoms may be removed from the same carbon atom or from different carbon atoms. As used herein, an alkylene group can be linear, branched or cyclic but is preferably linear. A C₁ to C₂₀ alkylene group is a linear or branched alkylene group containing from 1 to 20 carbon atoms. A C₁ to C₂₀ alkylene group is typically a C₁ to C₁₀ alkylene group such as a C₁ to C₆ alkylene group, a C₁ to C₅ alkylene group or a C₁ to C₄ alkylene group. Suitable C₁ to C₄ alkylene groups include methylene, ethylene, n-propylene, i-propylene, n-butylene, sec-butylene and tert-butylene. A C₁ to C₄ alkylene group is preferably a C₁ to C₃ alkylene group, more preferably ethylene or methylene. Unless otherwise specified, an alkylene group can be unsubstituted or substituted as described herein. For the avoidance of doubt, where two alkylene groups are present, the alkylene groups may be the same or different.

As used herein, an alkenylene group is a an unsubstituted or substituted bidentate moiety obtained by removing two hydrogen atoms from an alkene. The two hydrogen atoms may be removed from the same carbon atom or from different carbon atoms. As used herein, an alkenylene group can be linear, branched or cyclic but is preferably linear. An alkenylene group has one or more, e.g. one or two, typically one double bonds. A C₂ to C₂₀ alkenylene group is a linear or branched alkenylene group containing from 2 to 20 carbon atoms. A C₂ to C₂₀ alkenylene group is typically a C₂ to C₁₀ alkenylene group such as a C₂ to C₆ alkenylene group, a C₂ to C₅ alkenylene group or a C₂ to C₄ alkenylene group. A C₂ to C₄ alkenylene group is preferably a C₂ to C₃ alkenylene group. Examples of C₂-C₄ alkenylene groups include ethenylene, propenylene and butenylene. Unless otherwise specified, an alkenylene group can be unsubstituted or substituted as described herein. For the avoidance of doubt, where two alkenylene groups are present, the alkenylene groups may be the same or different.

As used herein, an alkynylene group is a an unsubstituted or substituted bidentate moiety obtained by removing two hydrogen atoms from an alkyne. As used herein, an alkynylene group can be linear, branched or cyclic but is preferably linear. An alkynylene group has one or more, e.g. one or two, typically one triple bonds. A C₂ to C₂₀ alkynylene group is a linear or branched alkynylene group containing from 2 to 20 carbon atoms. A C₂ to C₂₀ alkynylene group is typically a C₂ to C₁₀ alkynylene group such as a C₂ to C₆ alkynylene group, a C₂ to C₅ alkynylene group or a C₂ to C₄ alkynylene group. A C₂ to C₄ alkynylene group is preferably a C₂ to C₃ alkynylene group. Examples of C₂-C₄ alkynylene groups include ethynylene, propynylene and butynylene. Unless otherwise specified, an alkynylene group can be unsubstituted or substituted as described herein. For the avoidance of doubt, where two alkynylene groups are present, the alkynylene groups may be the same or different.

As used herein, a halogen is typically chlorine, fluorine, bromine or iodine, and is preferably chlorine, fluorine or bromine, more preferably chlorine or fluorine.

As used herein, a C₆ to C₁₀ aryl group is an aryl group having from 6 to 10 carbon atoms, for example, phenyl or naphthyl, preferably phenyl. An aryl group or moiety can be substituted or unsubstituted as described herein.

As used herein, a C₅ to C₁₀ carbocyclyl group can be a C₅, C₆, C₇, C₈, C₉ or Cio cycloalkyl group and is preferably cyclopentyl or cyclohexyl. Typically a cycloalkyl group is substituted or unsubstituted with up to three substituents, e.g. one or two substituents.

As used herein, a 5- to 10-membered heteroaryl group is a 5- to 10-membered ring system in which the ring is fully unsaturated and aromatic and contains at least one heteroatom. Typically, the ring contains up to three or four heteroatoms, e.g. one or two heteroatoms, selected from O, N and S. Thus, a 5- to 10-membered heteroaryl group is typically a 5- to 10-membered aromatic ring containing one, two or three heteroatoms selected from O, N and S. Preferably, the heteroatoms are selected from O and N. Suitable such heteroaryl groups include, for example:
monocyclic 5- to 7-membered heteroaryl rings such as furanyl, oxepinyl, pyrrolyl, pyrazolyl, imidazolyl, triazolyl, tetrazolyl, oxazolyl, isoxazolyl, oxadiazolyl, thiazolyl, isothiazolyl, thiadiazolyl, pyridinyl, pyradazinyl, pyrimidinyl, pyrazinyl, triazinyl, azepinyl, thiophenyl, oxepinyl and thiepinyl; and
bicyclic 8- to 10-membered heteroaryl rings such as benzofuranyl, indolyl, isoindolyl, indolizinyl, indazolyl, benzimidazolyl, azaindolyl, azaindazolyl, purinyl, benzooxazolyl, benzoisooxazolyl, benzothiazolyl, benzoisothiazolyl, benzothiadiazolyl, quinolinyl, isoquinolinyl, quinolizinyl, quinoxalinyl, phthalazinyl, quinazolinyl, cinnolinyl, naphthyridinyl, pteridinyl and benzothiophenyl, preferably benzofuranyl, indolyl, isoindolyl, quinolinyl and isoquinolinyl.

Preferably, the 5- to 10-membered heteroaryl group is a monocyclic 5- to 7-membered heteroaryl ring selected from furanyl, pyrrolyl, pyrazolyl, imidazolyl, triazolyl, oxazolyl, isoxazolyl, pyridinyl, pyradazinyl, pyrimidinyl and pyrazinyl.

As used herein, a 5- to 10-membered saturated heterocyclic group is a saturated 5-to 10-membered ring system in which the ring contains at least one heteroatom. Typically, the ring contains up to three or four heteroatoms, e.g. one or two heteroatoms, selected from O, S and N. Thus, a 5- to 10-membered saturated heterocyclic group is typically a 5-to 10-membered ring containing one, two or three heteroatoms selected from O, S and N. Suitable such saturated heterocyclic groups include, for example, monocyclic saturated 5-to 8-membered rings, more preferably 5- to 7-membered rings, such as tetrahydrofuranyl, piperidinyl, oxazolidinyl, morpholinyl, thiomorpholinyl, pyrrolidinyl, dioxolanyl, piperidonyl, azepanyl, oxepanyl, piperazinyl, tetrahydropyranyl and 1,4-diazepanyl, more preferably pyrrolidinyl, morpholinyl, piperazinyl, tetrahydropyranyl, piperidinyl, azepanyl and 1,4-diazepanyl.

As used herein, a 5- to 10-membered partially unsaturated heterocyclic group is a 5- to 10-membered ring system in which the ring contains at least one unsaturated bond and at least one heteroatom, but the ring is not fully unsaturated or aromatic. Typically, the ring contains up to three or four heteroatoms, e.g. one or two heteroatoms, selected from O, N and S. Thus, a 5- to 10-membered partially unsaturated heterocyclic group is typically a 5- to 10-membered ring containing one, two or three heteroatoms selected from O, N and S. Preferably, the heteroatoms are selected from O and N. Suitable such partially unsaturated heterocyclic groups include, for example:
monocyclic partially unsaturated 5- to 7-membered heterocyclic rings such as dihydrofuranyl, pyranyl, dihydropyranyl, dioxinyl, dihydrooxepinyl, tetrahydrooxepinyl, pyrrolinyl, pyrazolinyl, imidazolinyl, dihydrooxazolyl, dihydroisoxazolyl, dihydrothiazolyl, dihydroisothiazolyl, dihydropyridinyl, tetrahydropyridinyl, dihydropyridazinyl, tetrahydropyridazinyl, dihydropyrimidinyl, tetrahydropyrimidinyl, dihydropyrazinyl, tetrahydropyrazinyl, oxazinyl, dihydrooxazinyl, thiazinyl, dihydrothiazinyl, dihydroazepinyl, tetrahydroazepinyl, dihydrothiophenyl, thiopyranyl, dihydrothiopyranyl, dihydrothiepinyl, and tetrahydrothiepinyl; and
bicyclic partially unsaturated 8- to 10-membered heterocyclic rings such as dihydrobenzofuranyl, dihydroisobenzofuranyl, benzopyranyl, dihydrobenzopyranyl, benzodioxolyl, indolinyl, isoindolinyl, dihydroquinolinyl, tetrahydroquinolinyl, benzooxazinyl, dihydrobenzothiophenyl and benzodithiole; preferably dihydrobenzofuranyl, benzopyranyl, dihydrobenzopyranyl, benzodioxolyl, indolinyl, isoindolinyl, dihydroquinolinyl and tetrahydroquinolinyl.

Preferably, the 5- to 10-membered partially unsaturated heterocyclic group is a monocyclic partially unsaturated 5- to 7-membered ring selected from dihydrofuranyl, pyranyl, pyrrolinyl and oxazinyl.

A heterocyclic and/or heteroaryl group may be substituted or unsubstituted. Each ring atom may be unsubstituted or may carry one or two substituents. A nitrogen atom may be disubstituted, providing a positively charged heteroatom. A sulphur atom may be substituted, providing a positively charged heteroatom. Typically, a heterocyclic or heteroaryl group has up to three substituents, e.g. one or two substituents. The heterocyclic or heteroaryl ring may be connected to the remainder of the molecule by a bond to any of the available ring positions on the heterocyclic or heteroaryl ring.

As used herein, a group which is optionally substituted may unless otherwise specified be substituted with suitable substituents which may include a halogen such as chlorine and/or fluorine, a cyano group, -OR^{x}, -SR^{x}, -NR^{x}R^{x}, -C(O)OR^{x}, -C(O)NR^{x}R^{x}, - C(O)R^{x} and a C₁ to C₄ alkyl group such as methyl and/or ethyl, wherein a C₁ to C₄ alkyl substituent is itself either unsubstituted or substituted with 1 to 3 halogen atoms. Each R^{x} is independently selected from hydrogen and a C₁ to C₄ alkyl group which is unsubstituted or is substituted with 1, 2 or 3 halogen groups. An optional substituent is preferably a hydroxyl group, a halogen such as chlorine or fluorine, or a C₁ to C₄ alkyl group such as methyl or ethyl.

Unless otherwise specified, included in the above are the well known ionic, salt, solvate, and protected forms of these substituents. For example, a reference to carboxylic acid, carboxy or carboxyl group (-COOH) also includes the anionic (carboxylate) form (-COO⁻), a salt or solvate thereof, as well as conventional protected forms. Similarly, a reference to an amino group includes the protonated form (-N⁺HR¹R²), a salt or solvate of the amino group, for example, a hydrochloride salt, as well as conventional protected forms of an amino group. Similarly, a reference to a hydroxy or hydroxyl group (-OH) also includes the anionic form (-O⁻), a salt or solvate thereof, as well as conventional protected forms. Salts include salts formed with acids and bases. Suitable acids include both inorganic acids such as hydrochloric, sulphuric, phosphoric, diphosphoric, hydrobromic or nitric acid and organic acids such as oxalic, citric, fumaric, maleic, malic, ascorbic, succinic, tartaric, benzoic, acetic, methanesulphonic, ethanesulphonic, benzenesulphonic or *p*-toluenesulphonic acid. Suitable bases include alkali metal (e.g. sodium or potassium) and alkali earth metal (e.g. calcium or magnesium) hydroxides and organic bases such as alkyl amines, aralkyl amines and heterocyclic amines. Hydrochloride salts and acetate salts are preferred, in particular hydrochloride salts. When intended for use in pharmaceutical applications, salts of the compounds provided herein may be preferably pharmaceutically acceptable salts.

As used herein, a phosphoramidite group is a group of form -OP(NR₂)(OR), wherein each R group, which may be the same or different, is typically an optionally substituted hydrocarbyl group. Typically the R group of the OR moiety is a hydrogen, a lone pair of electrons resulting in a negative charge, a phosphate protecting group or a phosphite protecting group. More often the R group of the OR moiety is a hydrogen, a lone pair of electrons resulting in a negative charge, o-chlorophenyl, p-chlorophenyl or a C₁ to C₄ alkyl group optionally substituted with 1, 2 or 3 groups independently selected from halogen, -OR^{a}, -SR^{a}, -NR^{a}R^{a}, -C(O)OR^{a}, -C(O)NR^{a}R^{a}, -C(O)R^{b}, -OC(O)R^{b}, - NHC(O)R^{b} and -CN wherein R^{a} and R^{b} are independently H or methyl. More preferably, the R group of the OR moiety is a hydrogen, a lone pair of electrons resulting in a negative charge, o-chlorophenyl, p-chlorophenyl, a methyl group or a 2-cyanoethyl group. Each R group may alternatively be an optionally substituted C₁₋₄ alkyl, C₂₋₄ alkenyl, or C₂₋₄ alkynyl group, typically an optionally substituted C₁₋₃ alkyl group such as methyl, ethyl or isopropyl, which group may be optionally substituted by e.g. CN. Often a phosphoramidite group as used herein is an OCEP group of form - OP(NⁱPr₂)(OCH₂CH₂CN).

As used herein, a phosphoramidate group is an oxidised phosphoramidite of form - OP(O)(NR₂)(OR).

As used herein, a phosphonamidite group is a group of form -OP(NR₂)(R), wherein each R group, which may be the same or different, is an optionally substituted hydrocarbyl group, such as an optionally substituted C₁₋₄ alkyl, C₂₋₄ alkenyl, or C₂₋₄ alkynyl group, typically an optionally substituted C₁₋₃ alkyl group such as methyl, ethyl or isopropyl, which group may be optionally substituted by e.g. CN. Often a phosphonamidite group as used herein is a methyl phosphonamidite of form -OP(NⁱPr₂)(Me).

As used herein, a phosphonamadate group is an oxidised phosphonamidite of form -OP(O)(NR₂)(R).

Certain compounds may exist in one or more particular geometric, optical, enantiomeric, diasteriomeric, epimeric, atropic, stereoisomeric, tautomeric, conformational, or anomeric forms, including but not limited to, cis- and trans-forms; Eand Z-forms; c-, t-, and r- forms; endo- and exo-forms; R-, S-, and meso-forms; D- and L-forms; d- and 1-forms; (+) and (-) forms; keto-, enol-, and enolate-forms; syn- and antiforms; synclinal- and anticlinal-forms; α- and β-forms; axial and equatorial forms; boat-, chair-, twist-, envelope-, and halfchair-forms; and combinations thereof, hereinafter collectively referred to as "isomers" (or "isomeric forms"). Unless otherwise specified, a reference to a particular compound includes all such isomeric forms, including (wholly or partially) racemic and other mixtures thereof. Methods for the preparation (e.g., asymmetric synthesis) and separation (e.g., fractional crystallisation and chromatographic means) of such isomeric forms are either known in the art or are readily obtained by adapting known methods, in a known manner.

Unless otherwise specified, a reference to a particular compound or complex also includes ionic, salt, solvated and protected forms.

### Methods of modifying polynucleotides

In some embodiments the present invention provides a method for modifying a polynucleotide or an analogue or derivative thereof. The method involves reacting the polynucleotide or analogue or derivative thereof with a compound of formula (I): 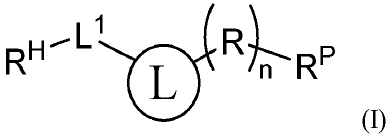 wherein:
- R^{H} is a hydrophobic group;
- L¹ is a linking group;
- Ⓛ is a photolabile group;
- R is a modifying group;
- n is an integer selected from 0 and 1; and
- R^{P} is a phosphorous-based group;
under conditions such that a reactive functional group of the polynucleotide or analogue or derivative thereof reacts with the phosphorous-based group R^{P}, thereby connecting the hydrophobic group R^{H} to the polynucleotide or analogue or derivative thereof.

The methods are not limited in application, but find particular utility in purification of polynucleotides or analogues or derivatives thereof. Therefore, in some embodiments the method is a method for purifying a polynucleotide or an analogue or derivate thereof. For example, the hydrophobic group R^{H} may be used to facilitate purification e.g. by HPCL (e.g. reversed phase HPLC) or other methods. This is described in more detail herein.

As explained in more detail below, the moiety Ⓛ is a photolabile group.

Photocleavage of Ⓛ yields a modified polynucleotide or analogue or derivative thereof which is modified by the presence of R. Photocleavage is described in more detail herein. The polynucleotide or analogue or derivative thereof may be modified by the presence of the R moiety in formula (I) which is retained on the polynucleotide or analogue or derivative thereof following the photocleavage of Ⓛ. This is discussed in more detail below.

The compound of formula (I) used in the method of the present invention is compatible with a wide range of well-known methods of polynucleotide synthesis, including phosphoramidite methods, H-phosphonate methods and phosphotriester methods. Thus, the method of the present invention includes phosphoramidite methods of polynucleotide synthesis, H-phosphonate methods of polynucleotide synthesis and phosphotriester methods of polynucleotide synthesis. Preferably, the method of the present invention is a phosphoramidite method of polynucleotide synthesis.

Also provided are compounds of formula (I) per se.

Further provided is a modified polynucleotide or analogue or derivative thereof comprising a group of formula (I^{∗}).

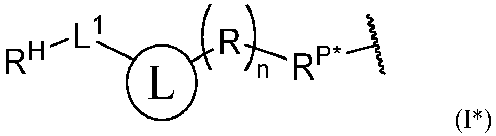

In formula (I^{∗}) R^{H}, L¹, Ⓛ, R and n are as defined herein. R^{P∗} is a phosphorus based linkage to the polynucleotide or analogue or derivative thereof.

### Hydrophobic group R^{H} and linking group L¹

In formula (I), R^{H} is a hydrophobic group. R^{H} may be any suitable hydrophobic group. For example, R^{H} may be any group which alters the hydrophobicity of a polynucleotide or analogue or derivative thereof when the compound of formula (I) is attached to the polynucleotide or analogue or derivative thereof such that the modified polynucleotide or analogue or derivative thereof can be separated from non-modified polynucleotides or analogues or derivatives thereof, e.g. by HPLC.

R may be any modifying group which increase the hydrophobicity of a polynucleotide or analogue or derivative thereof to which it is attached in accordance with the disclosed methods. R may increase the hydrophobicity of the polynucleotide or analogue or derivative thereof to which it is attached by at least 1%, at least 2%, at least 5%, at least 10%, at least 20%, at least 30%, at least 40%, at least 50%, at least 60%, at least 70%, at least 80%, at least 90%, at least 100%, at least 200%, at least 500% or more.

R may alter the properties of the polynucleotide or analogue or derivative thereof to which it is attached in accordance with the disclosed methods when the polynucleotide or analogue or derivative thereof is assessed by HPLC, e.g. by reversed-phase HPLC (RPHPCL).

R may increase the strength of the interaction between the polynucleotide or analogue or derivative thereof to which it is attached in accordance with the disclosed methods and an RPHPCL column. For example, an RPHPCL column may comprise a porous silica support modified with hydrocarbon chains, e.g. with C₁₋₂₀ alkyl, alkenyl and/or alkynyl chains, referred to as a stationary phase. Elution with a hydrophobic molecule (e.g. acetonitrile) in a hydrophilic phase (e.g. ammonium acetate) separates molecules from a mixture applied to the column according to their hydrophobicity. Molecules with a greater degree of hydrophobicity elute more slowly. Accordingly, R may cause the polynucleotide or analogue or derivative thereof to which it is attached in accordance with the disclosed methods to separate from an RPHPLC column more slowly than the corresponding unmodified polynucleotide or analogue or derivative thereof. R may improve the separation of the modified polynucleotide or analogue or derivative thereof from the unmodified polynucleotide or analogue or derivative thereof by at least 1%, at least 2%, at least 5%, at least 10%, at least 20%, at least 30%, at least 40%, at least 50%, at least 60%, at least 70%, at least 80%, at least 90%, at least 100%, at least 200%, at least 500% or more.

R^{H} is typically an organic chemical group having a molecular weight of up to about 5,000 g/mol, more usually up to about 1000 g/mol, such as up to about 500 g/mol, e.g. up to about 300 g/mol. Those skilled in the art will appreciate that as used herein R^{H} is a chemical group and is thus not a particle or nanoparticle such as a bead.

Typically, in formula (I), R^{H} is selected from C₁ to C₂₀ alkyl, C₂ to C₂₀ alkenyl, C₂ to C₂₀ alkynyl, C₅ to C₁₀ carbocyclyl, C₆ to C₁₈ aryl, 5- to 10-membered heteroaryl and 5-to 10-membered heterocyclyl. R^{H} may be optionally substituted or may be unsubstituted.

More typically, R^{H} is selected from C₄ to C₁₆ alkyl, C₄ to C₁₆ alkenyl, C₄ to C₁₆ alkynyl, C₅ to C₁₀ carbocyclyl and C₆ to C₁₀ aryl and is unsubstituted or optionally substituted e.g. as defined herein. More often, R^{H} is selected from C₄ to C₁₂ alkyl, C₄ to C₁₂ alkenyl, C₄ to C₁₂ alkynyl, C₅ to C₆ carbocyclyl and C₆ aryl, and is unsubstituted or optionally substituted e.g. as defined herein. Still more typically, R^{H} is selected from C₄ to C₁₂ alkyl, C₄ to C₁₂ alkenyl and C₄ to C₁₂ alkynyl, and is unsubstituted or optionally substituted e.g. as defined herein. More often, R^{H} is selected from C₅ to C₁₂ alkyl; wherein R^{H} is optionally substituted and is preferably unsubstituted; for example R^{H} may be unsubstituted C₅₋C₉ alkyl.

Typically, R^{H} is not a DMT or ODMT group.

When R^{H} is substituted, R^{H} is typically substituted by 1, 2 or 3 substituents independently selected from halogen, cyano, C₁ to C₄ alkyl and phenyl.

In formula (I), L¹ is a linking group which links the hydrophobic group R^{H} to the photolabile group Ⓛ. L¹ may be any suitable linking group.

In one embodiment, L¹ is a chemical bond such as a covalent bond. In embodiments wherein L¹ is a chemical bond (e.g. a covalent bond) the hydrophobic group R^{H} is attached directly to the photolabile group. In some embodiments this may be useful in simplifying synthesis and/or for reducing reagent costs.

In one embodiment, L¹ is a linker. Any suitable linker may be used. For example, L¹ may comprise or consist of a linker comprising a C₁ to C₂₀ alkylene group, a C₂ to C₂₀ alkenylene group and/or a C₂ to C₂₀ alkynylene group, wherein said alkylene, alkenylene or alkynylene group is optionally interrupted by and/or terminated in one or more groups selected from a heteroatom, a phosphite group, a phosphate group, a carbonyl group, a C₆ to C₁₀ aryl group, a C₅ to C₁₀ carbocyclyl group, a 5- to 10-membered heteroaryl group and a 5- to 10-membered saturated or partially unsaturated heterocyclic group. The linker may be unubstituted, or may be optionally further substituted. In some embodiments the use of a chemical linker such as one of the preceding linkers may be useful for increasing the hydrophobicity of the hydrophobic group and/or for providing a robust scaffold for attachment of many different hydrophobic groups according to the desired application without altering the chemistry of the photolabile group.

Typically, L¹ is or comprises a linker comprising a C₁ to C₆ alkylene group, a C₂ to C₆ alkenylene group and/or a C₂ to C₆ alkynylene group, wherein said alkylene, alkenylene or alkynylene group is optionally interrupted by and/or terminated in one or more groups selected from a heteroatom, a carbonyl group, a phenyl group, a cyclopentyl or cyclohexyl group, a 5- to 6-membered heteroaryl group and a 5- to 6-membered saturated or partially unsaturated heterocyclic group, wherein the linker is optionally further substituted.

Suitable heteroatoms and heterogroups include -O-, -S-, -SO-, -SO₂-, -NR^{z}-, - C(O)NR^{z}, etc, wherein R^{z} is H or methyl, preferably H. An exemplary alkylene group which is interrupted by and/or terminated in an -O- atom is an alkylene glycol group and therefore the linking group may comprise or consist of a polyalkylene glycol such as polyethylene glycol (PEG). Typically the linking group is not linked to a biotin or streptavidin moiety. Sometimes L¹ is or comprises a linker comprising a C₁ to C₆ alkylene group, a C₂ to C₆ alkenylene group and/or a C₂ to C₆ alkynylene group, wherein said alkylene, alkenylene or alkynylene group is not interrupted by or terminated in one or more oxygen groups.

When the linker is substituted, the linker is typically substituted by 1, 2 or 3 substituents independently selected from halogen, cyano, -OR^{x}, -SR^{x}, -NR^{x}R^{x}, -C(O)OR^{x}, -C(O)NR^{x}R^{x}, -C(O)R^{x} and a C₁ to C₄ alkyl group, wherein R^{x} is hydrogen or unsubstituted C₁ to C₄ alkyl.

More typically, L¹ is a C₁ to C₆ alkylene group, a C₂ to C₆ alkenylene group and/or a C₂ to C₆ alkynylene group, wherein said alkylene, alkenylene or alkynylene group is optionally interrupted by and/or terminated in one or more groups selected from a heteroatom such as -O-, -S-, -SO-, -SO₂-, -NR^{z}-, and -C(O)NR^{z}, wherein R^{z} is H or methyl; a carbonyl group, a phenyl group, a cyclopentyl or cyclohexyl group, a 5- to 6-membered heteroaryl group and a 5- to 6-membered saturated or partially unsaturated heterocyclic group.

Usually, when the linker comprises a 5- to 6-membered heteroaryl group the heteroaryl group is a triazole or imidazole, preferably triazole.

More typically, L¹ is a C₁ to C₃ alkylene group, a C₂ to C₃ alkenylene group and/or a C₂ to C₃ alkynylene group, wherein said alkylene, alkenylene or alkynylene group is optionally interrupted by and/or terminated in one or more groups selected from -O-, - NR^{z}-, and -C(O)NR^{z}, wherein R^{z} is H; a carbonyl group, a phenyl group, triazole or imidazole.

In some embodiments L¹ is or comprises a moiety of form: -A-B- wherein A is a C₁ to C₅ alkylene group which alkylene group is unsubstituted or substituted as described herein, preferably unsubstituted; and B is a cyclic group selected from a C₆ aryl group; a C₅ to C₆ carbocyclyl group; a 5- to 6-membered heteroaryl group; and a 5- to 6-membered saturated or partially unsaturated heterocyclic group.

Preferably, when the linking group comprises a moiety of form: -A-B-, A is an unsubstituted C₁ to C₄ alkylene group; and B is a cyclic group selected from a C₆ aryl group; a C₅ to C₆ carbocyclyl group; a 5- to 6-membered heteroaryl group; and a 5- to 6-membered saturated or partially unsaturated heterocyclic group.

More preferably, when the linking group comprises a moiety of form: -A-B-, A is an unsubstituted C₁ to C₃ alkylene group; and B is a cyclic group selected from triazole, benzene, cyclohexane, piperidine, pyridazine, pyridine, thiazole and imidazole, preferably triazole.

In some embodiments the linking group comprises a moiety of the form: 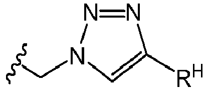 wherein the wavy line indicates the point of attachment to the rest of the molecule.

In some embodiments, the linking group is a moiety of form: -C- wherein C is a C₂ to C₅ alkynylene group which alkynylene group is unsubstituted or substituted as described herein, preferably unsubstituted. In some embodiments, C is an unsubstituted C₂ to C₃ alkynylene group. In some embodiments, the linking group is a moiety of the form: 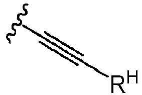 wherein the wavy line indicates the point of attachment to the rest of the molecule.

### Phosphorus based group R^{p}

In formula (I), R^{P} is a phosphorus based group suitable for reaction with a polynucleotide or analogue or derivative thereof so as to modify the polynucleotide or analogue or derivative thereof with the compound of formula (I).

In the disclosed methods, a reactive functional group of the polynucleotide or analogue or derivative thereof to be modified with the compound of formula (I) reacts with the group R^{P} thereby connecting the hydrophobic group R^{H} of the compound of formula (I) to the polynucleotide or analogue or derivative thereof, and thereby modifying the polynucleotide or analogue or derivative thereof.

The disclosed methods may comprise reacting a reactive functional group at either the 5' position or 3' position of the polynucleotide or analogue or derivative thereof with group R^{P}. Accordingly, the compound of formula (I) can be utilised to modify polynucleotides or analogues or derivatives thereof whether synthesized in either the 3' to 5' direction or the 5' to 3' direction.

Typically, the disclosed methods comprise reacting a reactive functional group at the 5' position of the polynucleotide or analogue or derivative thereof with group R^{P}. Typically, the disclosed methods comprise reacting a free hydroxyl group at the 5' position of the polynucleotide or analogue or derivative thereof with the phosphorous-based group R^{P}. This is particularly suitable to synthesis of the polynucleotide chain in the 3' to 5' direction, which is typically cheaper and more versatile, and which therefore typically leaves a free hydroxyl group at the 5' position for reaction with the R^{P} group of formula (I).

In some embodiments, however, the disclosed methods comprise reacting a reactive functional group at the 3' position of the polynucleotide or analogue or derivative thereof with group R^{P}. Typically, in such embodiments, the disclosed methods comprise reacting a free hydroxyl group at the 3' position of the polynucleotide or analogue or derivative thereof with the phosphorous-based group R^{P}.

Typically, the compound of formula (I) does not react with functional groups which may be present at positions apart from at the 5' or 3' position of the polynucleotide or analogue or derivative thereof. In some embodiments no such functional groups are present. In some embodiments such functional groups are protected or masked to prevent such reaction.

Typically, the terminal monomer unit of the polynucleotide or analogue or derivative thereof with which the compound of formula (I) is reacted is represented by formula (N-1) or formula (N-2), preferably by formula (N-1). 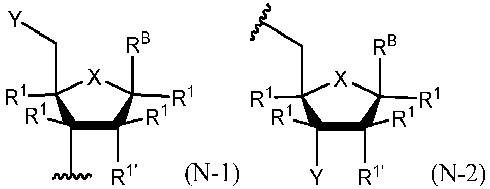 wherein the wavy line indicates the point of attachment to the remainder of the polynucleotide or analogue or derivative thereof.

In formula (N-1) and (N-2), X is an oxygen atom, a nitrogen atom, a sulphur atom or -C(R^{a}R^{a})-. Preferably, X is an oxygen atom.

Each R¹ and R^{1'} is independently selected from hydrogen, OH, halogen, optionally substituted C₁ to C₄ alkyl, -OR^{a}, -SR^{a}, -NR^{a}R^{a}, -C(O)OR^{a}, -C(O)NR^{a}R^{a} and -C(O)R^{b}. One R¹ group may be joined to R^{1'} to form a bridging motif such as a -CH₂-O- bridge e.g. between the 2' and 4' positions of the ring. Preferably, each R¹ is independently selected from hydrogen, OH, halogen, C₁ to C₂ alkyl, C₁ to C₂ haloalkyl and C₁ to C₂ alkoxyl. More preferably, each R¹ is selected from hydrogen, fluorine, methyl and methoxy. Further preferably, each R¹ is hydrogen. Preferably, R^{1'} is hydrogen, OH, halogen, C₁ to C₂ alkyl, C₁ to C₂ haloalkyl and C₁ to C₂ alkoxyl. More preferably, R^{1'} is selected from hydrogen, OH, fluorine, methyl and methoxy. Further preferably, R^{1'} is H or OH.

R^{B} is an optionally substituted natural or non-natural nucleobase or a derivative or analogue thereof. Natural nucleobases include adenine, cytosine, guanine, thymine, and uracil. Non-natural nucleobases are well known in the art, and include, for example, analogues of the natural nucleobases such as 2-thiothymine, isomers of the natural nucleobases such as isoguanine, isocyanine and 5-aza-7-deazaguanine, and other groups used in the art as synthetic nucleobases, such as xanthine. For example, non-natural nucleobases include 2-thiothymine, isoguanine, isocytosine, 1-methylcytosine, 5-aza-7-deazaguanine, 6-amino-5-nitropyridin-2-one, 1-methylcytosine, xanthine, hypoxanthine, 7-methylguanine, 5,6-dihydrouracil, 5-methylcytosine, 5-hydroxymethylcytosine, 2,6-diaminopurine, 6,8-diaminopurine, fluorocytosine, fluorouracil, mercaptopurine, thioguanine, 5-hydroxymethyl uracil, 5-carboxycytosine, and fluorescent nucleobases such 2-aminopurine and tricyclic cytosines tC, tCO and tCnitro. Preferably, R^{B} is a protected natural or non-natural nucleobase. More preferably, R^{B} is a protected natural nucleobase.

Y is a reactive functional group on the polynucleotide and represents the point of attachment to the R^{P} group of formula (I). Typically Y is an -OH or -O⁻ group, or an activated derivative thereof, most typically -OH. Typically Y reacts with the compound of formula (I) by nucleophilic attack at the phosphorus atom of the group R^{P} or an activated form thereof. This is described in more detail herein.

Typically, in formula (I), R^{P} is a phosphoramidite, a phosphoramidate, an alkyl phosphonamidite or an alkyl phosphonamidate. More typically, R^{P} is a phosphoramidite or an alkyl phosphonamidite. For example, R^{P} may be a phosphoramidite or a methyl phosphonamidite. Most often, R^{P} is 2-cyanoethyl N,N-diisopropyl phosphoramidite or N,N-diisopropyl methylphosphonamidite.

### Photolabile group Ⓛ

As explained above, Ⓛ is a photolabile group. The photolabile group may be cleaved by photolytic excitation, e.g. by exposure to UV light. The photolabile group is typically cleaved using under mild conditions that are orthogonal to reaction conditions typically used in polynucleotide synthesis. This is described in more detail herein.

The photolabile group typically does not include a hydrolysable motif such as an ester or carbonate group. Accordingly, it is typically stable under reaction conditions typically used to deprotect synthesised polynucleotides - for example, deprotecting conditions used to remove hydrolysable protecting groups from protected nucleobases present in synthesised polynucleotides. For example, typically the photocleavable linker is stable with respect to concentrated ammonium hydroxide and/or ammonia. This advantageously allows various protecting groups to be removed from a synthesised polynucleotide whilst retaining the photolabile group in situ. Accordingly, it is typically possible to modify a synthesized polynucleotide or analogue or derivative thereof with a compound of formula (I) and then to deprotect any protected functional groups on the polynucleotide, prior to purification of the deprotected polynucleotide or analogue or derivative thereof as described herein.

The photolabile group advantageously cleaves in a single, typically concerted reaction step to provide the modified nucleoside, nucleotide, polynucleotide or derivative or analogue thereof. This is a particular advantage compared to some photocleavable groups known in the art (in different contexts) which comprise e.g. diester moieties and which cleave to leave residual succinyl functionalisation which may not be desired; or which comprise carbonate moieties and which may cleave to leave residual carbonate functionalisation which again may not be desired. The avoidance of such groups is particularly useful as harsh reaction conditions are typically required to remove succinyl or carbonate groups from succinyl- or carbonate-functionalised polynucleotides which may lead to degradation. Furthermore, the by-products of the cleavage are typically volatile and thus easily removed. The photolabile group is typically stable under common hydrolysis conditions.

In some embodiments, Ⓛ is a moiety of formula

-Ⓐ-CHR³-W-

In such embodiments, the compound of formula (I) is therefore a compound of formula (II): 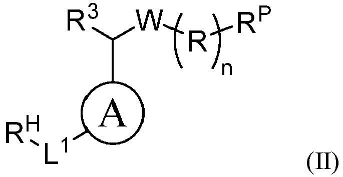 wherein R^{H}, L¹, R, n and R^{P} are as described herein.

In formula (II), Ⓐ is a 2-nitrobenzyl group, wherein said 2-nitrobenzyl group is optionally substituted with 1, 2 or 3 groups independently selected from halogen, optionally substituted C₁ to C₄ alkyl, -OR^{a}, -SR^{a}, -NR^{a}R^{a}, -C(O)OR^{a}, -C(O)NR^{a}R^{a}, -C(O)R^{b}, -OC(O)R^{b} and -NHC(O)R^{b}.

Each R^{a} is independently selected from hydrogen, optionally substituted C₁ to C₂ alkyl and optionally substituted C₁ to C₂ alkoxyl. Preferably each R^{a} is independently selected from hydrogen or methyl, more preferably each R^{a} is hydrogen.

Each R^{b} is independently selected from hydrogen and an optionally substituted C₁ to C₄ alkyl group. Preferably each R^{b} is independently selected from hydrogen or methyl, more preferably each R^{b} is hydrogen.

As used herein, the term "2-nitrobenzyl group" refers to a nitrobenzyl group that is attached to the adj acent carbon atom of formula (I) such that the nitro group is at the 2-position relative to the -C(R³)-W- moiety of formula (I), as indicated below (the L¹-R^{H} moiety and optional further substituents are not depicted for clarity).

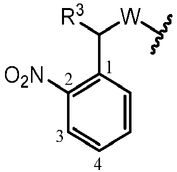

That is, in the 2-nitrobenzyl group represented by Ⓐ, the nitro group is ortho to the adjacent carbon atom of formula (I); i.e. the nitro group is ortho to the -C(R³)- carbon atom of formula (I). Therefore, in formula (II), Ⓐ is a 2-nitrobenzyl group in which the -L¹-R^{H} and -CHR³W(R)ₙR^{P} substituents are substituents of the benzene ring; i.e. the benzene ring is substituted by a -CHR³W(R)ₙR^{P} substituent at the 1-position; a nitro group at the 2' position; and a -L¹-R^{H} at a further position (e.g. at the 3-, 4-, 5- or 6- position); as well as being optionally further substituted as described herein; i.e.:

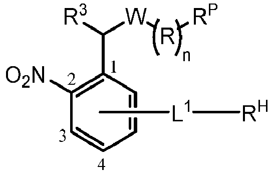

Those skilled in the art will thus appreciate that the compound of formula (I) has the photocleavable group e.g. the 2-nitrobenzyl group "in line". This differs from compounds in which the 2'nitrobenzyl group is pendant from a backbone; e.g. where a phosphorus based group is connected to a hydrophobic group by an alkylene chain which may be optionally interrupted by one or more heteroatoms and wherein the alkylene chain may be substituted by a aryl group which may be further substituted by a nitro group. Such compounds are not examples of a compound of formula (I).

As explained above, the nitrobenzene group Ⓐ may be unsubstituted (apart from by the -CR³-W- and -L¹-H¹ moieties as depicted in formula (II), or may be further substituted. When Ⓐ is further substituted it may be substituted by 1, 2 or 3 substituents independently selected from e.g. electron-donating groups. Ⓐ may be substituted by 1, 2 or 3 groups independently selected from halogen, optionally substituted C₁ to C₄ alkyl, - OR^{a}, -SR^{a}, -NR^{a}R^{a}, -C(O)OR^{a}, -C(O)NR^{a}R^{a}, -C(O)R^{b}, -OC(O)R^{b} and -NHC(O)R^{b}, wherein R^{a} and R^{b} are as defined above. Preferably, when Ⓐ is substituted it is substituted by 1, 2 or 3, preferably 1 or 2 groups selected from C₁ to C₄ alkyl, -OR^{a}, -SR^{a}, -NR^{a}R^{a}, - OC(O)R^{b} and -NHC(O)R^{b}. More preferably, when Ⓐ is substituted it is substituted by 1 or 2, preferably 1 group selected from OR^{a}, and -OC(O)R^{b}, preferably methoxy. Most preferably Ⓐ is unsubstituted apart from by the nitro group of the nitrobenzene and by the -CR³-W- and -L¹-H¹ moieties as depicted above.

Typically, in formula (II), Ⓐ, L¹ and R^{H} are together represented by formula (A-1) or formula (A-2): 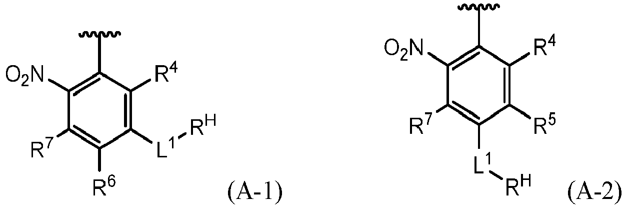 wherein L¹ and R^{H} are as defined herein.

In formula (A-1) and (A-2), R⁴, R⁵, R⁶ and R⁷ are each independently selected from hydrogen and a substituent which may be an electron-donating group. In formula (A-1) and (A-2), R⁴, R⁵, R⁶ and R⁷ are typically each independently selected from hydrogen, halogen, optionally substituted C₁ to C₄ alkyl, -OR^{a}, -SR^{a}, -NR^{a}R^{a}, -C(O)OR^{a}, - C(O)NR^{a}R^{a}, -C(O)R^{b}, -OC(O)R^{b} and -NHC(O)R^{b}, wherein R^{a} and R^{b} are as defined above.

Preferably, R⁴, R⁵, R⁶ and R⁷ are each independently selected from hydrogen, fluorine, optionally substituted C₁ to C₄ alkyl, -OR^{a}, -SR^{a}, -NR^{a}R^{a}, -OC(O)R^{b} and -NHC(O)R^{b}. More preferably, R⁴, R⁵, R⁶ and R⁷ are each independently selected from hydrogen, -OR^{a}, -SR^{a}, -NR^{a}R^{a}, -OC(O)R^{b} and -NHC(O)R^{b}. Still more preferably R⁴, R⁵, R⁶ and R⁷ are each independently selected from hydrogen, -OR^{a}, and -OC(O)R^{b}. Further preferably, R⁴, R⁵, R⁶ and R⁷ are each independently selected from hydrogen and methoxy. Without being bound by theory, the inventors believe that when one or more of R⁴, R⁵, R⁶ and R⁷ are electron donating groups, the linker is more easily cleaved using photoexcitation. When R⁴, R⁵, R⁶ and/or R⁷ is hydrogen, the synthesis of the compound of formula (I) may be more facile.

Preferably, at least one of R⁴, R⁵, R⁶ and/or R⁷ is hydrogen. For example, one, two or three of R⁴, R⁵, R⁶ and/or R⁷ is hydrogen. Preferably, one of R⁴ and R⁷ is hydrogen and the other of R⁴ and R⁷ is selected from hydrogen, -OR^{a}, and -OC(O)R^{b}. Preferably, R⁵ or R⁶ is hydrogen. For example, in some embodiments one of R⁴ and R⁷ is hydrogen and the other of R⁴ and R⁷ is selected from hydrogen, -OR^{a}, and -OC(O)R^{b} and R⁵ or R⁶ is hydrogen or methoxy.

In formula (II), R³ is methyl, ethyl or C₁ to C₂ haloalkyl. Preferably, R³ is methyl, ethyl or C₁ to C₂ fluoroalkyl. More preferably, R³ is methyl or ethyl. Alternatively, R³ may be methyl or CF₃. Most preferably, R³ is methyl. When R³ is one of these groups, the photolabile group may be more easily cleaved using photoexcitation.

In formula (II), W is selected from a group of formula (W-2); an oxygen atom, and a group of formula (W-1): 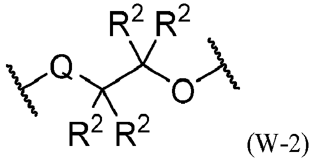 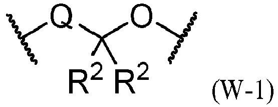 wherein:
- when n is 1, R^{P} is a phosphoramidite, a phosphoramidate, an alkyl phosphonamidite or an alkyl phosphonamidate; and when n is 0 R^{P} together with the oxygen atom to which it is attached forms a phosphoramidite, a phosphoramidate, an alkyl phosphonamidite or an alkyl phosphonamidate;
   wherein said phosphoramidite, a phosphoramidate, an alkyl phosphonamidite or an alkyl phosphonamidate is preferably a phosphoramidite or a methyl phosphonamidite, more preferably 2-cyanoethyl N,N-diisopropyl phosphoramidite or N,N-diisopropyl methylphosphonamidite;
- Q is an oxygen atom or a sulphur atom; and
- each R² is independently selected from hydrogen, methyl, ethyl, C₁ to C₂ haloalkyl and a halogen group.

Typically, in formulae (W-2) and (W-1), Q is an oxygen atom.

Typically, in formulae (W-2) and (W-1), each R² is independently selected from hydrogen, methyl, ethyl, C₁ to C₂ haloalkyl and a halogen group. More typically each R² is independently selected from hydrogen, methyl, and ethyl.

When two R² groups are attached to the same carbon atom, typically one R² group is hydrogen and one R² group is selected from hydrogen, methyl, ethyl, C₁ to C₂ haloalkyl and a halogen group, typically from hydrogen, methyl, and ethyl.

Typically each R² is independently hydrogen or methyl. More typically, when two R² groups are attached to the same carbon atom, one R² group is hydrogen and the other R² group is hydrogen or methyl.

Most typically each R² is hydrogen. This can enable more facile synthesis of the compound of formula (I).

Typically, W is a group of formula (W-2) or an oxygen atom. Most typically W is a group of formula (W-2).

When W is a group of formula (W-2), n is typically 0, such that the compound of formula (II) is a compound of formula (II-3): 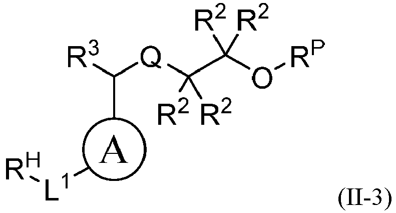 wherein R^{H}, L¹, Ⓐ, R³, Q, R² are as defined herein.

Typically, in formula (II-3), R^{P} together with the oxygen atom to which it is attached forms an alkyl phosphonamidite. Typically R^{P} together with the oxygen atom to which it is attached forms a C₁₋₄ alkyl phosphonamidite. More typically, R^{P} together with the oxygen atom to which it is attached forms a methyl phosphonamidite. Most typically, R^{P} together with the oxygen atom to which it is attached forms N,N-diisopropyl methylphosphonamidite such that the moiety of formula (II) is as depicted below: 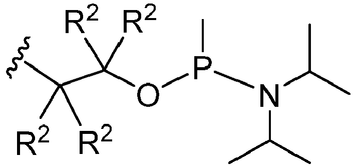 wherein the wiggly line indicates the point of attachment to the Q-R³-Ⓐ-L¹-R^{H} moiety.

Accordingly, in some embodiments the compound of formula (II) is: 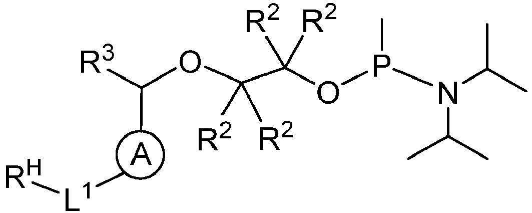 wherein R^{H}, L¹, Ⓐ, R³, and R² are as defined herein.

In some embodiments, the compound of formula (II) is: 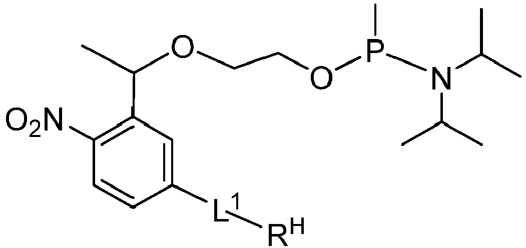 wherein R^{H} and L¹ are as defined herein.

Typically, L¹-R^{H} is 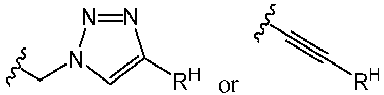 wherein the wavy line indicates the point of attachment to the rest of the molecule. Typically R^{H} is C₅ to C₁₂ alkyl; wherein R^{H} is optionally substituted and is preferably unsubstituted; for example R^{H} may be unsubstituted C₅₋C₉ alkyl.

Solely to aid understanding, and without in any way being bound by theory, the inventors believe that the compound of formula (II-3) may undergo photolytic cleavage as shown by way of one specific example, below, 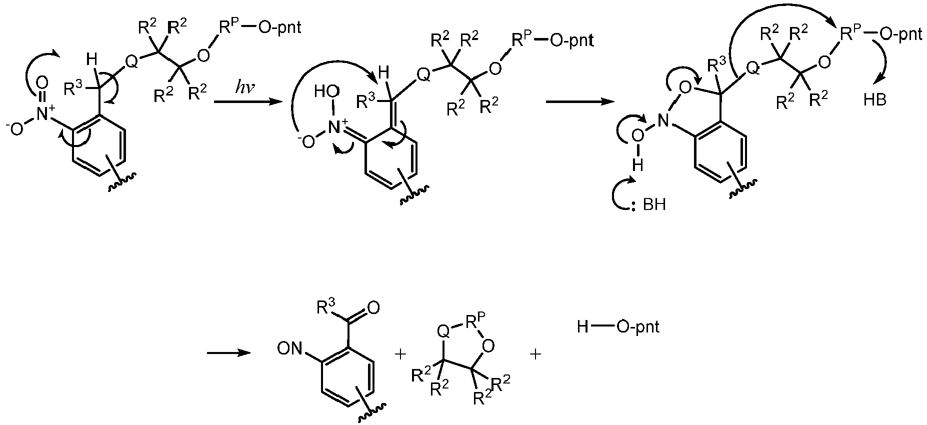 wherein pnt is the polynucleotide or analogue or derivative thereof which is modified at an oxygen atom with the compound of formula (I), HB is an acid and B is a base. However, the present invention is not limited to this specific compound or to methods using this specific compound.

When W is an oxygen atom, n is typically 0, such that the compound of formula (II) is a compound of formula (II-1): 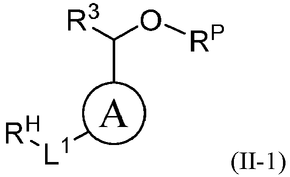 wherein R^{H}, L¹, Ⓐ, and R³ are as defined herein.

Typically, in formula (II-1), R^{P} together with the oxygen atom to which it is attached forms a phosphoramidite. Typically R^{P} together with the oxygen atom to which it is attached forms a substituted C₁₋₄ alkyl phosphoramidite. Most typically, R^{P} together with the oxygen atom to which it is attached forms 2-cyanoethyl N,N-diisopropyl phosphoramidite, such that the moiety of formula (II) is as depicted below: 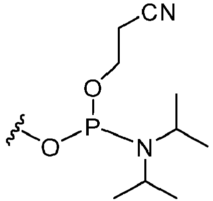 wherein the wiggly line indicates the point of attachment to the CHR³-^{®}-L¹-R^{H} moiety.

Accordingly, in some embodiments the compound of formula (II) is: 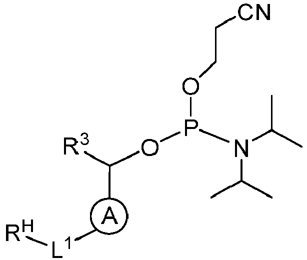 wherein R^{H}, L¹, Ⓐ, R³, and R² are as defined herein.

In some embodiments, the compound of formula (II) is: 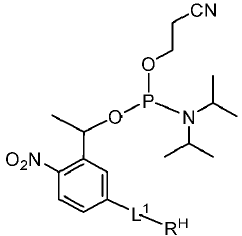 wherein R^{H} and L¹ are as defined herein.

Typically, L¹-R^{H} is 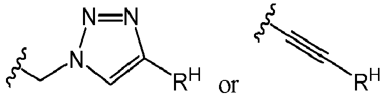 wherein the wavy line indicates the point of attachment to the rest of the molecule. Typically R^{H} is C₅ to C₁₂ alkyl; wherein R^{H} is optionally substituted and is preferably unsubstituted; for example R^{H} may be unsubstituted C₅₋C₉ alkyl.

Solely to aid understanding, and without being bound by theory in any way, the inventors believe that the compound of formula (II-1) may undergo photolytic cleavage as shown by way of one specific example, below, 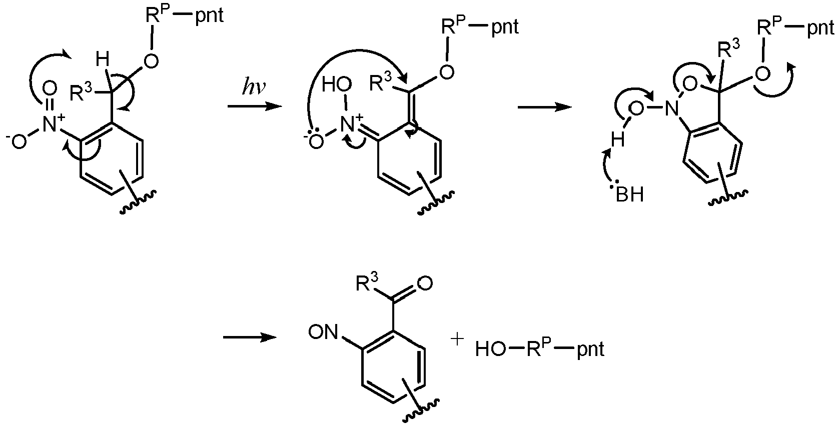 wherein pnt is the polynucleotide or analogue or derivative thereof which is modified with the compound of formula (I), HB is an acid and B is a base. However, the present invention is not limited to this specific compound or to methods using this specific compound.

When W is a group of formula (W-1), n is typically 1, such that the compound of formula (II) is a compound of formula (II-2): 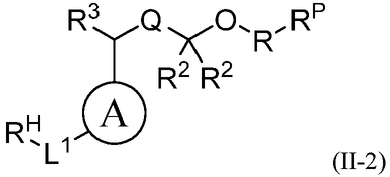 wherein R^{H}, L¹, Ⓐ, R³, Q, and R² are as defined herein.

Typically, in formula (II-2), R^{P} is a phosphoramidite. Typically R^{P} is a substituted C₁₋₄ alkyl phosphoramidite. Most typically, R^{P} is 2-cyanoethyl N,N-diisopropyl phosphoramidite, such that the moiety of formula (II) is as depicted below: 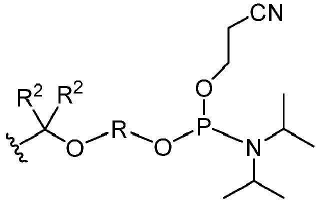 wherein the wiggly line indicates the point of attachment to the Q-R³-Ⓐ-L¹-R^{H} moiety.

Accordingly, in some embodiments the compound of formula (II) is: 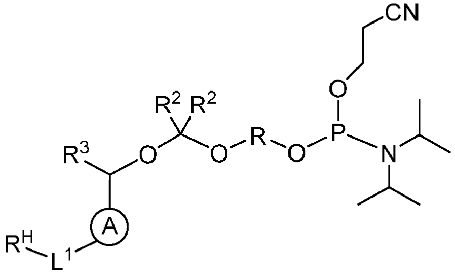 wherein R^{H}, L¹, Ⓐ, R³, R² and R are as defined herein.

In some embodiments, the compound of formula (II) is: 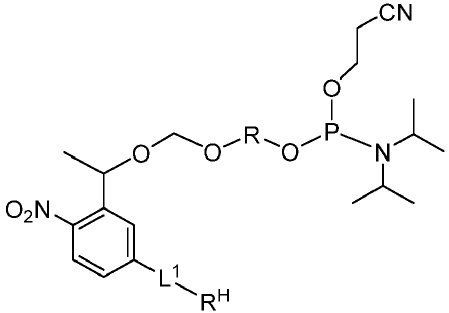 wherein R, R^{H} and L¹ are as defined herein.

Typically, L¹-R^{H} is 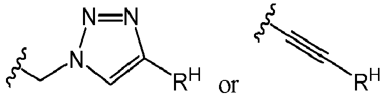 wherein the wavy line indicates the point of attachment to the rest of the molecule. Typically R^{H} is C₅ to C₁₂ alkyl; wherein R^{H} is optionally substituted and is preferably unsubstituted; for example R^{H} may be unsubstituted C₅₋C₉ alkyl.

Solely to aid understanding, and without in any way being bound by theory, the inventors believe that the compound of formula (II-2) may undergo photolytic cleavage as shown by way of one specific example, below, 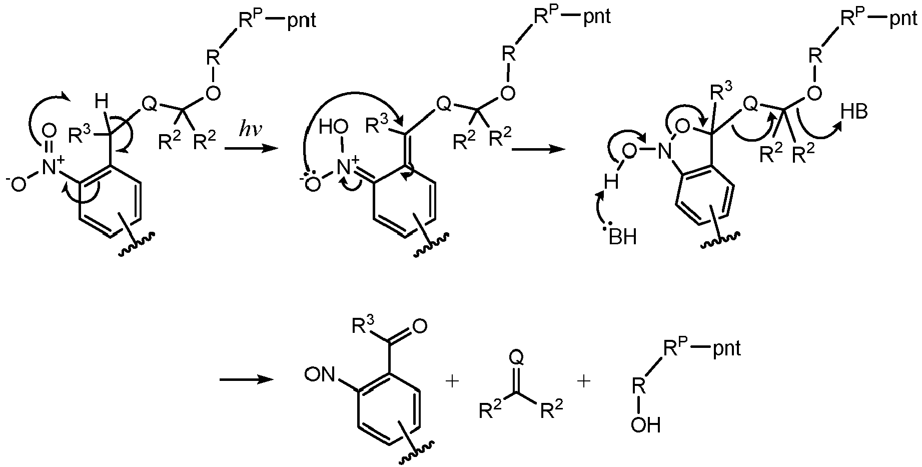 wherein pnt is the polynucleotide or analogue or derivative thereof which is modified with the compound of formula (I), HB is an acid and B is a base. However, the present invention is not limited to this specific compound or to methods using this specific compound.

### Modifying group R

In formula (I), R is a modifying group and n is 0 or 1. Accordingly, R is optionally present. For example, R may be present (i.e. n may be 1) particularly when Ⓛ is a moiety of formula

-Ⓐ-CHR³-W-

as defined herein and W is a group of formula (W-1) as defined herein.

In formula (I), R may be any suitable modifying group. Suitable modifying groups are groups which may be incorporated e.g. at the terminus e.g. the 5' terminus of a synthesized polynucleotide.

For example, R may be selected from:
(i) a nucleoside or a derivative or analogue thereof;
(ii) a group comprising or consisting of a C₁ to C₂₀ alkylene group, a C₂ to C₂₀ alkenylene group and/or a C₂ to C₂₀ alkynylene group, wherein said alkylene, alkenylene or alkynylene group is optionally interrupted by and/or terminated in one or more groups selected from: a heteroatom; a phosphite group; a phosphate group; a carbonyl group; a C₆ to C₁₀ aryl group; a C₅ to C₁₀ carbocyclyl group; a 5- to 10-membered heteroaryl group; and a 5- to 10-membered heterocyclic group; and wherein R is optionally further substituted; and
(iii) a second polynucleotide or a derivative or analogue thereof.

When R is a nucleoside or a derivative or analogue, R is typically a ribose or deoxyribose nucleoside. R may be a nucleoside having a purine or pyrimidine base. R may be an abasic nucleoside.

In some embodiments, R may be selected from adenosine, guanosine, thymidine, uridine, 5-methylcytidine, 5-hydroxymethylcytidine, cytidine, cyclic adenosine, cyclic guanosine, deoxyadenosine, deoxyguanosine, deoxythymidine, deoxyuridine, deoxycytidine and deoxymethylcytidine.

In some embodiments, R may comprise of consists of a nitroindole, a inosine, an acridine, a 2-aminopurine, a 2-6-diaminopurines, a 5-bromo-deoxyuridine, an inverted thymidine (inverted dT), an inverted dideoxy-thymidines (ddT), a dideoxy-cytidine (ddC), a 5-hydroxymethylcytidine, a 2'-O-methyl RNA base, an iso-deoxycytidine (Iso-dC), an iso-deoxyguanosine (Iso-dGs).

In some embodiments, R is a nucleoside analogue such as a peptide nucleic acid (PNA), glycerol nucleic acid (GNA), threose nucleic acid (TNA), or locked nucleic acid (LNA) analogue of an abasic nucleoside or of adenosine, guanosine, thymidine, uridine, 5-methylcytidine, 5-hydroxymethylcytidine, cytidine, cyclic adenosine, cyclic guanosine, deoxyadenosine, deoxyguanosine, deoxythymidine, deoxyuridine, deoxycytidine and deoxymethylcytidine.

An example of the synthesis and utilisation of a compound of formula (I) providing a deoxythymidine (dT) modification is provided in examples 3 and 8.

In some embodiments, R comprises or consists of a C₁ to C₂₀ alkylene group, a C₂ to C₂₀ alkenylene group and/or a C₂ to C₂₀ alkynylene group, wherein said alkylene, alkenylene or alkynylene group is optionally interrupted by and/or terminated in one or more groups selected from: a heteroatom; a phosphite group; a phosphate group; a carbonyl group; a C₆ to C₁₀ aryl group; a C₅ to C₁₀ carbocyclyl group; a 5- to 10-membered heteroaryl group; and a 5- to 10-membered heterocyclic group; and wherein R is optionally further substituted.

In some embodiments, R comprises or consists of a C₂ to C₆ alkylene group, a C₂ to C₆ alkenylene group and/or a C₂ to C₆ alkynylene group, wherein said alkylene, alkenylene or alkynylene group is optionally interrupted by and/or terminated in one or more groups selected from a heteroatom, a carbonyl group, a phenyl group, a cyclopentyl or cyclohexyl group, a 5- to 6-membered heteroaryl group and a 5- to 6-membered saturated or partially unsaturated heterocyclic group, wherein R is optionally further substituted.

In some embodiments, R comprises or consists of an unsubstituted C₂ to C₆ alkylene group. For example, in some embodiments R comprises or consists of a C₃ alkylene group. Together with the R^{P} group to which it is attached, R may therefore comprise a C3 spacer. An example of the synthesis and utilisation of a compound of formula (I) providing a C3 spacer modification is provided in examples 2 and 6.

In some embodiments, R comprises or consists of one or more PEG (CH₂CH₂O) moieties. For example, in some embodiments R comprises or consists of a (OCH₂CH₂)ₙ moiety wherein n is an integer between 1 and 10, e.g. between 3 and 6. Together with the R^{P} group to which it is attached, R may therefore comprise an iSp9 spacer (spacer 9) or an iSp18 spacer (spacer 18).

In some embodiments, R is a second polynucleotide or a derivative or analogue thereof. In some embodiments R is a polynucleotide comprising between 2 and 100 nucleotide monomers, e.g. between 2 and 10 monomers, e.g. 2, 3, 4, 5, 6, 7, 8, 9, or 10 monomers.

### Preferred aspects

Preferably, in the disclosed methods and compounds:
- the compound of formula (I) is a compound of formula (II) 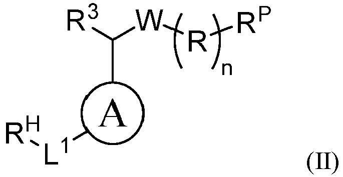
- Ⓐ, L¹ and R^{H} are together represented by formula (A-1): 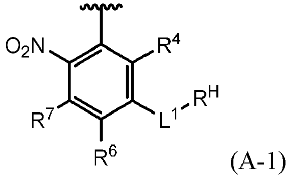 - R⁴, R⁵, R⁶ and R⁷ are each independently selected from hydrogen, -OR^{a}, -SR^{a}, - NR^{a}R^{a}, -OC(O)R^{b} and -NHC(O)R^{b};
- L¹ is a C₁ to C₃ alkylene group, a C₂ to C₃ alkenylene group and/or a C₂ to C₃ alkynylene group; or is a moiety of form: -A-B-, wherein A is an unsubstituted C₁ to C₄ alkylene group; and B is a cyclic group selected from a C₆ aryl group; a C₅ to C₆ carbocyclyl group; a 5- to 6-membered heteroaryl group; and a 5- to 6-membered saturated or partially unsaturated heterocyclic group;
- R^{H} is selected from C₄ to C₁₆ alkyl, C₄ to C₁₆ alkenyl, C₄ to C₁₆ alkynyl, C₅ to C₁₀ carbocyclyl and C₆ to C₁₀ aryl;
- R³ is methyl, ethyl or C₁ to C₂ haloalkyl;
- W is selected from a group of formula (W-2); an oxygen atom, and a group of formula (W-1): 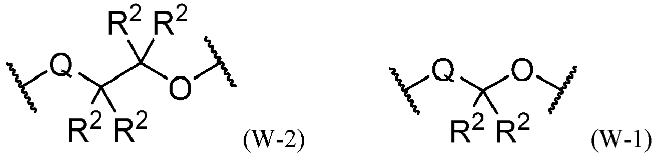
- When n is 1 R^{P} is a phosphoramidite or an alkyl phosphonamidite and when n is 0 R^{P}, together with the oxygen atom to which it is attached, forms a phosphoramidite, or an alkyl phosphonamidite;
- Q is an oxygen atom or a sulphur atom; and
- each R² is independently selected from hydrogen, methyl, ethyl, C₁ to C₂ haloalkyl and a halogen group; and
- R is a nucleoside or a derivative or analogue thereof; or is a C₂ to C₆ alkylene group, a C₂ to C₆ alkenylene group and/or a C₂ to C₆ alkynylene group, wherein said alkylene, alkenylene or alkynylene group is optionally interrupted by and/or terminated in one or more groups selected from a heteroatom, a carbonyl group, a phenyl group, a cyclopentyl or cyclohexyl group, a 5- to 6-membered heteroaryl group and a 5- to 6-membered saturated or partially unsaturated heterocyclic group.

In a first preferred aspect,
- the compound of formula (I) is a compound of formula: 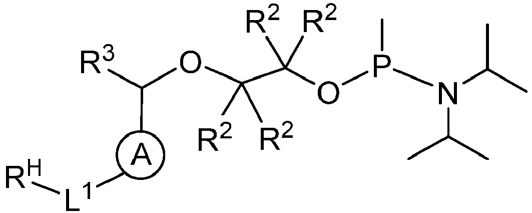
- Ⓐ, L¹ and R^{H} are together represented by formula (A-1): 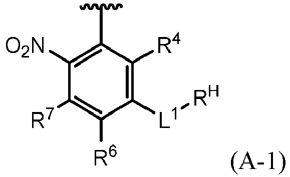
- R⁴, R⁵, R⁶ and R⁷ are each independently selected from hydrogen, -OR^{a}, -SR^{a}, - NR^{a}R^{a}, -OC(O)R^{b} and -NHC(O)R^{b}; preferably hydrogen;
- L¹ is a C₁ to C₃ alkylene group, a C₂ to C₃ alkenylene group or a C₂ to C₃ alkynylene group; or is a moiety of form: -A-B-, wherein A is an unsubstituted C₁ to C₃ alkylene group; and B is a cyclic group selected from triazole, benzene, cyclohexane, piperidine, pyridazine, pyridine, thiazole and imidazole;
- R^{H} is selected from C₄ to C₁₂ alkyl, C₄ to C₁₂ alkenyl and C₄ to C₁₂ alkynyl;
- R³ is methyl;
- each R² is independently hydrogen or methyl.

In a second preferred aspect,
- the compound of formula (I) is a compound of formula: 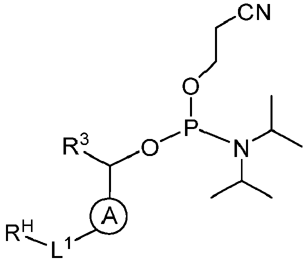
- Ⓐ, L¹ and R^{H} are together represented by formula (A-1): 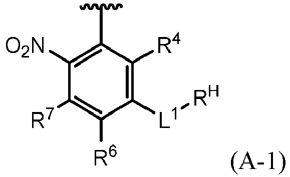 - R⁴, R⁵, R⁶ and R⁷ are each independently selected from hydrogen, -OR^{a}, -SR^{a}, - NR^{a}R^{a}, -OC(O)R^{b} and -NHC(O)R^{b}; preferably hydrogen;
- L¹ is a C₁ to C₃ alkylene group, a C₂ to C₃ alkenylene group or a C₂ to C₃ alkynylene group; or is a moiety of form: -A-B-, wherein A is an unsubstituted C₁ to C₃ alkylene group; and B is a cyclic group selected from triazole, benzene, cyclohexane, piperidine, pyridazine, pyridine, thiazole and imidazole;
- R^{H} is selected from C₄ to C₁₂ alkyl, C₄ to C₁₂ alkenyl and C₄ to C₁₂ alkynyl;
- R³ is methyl.

In a third preferred aspect,
- the compound of formula (I) is a compound of formula: 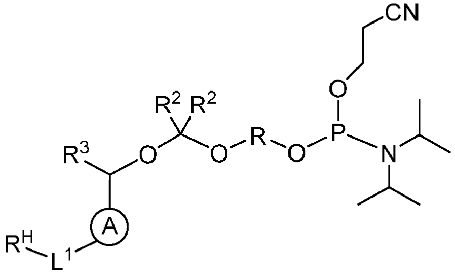
- Ⓐ, L¹ and R^{H} are together represented by formula (A-1): 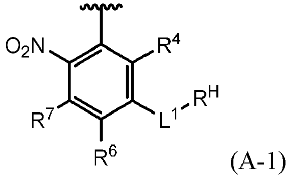 - R⁴, R⁵, R⁶ and R⁷ are each independently selected from hydrogen, -OR^{a}, -SR^{a}, - NR^{a}R^{a}, -OC(O)R^{b} and -NHC(O)R^{b}; preferably hydrogen;
- L¹ is a C₁ to C₃ alkylene group, a C₂ to C₃ alkenylene group or a C₂ to C₃ alkynylene group; or is a moiety of form: -A-B-, wherein A is an unsubstituted C₁ to C₃ alkylene group; and B is a cyclic group selected from triazole, benzene, cyclohexane, piperidine, pyridazine, pyridine, thiazole and imidazole;
- R^{H} is selected from C₄ to C₁₂ alkyl, C₄ to C₁₂ alkenyl and C₄ to C₁₂ alkynyl;
- R³ is methyl;
- each R² is independently hydrogen or methyl; and
- R is a nucleoside or a derivative or analogue thereof; or is a C₂ to C₆ alkylene group, a C₂ to C₆ alkenylene group and/or a C₂ to C₆ alkynylene group, wherein said alkylene, alkenylene or alkynylene group is optionally interrupted by and/or terminated in one or more groups selected from a heteroatom, a carbonyl group, a phenyl group, a cyclopentyl or cyclohexyl group, a 5- to 6-membered heteroaryl group and a 5- to 6-membered saturated or partially unsaturated heterocyclic group.

### Polynucleotide or analogue or derivative thereof

As explained herein, the provided methods comprise modifying a polynucleotide or analogue or derivative thereof by reaction with a compound of formula (I).

The polynucleotide or analogue or derivative thereof may be selected from any suitable natural, non-natural, functionalised and/or modified polynucleotides. Such derivatives and analogues of polynucleotides are well known in the art.

Polynucleotides and analogues thereof may be modified with protecting groups at any of their groups. In particular, it is common for polynucleotides to have protecting groups on their saccharide rings, nucleobases and/or phosphorous-based linkages. Commonly used protecting groups are well known in the art.

For example, the amine groups present in nucleobases (such as adenine, guanine, cytosine and derivatives and analogues thereof) may be protected by amine protecting groups known in the art, which include acyl groups such as acetyl, oxyacetyl (including phenoxyacetyl and t-butylphenoxyacetyl), propionyl, isobutyryl, benzoyl and benzylidene. Formamidine groups such as dimethylformamidine and di-N-butylformamidine groups may also be used.

Phosphate linkages may be protected by phosphate protecting groups such as ethyl or 2-cyanoethyl, and phosphite linkages may be protected by phosphite protecting groups such as ethyl or 2-cyanoethyl. For example, phosphodiester backbone linkages with a protecting group may be present as phosphotriesters, for example as 2-cyanoethyl phosphotriesters.

The compound of formula (I) may be reacted with a derivative of a polynucleotide comprising one or more monomers wherein the saccharide ring is a non-natural saccharide, such as arabinose or 'locked ribose', or a saccharide ring analogue such as an iminosugar, thiosugar or carbasugar ring, in which the oxygen atom usually present in a saccharide ring is replaced with a nitrogen atom, sulphur atom or methylene motif, respectively. 'Locked ribose' is a modified ribose moiety having an extra bridge connecting the 2' oxygen and the 4' carbon. An example of a locked ribose moiety is provided below.

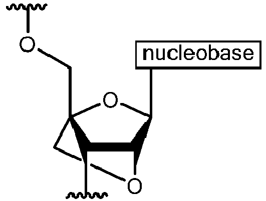

Nucleic acids containing 'locked ribose' saccharide rings are known as 'locked nucleic acids' or LNAs. LNAs have increased stability against enzymatic degradation and are a commercially important nucleic acid analogue of RNA and DNA.

The polynucleotide or analogue or derivative thereof modified in accordance with the disclosed methods preferably comprises monomer units wherein the saccharide rings are ribose or deoxyribose rings. The polynucleotide or analogue or derivative thereof is thus preferably a polyribonucleotide or a polydeoxyribonucleotide. Preferably the polynucleotide or analogue or derivative thereof is a polydeoxyribonucleotide, or a derivative or analogue thereof.

The polynucleotide or analogue or derivative thereof modified in accordance with the disclosed methods may comprise one or more phosphorous-based linkages between adjacent nucleoside monomer units which are selected from phosphodiester linkages, phosphotriester linkages such as alkyl phosphotriester linkages, phosphite-triester linkages such as alkyl phosphotriester linkages, phosphorothioate linkages, phosphorodithioate linkages and/or alkylphosphonate linkages such as methyl phosphonate. In particular, the phosphoramidite method is a common method of polynucleotide synthesis and initially produces polynucleotides in which the phosphorous-based linkage is a phosphite-triester linkage. Such phosphite-triester linkages may be oxidised to produce phosphotriester linkages, which may be deprotected to produce phosphodiester linkages.

### Cleavage of the photolabile group Ⓛ

The photolabile group Ⓛ of the compound of formula (I) may be cleaved by photolysis.

Typically, the photolabile group Ⓛ may be cleaved by photo-illuminating the compound of formula (I) using UV light. Accordingly, the photolabile group Ⓛ may be cleaved by photo-illuminating the photolabile group of the compound of formula (I) using UV light.

Typically, the UV light used to cleave the photolabile group has a wavelength of about 300 to about 500 nm, preferably about 325 to about 475 nm, more preferably about 350 to about 450 nm. For example, an UV lamp producing UV light of wavelength about 365 nm may be used.

Photoillumination may be carried out for any suitable time scale depending on the power of the light source used. For example, a power of from about 1 W to about 5000 W, e.g. from about 2 W to about 1000 W can be applied. Typically, the higher the power of the light source, the shorter the exposure time required. For example, a light source providing about 2 W to about 10 W such as from about 3 W to about 6 W e.g. about 4 or 5 W can be used to expose a sample in diionised water for about 0.5 hours to about 2 hours, e.g. about 40 to about 60 minutes, and may result in near quantitative cleagave. Alternatively, higher power light sources can be used to illuminate a sample for e.g. from about 1 minute to about 30 minutes. In general, a sample may be photoilluminated for any suitable time from about 1 minute to about 10 such as from about 10 minutes to about 1 hour e.g. for about 30 minutes. A mercury (Hg) lamp may be used to provide UV irradiation.

Selecting appropriate illumination conditions is routine for those skilled in the art. Appropriate illumination conditions can be determined by monitoring the extent of cleavage and selecting conditions which result in substantial or complete cleavage in the minimum time possible without causing damage to the synthesized polynucleotide, according to the equipment used. The product of the polynucleotide synthesis can be assessed using techniques such as e.g. NMR and mass spectroscopy (e.g. UPLC-MS) to monitor the reaction.

### Purification of modified polynucleotides

As will be apparent from the discussion herein, the modified polynucleotide or analogue or derivative thereof produced using the compound of formula (I) in accordance with the present disclosure may be more readily purified by separating the modified polynucleotide or analogue or derivative thereof from other contaminants based on its hydrophobicity.

Accordingly, provided herein is a method for purifying a polynucleotide or an analogue or derivative thereof, comprising:
(i) modifying a polynucleotide or analogue or derivative thereof in a reaction mixture as described herein, thereby increasing the hydrophobicity of the polynucleotide or analogue or derivative thereof; and
(ii) separating the modified polynucleotide or analogue or derivative thereof from other components in the reaction mixture according to the hydrophobicity of the modified polynucleotide or analogue or derivative thereof.

A preferred purification method is chromatography. Chromatography is a technique for the separation of the components in a mixture by differential adsorption onto an adsorbent surface (stationary phase). The components are then eluted by a solvent or mixture of solvents (mobile phase).

One simply chromatographic method is gel filtration. The primary means by which gel filtration can separate components in a mixture is based on molecular size. The modification of a polynucleotide or analogue or derivative thereof with a compound of formula (I) will increase the size of the polynucleotide or analogue or derivative thereof and so the modified polynucleotide or analogue or derivative thereof will have different migration properties through the gel matrix compared to unmodified contaminants such as truncated sequences. Accordingly, gel filtration may be used to purify the modified polynucleotide or analogue or derivative thereof from contaminants.

More typically, however, high-performance liquid chromatography (HPLC) is used to separate modified polynucleotides from unmodified contaminants and other components in a reaction mixture. HPLC systems typically consist of the following components: an injector, pumps for delivery of solvents through the column, an interchangeable HPLC column, a column oven, a solvent mixing system, and a detector (normally UV/visible), all controlled by a PC.

Typically, purification of a modified polynucleotide or analogue or derivative thereof according to the present disclosure may involve Reversed-phase HPLC (RPHPLC). Reversed-phase HPLC separates oligonucleotides and contaminants on the basis of differences in hydrophobicity. Typically, in RPHPLC, a mixture containing the desired modified polynucleotide or analogue or derivative thereof and other components such as unmodified truncated sequences and other impurities is applied to a column. The column typically comprises a porous silica support having pores of from about 10 Å to about 1000 Å, such as from about 100 Å to about 500 Å, e.g. from about 200 to about 400 Å such as about 300 Å. The support is typically modified with hydrocarbon groups, e.g. with C₁₋₃₀ alkyl, alkenyl and/or alkynyl chains, e.g. with C₅₋₂₀ alkyl, alkenyl and/or alkynyl chains. C₈ and C₁₈ alkyl, alkenyl and/or alkynyl chains are often used. Such a support is often referred to as a stationary phase.

The modified polynucleotide or analogue or derivative thereof may be eluted with a hydrophilic (typically aqueous) mobile phase. Typically, ammonium acetate (e.g. tetraethyl ammonium acetate, TEAA) may be used. The aqueous phase forces the modified oligonucleotide to interact strongly with the reversed-phase column. Further elution with a gradient of acetonitrile in aqueous ammonium acetate causes the individual components in the mixture to enter the mobile phase, which becomes more hydrophobic as the percentage of acetonitrile increases. Molecules with a greater degree of hydrophobicity elute more slowly, and this leads to separation of the components in the mixture.

The operating temperature can be controlled by the user in order to optimise the purification achieved, according to the identity and structure of the polynucleotide or analogue or derivative thereof. For example, secondary structure such as (e.g. hairpin loops) can cause an oligonucleotide to elute as a broad peak or even a series of peaks. In some embodiments, the HPLC column may be heated (e.g. at from about 30 to about 100 °C, e.g. from about 40 to about 80 °C, such as about 50°C to about 70 °C, e.g. about 60 °C) for a time from about 1 minute to about 1 hour to overcome this,

The operating pH and salt concentration can be adjusted by the user. For example, a pH of from about pH 4 to about pH 10 e.g. from about pH 6 to about pH 8 e.g. about pH 7 can be used. A salt concentration of from about 0.01 M to about 1 M e.g. from about 0.05 M to about 0.5 M e.g. about 0.1 M salt (e.g. from about 0.01 M to about 1 M e.g. from about 0.05 M to about 0.5 M e.g. about 0.1 M TEAA) can be used.

Other purification techniques which can be used to separate a modified polynucleotide or analogue or derivative thereof according to the present disclosure from other components in a reaction mixture include anion-exchange HPLC, fluorous affinity chromatography, and polyacrylamide gel electrophoresis (PAGE).

Selecting appropriate purification or separation techniques is routine for those skilled in the art. Appropriate conditions can be determined by monitoring purification profiles and selecting conditions which result in substantial or complete purification in the minimum time possible without causing damage to the synthesized polynucleotide, according to the equipment used. The product of the polynucleotide synthesis can be assessed using techniques such as e.g. NMR and mass spectroscopy (e.g. UPLC-MS) to monitor the reaction.

Optionally, said purification method further comprises removing the hydrophobic group from the modified polynucleotide or analogue or derivative thereof following the purification.

Accordingly, provided herein is a method for purifying a polynucleotide or an analogue or derivative thereof, comprising:
(i) modifying a polynucleotide or analogue or derivative thereof in a reaction mixture as described herein, thereby increasing the hydrophobicity of the polynucleotide or analogue or derivative thereof;
(ii) separating the modified polynucleotide or analogue or derivative thereof from other components in the reaction mixture according to the hydrophobicity of the modified polynucleotide or analogue or derivative thereof; and
(iii) optionally removing the hydrophobic group from the modified polynucleotide or analogue or derivative thereof by photo-illuminating the modified polynucleotide, analogue or derivative thereof, preferably by photo-illuminating the modified polynucleotide, analogue or derivative thereof using UV light at a wavelength of about 300 to about 500 nm.

### Method of producing a polynucleotide or analogue or derivative thereof.

Also provided is a first variation of a method of producing a polynucleotide or analogue or derivative thereof, comprising:
(i) providing a support-attached polynucleotide or derivative or analogue thereof,
(ii) optionally increasing the chain length of the support-attached nucleoside, nucleotide, polynucleotide or derivative or analogue thereof by introducing one or more additional nucleoside units, nucleotide units and/or analogues or derivatives thereof;
(x) modifying the polynucleotide or analogue or derivative thereof by reacting a reactive functional group of said polynucleotide or analogue or derivative thereof with a compound of formula (I) as described herein;
(iii) cleaving the polynucleotide or analogue or derivative thereof from the solid support;
(iv) purifying the modified polynucleotide or analogue or derivative thereof; and
(v) optionally cleaving the photolabile group of the compound of formula (I) by photo-illuminating the modified polynucleotide, analogue or derivative thereof.

Further provided is a second variation of said method, which is a method of producing a polynucleotide or analogue or derivative thereof, comprising:
(i) providing a support-attached polynucleotide or derivative or analogue thereof,
(ii) optionally increasing the chain length of the support-attached nucleoside, nucleotide, polynucleotide or derivative or analogue thereof by introducing one or more additional nucleoside units, nucleotide units and/or analogues or derivatives thereof;
(iii) cleaving the polynucleotide or analogue or derivative thereof from the solid support;
(x) modifying the polynucleotide or analogue or derivative thereof by reacting a reactive functional group of said polynucleotide or analogue or derivative thereof with a compound of formula (I) as described herein;
(iv) purifying the modified polynucleotide or analogue or derivative thereof; and
(v) optionally cleaving the photolabile group of the compound of formula (I) by photo-illuminating the modified polynucleotide, analogue or derivative thereof.

Step (i) of such methods may comprise providing a previously prepared support-attached polynucleotide or analogue or derivative thereof. Alternatively, step (i) may comprise attaching a polynucleotide or analogue or derivative thereof to a solid support.

Any suitable solid support may be used. Preferably, the solid support comprises a glass, silica, ceramic, or a polymeric resin. More preferably, the solid support comprises controlled pore glass (CPG) or polystyrene. CPG may be treated with an appropriate surface treatment to introduce one or more functional groups. Suitable surface treatments include silanes such as (3-aminopropyl)triethoxysilane to give aminopropyl CPG. Such functionalised CPG can be derivatised by reacting the amino moiety of the aminopropyl group with a suitable moiety such as an ester group. Suitable polystyrene materials are typically low-swellable and/or cross-linked. For example, polystyrene can be obtained by polymerization of divinylbenzene, styrene, and 4-chloromethylstyrene in the presence of a porogeneous agent. The macroporous chloromethyl MPPS obtained may be converted to aminomethyl MPPS. Typically the solid support is not functionalised with a protein such as streptavidin.

Preferably, the solid support comprises particles having a diameter of from about 1 µm to about 10000 µm, more preferably from about 5 µm to about 1000 µm, further preferably from about 10 µm to about 500 µm such as from about 50 to about 250 µm. Preferably, the solid support material particles are porous. Preferably, when the solid support material is porous the pore size is from about 1 to about 1000 nm e.g. from about 10 to about 100 nm such as about 50 nm.

Preferably, the support material is or comprises beads. Such beads may be magnetic e.g. may comprise a magnetic material. Beads may be ferrimagentic or paramagnetic. Beads may comprise an iron oxide core.

Preferably, the support material is contained in a column. The column may have a bed volume of from about 100 µL to about 100 L; e.g. from about 1 mL to about 1 L. Any suitable bed volume can be used depending on the scale of the synthesis envisaged.

Step (ii) of such methods typically comprises performing the following step one or more times: contacting the support-attached polynucleotide or analogue or derivative thereof with a nucleoside or a derivative thereof, thereby forming a phosphorous-based linkage.

The nucleoside or a derivative thereof may be a nucleoside phosphoramidite, a nucleoside H-phosphonate or a nucleoside phosphotriester, preferably an activated nucleoside phosphoramidite, an activated nucleoside H-phosphonate or an activated nucleoside phosphotriester. A nucleoside phosphoramidite may be activated by reaction with tetrazole or a derivative thereof as an activator; for example, a group of the phosphoramidite (such as a diisopropylamino group of the phosphoramidate) may be protonated by acidic tetrazole or a derivative thereof. A nucleoside H-phosphonate may be activated by reaction with a chlorinating agent such as pivaloyl chloride. A nucleoside nucleoside phosphotriester may be activated by reaction with a coupling agent such as 1-(mesitylsulfonyl)-3-nitro-1H-1,2,4-triazole.

Preferably, in step (ii) the nucleoside or a derivative thereof is an activated nucleoside phosphoramidite. Preferably, the activated nucleoside phosphoramidite has an activated phosphoramidite group at the 3' position or the 5' position. More preferably, the activated nucleoside phosphoramidite has an activated phosphoramidite group at the 3' position. Often, the activated nucleoside phosphoramidite with an activated phosphoramidite group at the 3' position has a hydroxyl protecting group at the 5' position. Often, the activated phosphoramidite group is represented by formula (PH-A): 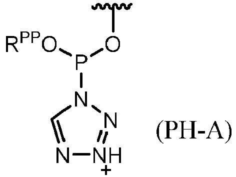 wherein R^{PP} is hydrogen, a lone pair of electrons resulting in a negative charge, o-chlorophenyl, p-chlorophenyl, a methyl group or a 2-cyanoethyl group; preferably a 2-cyanoethyl group; and

The phosphorous-based linkage may be a phosphodiester linkage, a phosphotriester linkage such as an alkyl phosphotriester linkage, a phosphite-triester linkage such as an alkyl phosphite-triester linkage, a phosphorothioate linkage, a phosphorodithioate linkage, or an alkylphosphonate linkage such as a methyl phosphonate linkage. Preferably, the phosphorous-based linkage is a phosphite-triester linkage. More preferably, the phosphorous-based linkage is a 2-cyanoethyl phosphite-triester linkage.

Preferably, step (ii) comprises performing the following steps one or more times: providing a nucleoside phosphoramidite; activating the nucleoside phosphoramidite; and contacting the support-attached nucleoside, nucleotide, polynucleotide, or derivative or analogue thereof with the activated nucleoside phosphoramidite, thereby forming a phosphite triester linkage.

The nucleoside phosphoramidite preferably has a phosphoramidite group at the 3' position or the 5' position. More preferably, the nucleoside phosphoramidite has a phosphoramidite group at the 3' position. Preferably, the nucleoside phosphoramidite with a phosphoramidite group at the 3' position has a hydroxyl protecting group at the 5' position. Preferably, the phosphoramidite group is represented by formula (PH): 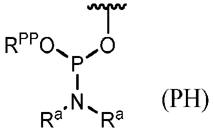 Wherein R^{PP} is hydrogen, a lone pair of electrons resulting in a negative charge, o-chlorophenyl, p-chlorophenyl, a methyl group or a 2-cyanoethyl group; preferably a 2-cyanoethyl group; and each R^{a} is H or C₁₋₃ alkyl.

Step (ii) may optionally further comprise oxidising the phosphite triester linkage to form a phosphotriester linkage.

Step (ii) may optionally further comprise removing a hydroxyl protecting group.

More preferably, step (ii) comprises performing the following steps (a) to (e) one or more times:
(a) providing a nucleoside having: (i) a phosphoramidite group at the 3' position and a hydroxyl protecting group at the 5' position;
(b) activating the nucleoside phosphoramidite of step (a);
(c) contacting the support-attached polynucleotide or analogue or derivative thereof with the activated nucleoside phosphoramidite, thereby forming a phosphite triester linkage;
(d) oxidising the phosphite triester linkage to form a phosphotriester linkage; and
(e) removing the hydroxyl protecting group from the terminal nucleoside of the resulting support-attached polynucleotide.

Step (b) typically comprises contacting the nucleoside phosphoramidite with a suitable activating agent. Any suitable activating agent can be used. Typically the activating agent is tetrazole or a derivative thereof. Typically the activating agent and nucleoside phosphoramidite are contacted in an organic solvent such as acetonitrile.

Step (c) may be conducted by flowing the activated nucleoside phosphoramidite of step (b) through a column containing the support-attached nucleoside, nucleotide, polynucleotide, or derivative or analogue thereof.

Step (d) typically comprises oxidising the phosphite triester using a suitable oxidising agent. Any suitable oxidising agent can be used. Typically the oxidising agent is iodine. Iodine may be used in reaction with suitable solvents e.g. with water and pyridine (e.g. at a concentration of about 0.01 to 0.02 M iodine in water/pyridine/THF 2/20/78).

Step (e) may be conducted by contacting the resulting support-attached polynucleotide of step (d) with a suitable reagent. Typically, deprotection comprises contacting the support-attached polynucleotide with concentrated aqueous base such as aqueous ammonia. The aqueous solution may be removed by evaporation.

Step (ii) may comprise repeating said reaction step(s) at least once, at least twice, at least 5 times, at least 10 times, at least 20 times, at least 30 times, at least 40 times, at least 50 times, at least 100 times, at least 150 times, at least 200 times, at least 500 times, at least 1000 times, or more. Accordingly, step (ii) may comprise forming a polynucleotide of length at least 2, at least 5, at least 10, at least 20, at least 30, at least 40, at least 50, at least 100, at least 150, at least 200, at least 500, at least 1000 monomer units, or more. Those skilled in the art will appreciate that at each repeat the nucleoside or derivative thereof attached to the support-attached nucleoside provided in step (i) may be the same or different. Accordingly, the sequence of the polynucleotide synthesized in the method may be determined by the user of the method, e.g. the operator of equipment configured for the synthesis of polynucleotides.

Step (x) comprises reacting the polynucleotide or analogue or derivative thereof either when attached to the support or after cleavage from the support, see below, with a compound of formula (I) thereby modifying the polynucleotide or analogue or derivative thereof. The reaction of the polynucleotide or analogue or derivative thereof with the compound of formula (I) typically proceeds as described or essentially as described according to step (ii) above.

Preferably, the disclosed method comprises one or more steps of deprotecting the polynucleotide or analogue or derivative thereof. Preferably, deprotecting the polynucleotide or analogue or derivative thereof comprises removing protecting groups from one or more nucleobases and/or one or more phosphorous-based linkages present in the support-attached polynucleotide. Deprotection may be achieved by treatment of the polynucleotide or analogue or derivative thereof with a suitable reagent depending on the protecting group used. For example, benzoyl, isobutryl and dimethylformamidyl protecting groups typically used to protect nucleobases can be removed using concentrated ammonium hydrocide. Ultramild protecting groups typically used to protect nucleobases such as phenoxyacetyl, acetyl and isopropyl phenyoxyacetyl groups can be removed using a methanolic solution of potassium carbonate, or a mixture of aqueous ammonia and aqueous methylamine. Cyanoethyl phosphotriester groups can be used to protect the phosphodiester backbone and can be removed using concentrated ammonium hydroxide. Phosphate groups can be protected as methyl triesters and removed using thiophenol.

In step (iii) the polynucleotide or analogue or derivative thereof is cleaved from the solid support. Typically the polynucleotide or analogue or derivative thereof is attached to the solid support using a succinyl linker which may be readily cleaved by treatment with concentrated ammonium hydroxide at room temperature for one hour

In step (iv) the modified polynucleotide or analogue or derivative thereof is purified. Typically the modified polynucleotide or analogue or derivative thereof is purified by being separated from other components in the reaction mixture, such as truncated polynucleotides, unreacted monomers, and removed protecting groups. The modified polynucleotide or analogue or derivative thereof is typically separated from such groups according to the hydrophobicity of the modified polynucleotide or analogue or derivative thereof. The modified polynucleotide or analogue or derivative thereof is typically separated from other components in the reaction mixture by chromatography, e.g. by high performance liquid chromatography (HPCL). The modified polynucleotide or analogue or derivative thereof may be purified by reversed phase (RP) HPLC as described in more detail herein.

In optional step (v) the modified polynucleotide or analogue or derivative thereof is subjected to photolysis to cleave the hydrophobic modification R^{H} from the purified polynucleotide or analogue or derivative thereof. Photo cleavage typically comprises photo-illuminating the modified polynucleotide or analogue or derivative thereof. Accordingly photolytic cleavage typically involves photo-illuminating the photolabile group of the compound of formula (I). Typically the modified polynucleotide or analogue or derivative thereof is photoilluminated using ultraviolet light as described herein.

The methods disclosed herein can be conducted at any appropriate temperature and under routine solvent conditions that are readily accessible to those skilled in the art. Typically, reactions are conducted at from about 10 °C to about 100 °C, such as from about 20 °C to about 50 °C such as from about 25 °C to about 30 °C. Often reactions can be conducted at room temperature.

### Products of the disclosed methods

The disclosed methods may be used to produce a modified polynucleotide or an analog or derivative thereof. Accordingly, also provided herein is a polynucleotide or derivative or analog thereof obtainable or obtained by a method provided herein.

The term "polynucleotide", when referring to a polynucleotide which is or has been modified in accordance with the present disclosure, according to the context, refers to a single or double stranded covalently-linked sequence of nucleotides in which the 3' and 5' ends on each nucleotide are joined by phosphorous-based linkages. The phosphorous-based linkages present in products of the disclosed methods may be phosphodiester bonds, as in natural polynucleotides, or may be non-natural phosphorous-based bonds such as: phosphotriester linkages, for example, alkyl phosphotriester linkages such as methyl phosphotriester linkages and ethyl phosphotriester linkages; phosphorothioate linkages; phosphorodithioate linkages; and alkylphosphonate linkages such as methyl phosphonate linkages.

A polynucleotide may be made up of deoxyribonucleotide bases or ribonucleotide bases. Nucleic acids may further include modified DNA or RNA, for example DNA or RNA that has been methylated, or RNA that has been subject to post-translational modification, for example 5'-capping with 7-methylguanosine, 3'-processing such as cleavage and polyadenylation, and splicing. Nucleic acids may also include synthetic nucleic acids (XNA), such as hexitol nucleic acid (HNA), cyclohexene nucleic acid (CeNA), threose nucleic acid (TNA), glycerol nucleic acid (GNA), locked nucleic acid (LNA) and peptide nucleic acid (PNA). Sizes of nucleic acids, also referred to herein as "polynucleotides" are typically expressed as the number of base pairs (bp) for double stranded polynucleotides, or in the case of single stranded polynucleotides as the number of nucleotides (nt). One thousand bp or nt equal a kilobase (kb). Polynucleotides of less than around 40 nucleotides in length are typically called "oligonucleotides" and may comprise primers for use in manipulation of DNA such as via polymerase chain reaction (PCR).

A polynucleotide may comprise any combination of any nucleotides. The nucleotides can be naturally occurring or artificial. One or more nucleotides in the polynucleotide can be oxidized or methylated. One or more nucleotides in the polynucleotide may be damaged. For instance, the polynucleotide may comprise a pyrimidine dimer. Such dimers are typically associated with damage by ultraviolet light and are the primary cause of skin melanomas. One or more nucleotides in the polynucleotide may be modified, for instance with a label or a tag, for which suitable examples are known by a skilled person. The polynucleotide may comprise one or more spacers. A nucleotide typically contains a nucleobase, a sugar and at least one phosphate group as described in more detail herein. The nucleobase and sugar form a nucleoside. The nucleobase is typically heterocyclic. Nucleobases include, but are not limited to, purines and pyrimidines and more specifically adenine (A), guanine (G), thymine (T), uracil (U) and cytosine (C). The sugar is typically a pentose sugar. Nucleotide sugars include, but are not limited to, ribose and deoxyribose. The sugar is preferably a deoxyribose. The polynucleotide preferably comprises the following nucleosides: deoxyadenosine (dA), deoxyuridine (dU) and/or thymidine (dT), deoxyguanosine (dG) and deoxycytidine (dC). The nucleotide is typically a ribonucleotide or deoxyribonucleotide. The nucleotide typically contains a monophosphate, diphosphate or triphosphate. The nucleotide may comprise more than three phosphates, such as 4 or 5 phosphates. Phosphates may be attached on the 5' or 3' side of a nucleotide. The nucleotides in the polynucleotide may be attached to each other in any manner. The nucleotides are typically attached by their sugar and phosphate groups as in nucleic acids. The nucleotides may be connected via their nucleobases as in pyrimidine dimers.

Nucleotides comprised in the polynucleotide may include, but are not limited to, adenosine monophosphate (AMP), guanosine monophosphate (GMP), thymidine monophosphate (TMP), uridine monophosphate (UMP), 5-methylcytidine monophosphate, 5-hydroxymethylcytidine monophosphate, cytidine monophosphate (CMP), cyclic adenosine monophosphate (cAMP), cyclic guanosine monophosphate (cGMP), deoxyadenosine monophosphate (dAMP), deoxyguanosine monophosphate (dGMP), deoxythymidine monophosphate (dTMP), deoxyuridine monophosphate (dUMP), deoxycytidine monophosphate (dCMP) and deoxymethylcytidine monophosphate. Nucleotides are preferably selected from AMP, TMP, GMP, CMP, UMP, dAMP, dTMP, dGMP, dCMP and dUMP. A nucleotide may be abasic (i.e. lack a nucleobase). A nucleotide may also lack a nucleobase and a sugar (i.e. may be a C3 spacer).

Typically the polynucleotide consists or comprises DNA and/or RNA, preferably DNA.

The polynucleotide can be at least 10, at least 50, at least 100, at least 150, at least 200, at least 250, at least 300, at least 400 or at least 500 nucleotides or nucleotide pairs in length.

### Populations of polynucleotides

Also provided is a population of a purified polynucleotide or analogue or derivative thereof. The population has improved properties compared to populations of polynucleotides produced by prior techniques. Such advantages arise when the polynucleotide is used in high-tech applications including pharmaceuticals (e.g. for gene therapy) and in other applications of gene synthesis. It is particularly important to eliminate as far as possible any chance of contamination arising from incorporation of impurities into genetic constructs, e.g. arising from truncation impurities. However, present methods of purifying polynucleotides are incapable of providing the very high purity levels required for these applications. This is particularly the case when the desired polynucleotide is modified with a modifying group such as a group R as defined herein. In such cases, there are essentially two options. If the modification is undertaken before purification then limitations in purification techniques means that a homogeneous population often cannot be generated. If purification is undertaken prior to modification then inefficiencies in modification chemistries means that a homogeneous population again often cannot be generated.

Accordingly the disclosure provides a homogeneous population comprising a plurality of polynucleotides or analogues or derivatives thereof; e.g. comprising a plurality of modified polynucleotide or analogues or derivatives thereof. The population may have a purity of at least 90%, e.g. at least 91%, e.g. at least 92%, at least 93%, at least 94%, at least 95%, at least 96%, at least 97%, at least 98%, at least 99%, at least 99.9% or at least 99.99% or more.

The disclosure also provides a homogeneous population comprising a plurality of modified polynucleotides or analogues or derivatives thereof; wherein at least 90% of the monomers in the population are chemically modified with a group R as defined herein. In some embodiments at least 90%, e.g. at least 91%, e.g. at least 92%, at least 93%, at leat 94%, at least 95%, at least 96%, at least 97%, at least 98%, at least 99%, at least 99.9% or at least 99.99% or more of the monomers in the population are modified with the modifying group. In some embodiments 100% of the monomers in the population are modified with the modifying group. As explained above, previously known methods are typically not capable of providing such homogeneous populations as modification chemistry is typically not 100% efficient, and modified monomers typically cannot be readily separated or purified from impurities.

### Applications of polynucleotides produced by the disclosed methods

The polynucleotides modified and/or purified in accordance with the present disclosure have a multitude of potential applications. The applications are not particularly limited and the polynucleotides provided here and by the methods disclosed herein can be used in any application for which polynucleotides are required.

In some embodiments the methods provided herein are capable of providing polynucleotides with a very high degree of purity. Accordingly, the polynucleotides provided herein are particularly useful in applications wherein high purity is necessary. Preferably, the purity of the polynucleotides obtained in the disclosed methods is at least 99%, such as at least 99.9%, e.g. at least 99.99% more preferably at least 99.999% or more.

One application of the polynucleotides provided herein is in PCR (polymerase chain reaction). Methods for conducting PCR are well known to those skilled in the art. For example, in one embodiment conducting PCT comprises contacting primers complementary to a target polynucleotide with the target polynucleotide under conditions such that a polymerase can catalyse chain extension thereby amplifying the target polynucleotide. The polynucleotides provided herein are particularly useful as primers for PCR due to their high degree of purity.

Another application of the polynucleotides provided herein is in the preparation of therapeutic polynucleotides. Therapeutic polynucleotides are being investigated for use in treating various different pathological conditions and have particular applications as anticancer medications and as antivirals.

Further applications of the polynucleotides provided herein include in next generation sequencing technologies. For example, the polynucleotides provided herein can be used as barcodes in single cell sequencing techniques such as drop-seq sequencing.

In drop-seq techniques, a microfluidic device is used to create an emulsion of water droplets in oil in which each water droplet contains at most a single biological cell (e.g. a mammalian e.g. human cell or a bacterial cell) and a barcoded solid support, such as a barcoded bead, which is attached to a polynucleotide sequence defining a polynucleotide "barcode". The polynucleotides provided herein may be used in this manner.

In more detail, support-attached polynucleotides for use in drop-seq sequencing typically comprise a molecular index for identifying cellular target polynucleotides; a polynucleotide "barcode" and a universal sequence. The support-attached polynucleotides typically comprises a 3'-poly T end - i.e. at the 3' ends of the polynucleotides, there are several e.g. from about 5 to about 50 such as from about 10 to about 40 e.g. about 30 nucleotide units in a row having thymine as their nucleobase. The molecular index may comprise from about 5 to about 15 nucleotides. The barcode may comprise from about 4 to about 50 such as from about 5 to about 30 e.g. about 6 to about 20 e.g. about 8 to about 15 monomer units. The universal sequence can be of any desired length.

The cells typically contain mRNA having a 5'-poly A region - i.e. at the 5' end of the mRNA there are several nucleotides in a row having adenine as their nucleobase. Typically, in the drop-seq technique, the cells are lysed and their mRNA is released into their respective droplets. The 5'-poly A regions of the mRNA strands hybridise to the 3'-poly T ends of the support-attached polynucleotides, thereby capturing the mRNA strands from a single cell onto a single solid support. The resulting support-attached polynucleotide-mRNA assemblies are often referred to as 'STAMPs' - Single cell Transcriptomes Attached to MicroParticles. The emulsion may then be then disrupted, releasing the STAMPS, which may be used to create a transcriptome library. The STAMPS can be identified by the barcode on the solid support, and can undergo reverse transcription, PCR and sequencing to determine the mRNA strand sequences.

Support-attached polynucleotides with 3'-poly T ends used in conventional drop-seq techniques may be synthesised directly on the solid support using solid-phase polynucleotide synthesis, e.g. in the 3' to 5' direction and the chemistry at the 5' end may be altered by modification herein. Alternatively a polynucleotide may be synthesized as described herein and then coupled to a solid support for use in a drop-seq sequencing method. Still alternatively the polynucleotide may be synthesized in the 5' to 3' direction, in order to yield free 3' ends available for hybridising with mRNA strands. The chemistry at the 3' end may be altered by modification herein. Alternatively a polynucleotide may be synthesized as described herein and then coupled to a solid support for use in a drop-seq sequencing method. The subsequent reverse transcription and PCR steps can then be performed 'in droplet' to sequence the mRNA chains present in each of the individual water droplets.

Accordingly, provided herein is a method of sequencing a polynucleotide expressed by a cell, the method comprising providing a support attached polynucleotide, wherein the polynucleotide is modified as described herein; contacting said support attached polynucleotide with a target polynucleotide under conditions such that the support attached polynucleotide hybridises to the target polynucleotide; enzymatically synthesizing a complement polynucleotide strand complementary to the target polynucleotide; and sequencing the synthesized complement polynucleotide strand or a polynucleotide strand complementary thereto, thereby determining the sequence of the target polynucleotide.

### Further aspects of the invention

As discussed in more detail herein, the present invention provides methods comprising the use of a compound of formula (I). The present invention also provides compounds of formula *(I) per se.*

Accordingly, provided herein is a compound of formula (I): 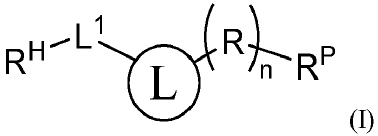 wherein R^{H}, L¹, Ⓛ, R, n and R^{P} are as defined herein.

As will be apparent, this compound is useful in the methods provided herein.

Also provided is a modified polynucleotide or a derivative or analogue thereof, comprising a group of formula (I^{∗}): 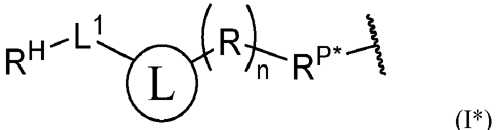 wherein R^{H}, L¹, Ⓛ, R and n are as defined herein, and wherein:
- R^{P∗} is a phosphorous-based linkage; and
- the wavy line indicates the point of attachment to the polynucleotide or derivative or analogue thereof.

Typically, R^{P∗} is a phosphodiester linkage, a phosphotriester linkage, a phosphite-triester linkage, a phosphite-diester linkage, a phosphorothioate linkage, a phosphorodithioate linkage, an alkylphosphonate linkage or an alkylphosphonite linkage. More typically, R^{P∗} is a phosphite-triester linkage or an alkylphosphonite linkage or a phosphodiester linkage or an alkylphosphonate linkage. More typically, R^{P∗} is a phosphite-triester linkage or an alkylphosphonite linkage. Still more typically, R^{P∗} is a phosphite-triester linkage or a methylphosphonite linkage.

Typically, the group of formula (I^{∗}) is attached at the 3' position or the 5' position of the polynucleotide or analogue or derivative thereof which is thereby modified with said group. Most often, the group of formula (I^{∗}) is attached at the 5' position of the polynucleotide or analogue or derivative thereof. Typically, the group of formula (I^{∗}) is attached via a bond to an oxygen atom at the 5' position of the polynucleotide or analogue or derivative thereof.

Also provided herein is the use of a compound of formula (I) 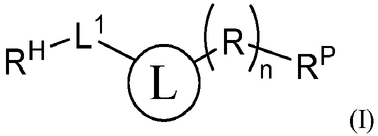 wherein R^{H}, L¹, Ⓛ, R, n and R^{P} are as defined herein; for modifying a polynucleotide or a derivative or analogue thereof. Typically, said use is for increasing the hydrophobicity of the polynucleotide or a derivative or analogue thereof.

Also provided is the use of a compound of formula (I) as defined herein for purifying a polynucleotide or a derivative or analogue thereof. Typically said use is for purifying a polynucleotide or a derivative or analogue thereof by chromatography based on the hydrophobicity of the modified polynucleotide or analogue or derivative thereof, e.g. by HPLC such as via RPHPLC as described in more detail herein.

### Further embodiments of the invention

Further provided is an apparatus for performing polynucleotide synthesis, the apparatus comprising:
- a solid support attached to a polynucleotide; wherein the polynucleotide is modified with a compound of formula (I) as defined herein; and
- a light source for illuminating the solid support thereby cleaving the photolabile group of the compound of formula (I).

Preferably the solid support, light source and photocleavable linker are as defined herein.

Further provided is a system comprising:
- a solid support attached to a polynucleotide having a reactive functional group for reaction with a compound of formula (I);
- a compound of formula (I) for reaction with the polynucleotide;
- a light source for illuminating the solid support thereby cleaving the photolabile group of the compound of formula (I); and

Preferably the solid support, light source and photocleavable linker are as defined herein.

### Methods

The compounds of the invention can be prepared by any suitable method. Detailed general synthetic routes for representative compounds of the invention are set out below and in the Examples. Those skilled in the art will be readily able to adapt the detailed syntheses provided herein to prepare other compounds disclosed herein. However, by way of summary, the compounds provided herein can typically be prepared in a reaction according to one of the following schemes:

Exemplary compounds of formula (I) which are compounds of formula (II-3) can typically be prepared according to the following reaction scheme:

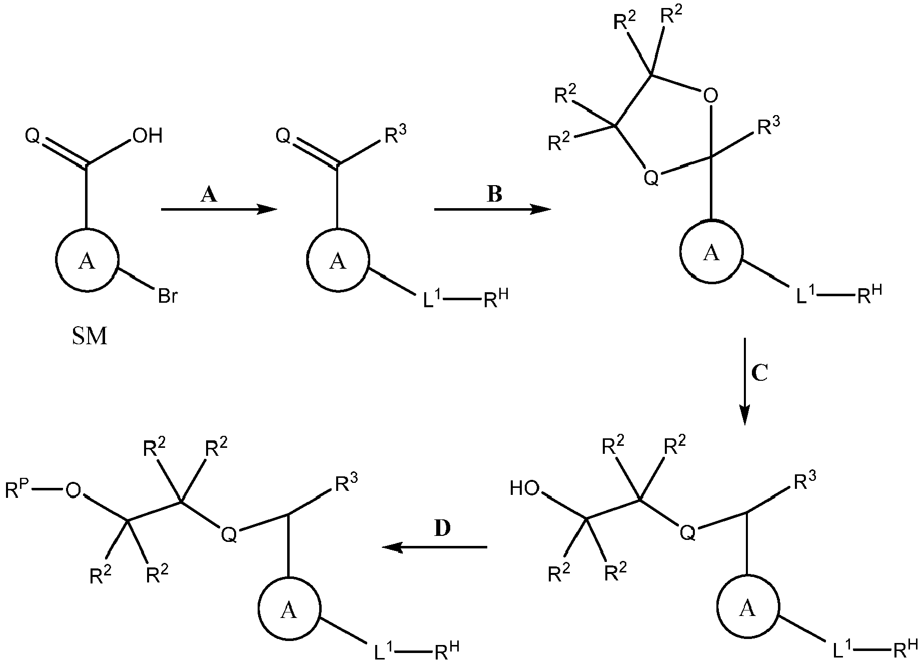

Starting material SM is readily available. A: Reaction of SM with e.g. L¹-R^{H} (e.g. a compound such as 1-octyne can be used to provide compounds wherein L¹ is C₂ alkynylene and R^{H} is C₆ alkyl). An ethylene glycol derivative yields the cyclic adduct in step B with hydride donation e.g. by sodium borohydride leading to the decyclised compound in step C. Reaction of the terminal hydroxy group with a phosphorus centre in R^{P} (e.g. bis(diisopropylamino)(methyl)phosphine) yields the final compound.

Exemplary compounds of formula (I) which are compounds of formula (II-1) can typically be prepared according to the following reaction scheme:

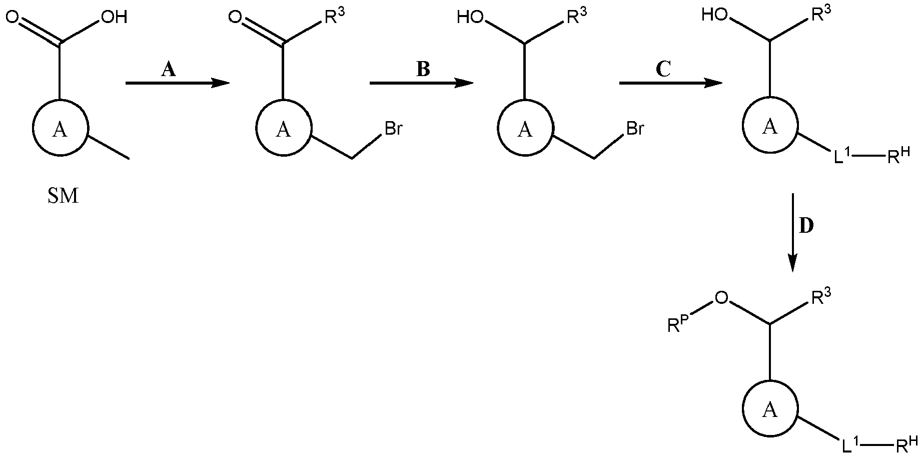

SOCl₂ activates the carboxylic acid for reaction with e.g. a Grignard derivative of R³ before e.g. NBS is used to functionalise the alkyl on the (A) group. Other functionalisation chemistry is readily available. B: Reduction of the carboxylic acid can achieved using e.g. NaBH₄ to yield the alcohol. C: Reaction with L¹-R^{H} or a precursor can be achieved, e.g. by reaction using NaN₃ to convert the bromine to an azide for conversion to L¹-R^{H} e.g. via routine nucleophilic substitution. In step D reaction of the terminal hydroxy group with a phosphorus centre in R^{P} (e.g. 2-Cyanoethoxy-*N*,*N-*diisopropylaminochlorophosphine) yields the final compound.

Exemplary compounds of formula (I) which are compounds of formula (II-2) can typically be prepared according to the following reaction scheme:

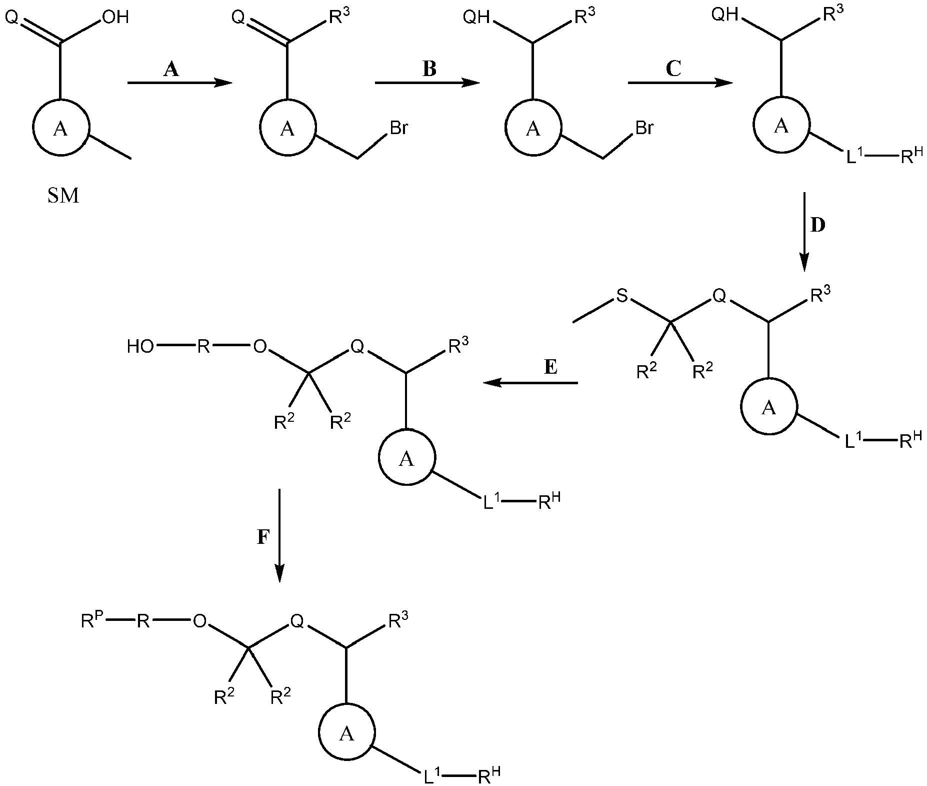

SOCl₂ activates the carboxylic acid for reaction with e.g. a Grignard derivative of R³ before e.g. NBS is used to functionalise the alkyl on the (A) group. Other functionalisation chemistry is readily available. B: Reduction of the carboxylic acid can achieved using e.g. NaBH₄ to yield the alcohol (when Q is O). Standard conversion can be used to convert the alcohol to other functional groups when Q is other than O. C: Reaction with L¹-R^{H} or a precursor can be achieved, e.g. by reaction using NaN₃ to convert the bromine to an azide for conversion to L¹-R^{H} e.g. via routine nucleophilic substitution. In step D reaction with a sulfur-containing CR²₂-derivative installs a reactive group for (E) reaction with an R derivative such as an HO-R-OH moiety. F: Reaction of the terminal hydroxy group with a phosphorus centre in R^{P} (e.g. 2-Cyanoethoxy-*N*,*N-*diisopropylaminochlorophosphine) yields the final compound.

Detailed synthetic routes to exemplary compounds of the invention are set out below.

It is to be understood that although particular embodiments, specific configurations as well as materials and/or molecules, have been discussed herein for methods according to the present invention, various changes or modifications in form and detail may be made without departing from the scope and spirit of this invention. The following examples are provided to better illustrate particular embodiments, and they should not be considered limiting the application. The application is limited only by the claims.

### EXAMPLES

### Experimental Procedures

All reagents were purchased from Sigma-Aldrich, Alfa Aesar, Fisher Scientific, Fluorochem, Carbosynth, TCI or Link Technologies and used without further purification. Anhydrous pyridine, triethylamine, diisopropylethylamine and dichloromethane were freshly distilled over calcium hydride. All air/moisture sensitive reactions were carried out under argon in oven-dried glassware. Reactions were monitored by thin layer chromatography using Merck Silica gel 60 F₂₅₄ aluminium backed plates. The compounds were visualized by UV irradiation at 254/260 nm and by staining with an appropriate solution (p-anisaldehyde, potassium permanganate, ninhydrin, Mary's reagent). Flash chromatography was carried out using a Biotage Isolera on KP-Sil or Sfa Duo cartridges or manually with Merck Silicagel 60 (40-63 µm). NMR spectra were measured on a Bruker AVII400 FT-NMR Spectrometer or a Bruker AVIIIHD400 FT-NMR Spectrometer. Chemical shifts are given in ppm and were internally referenced to the appropriate solvent signal. Coupling constants (*J*) are given in Hertz (Hz) and assignment was aided by COSY, ¹³C, HMBC, DEPT experiments. Chemical names were generated using ChemDraw (Perkin Elmer).

### Example 1: Synthesis of 5'-PC PO₃

This example describes the synthesis of a compound of Formula (I) which is a compound of formula (II-1).

### 1-(5-Methyl-2-nitrophenyl)ethan-1-one

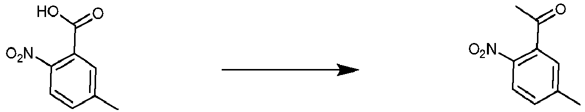

To a solution of 5-methyl-2-nitrobenzoic acid (5 g, 27.6 mmol) in anhydrous toluene (30 mL) was added thionyl chloride (6 mL, 82.8 mmol) and the mixture refluxed at 80 °C under argon for 3 hours. The mixture was concentrated to give 5-methyl-2-nitrobenzoyl chloride as a yellow oil (5.5 g) which was taken forward without further purification.

To a suspension of anhydrous magnesium chloride (2.36 g, 24.8 mmol) and diethyl malonate (5 mL, 33 mmol) in anhydrous ethyl acetate (80 mL) was added anhydrous triethylamine (16 mL) slowly at 0 °C. After stirring for 45 minutes, a solution of 5-methyl-2-nitrobenzoyl chloride (5.5 g) in anhydrous ethyl acetate (10 mL) was added. The mixture was the heated to 60 °C and stirred for 30 mins. Once complete, the reaction was extracted with ethyl acetate, washed with dilute hydrochloric acid (1 M), water, dried over anhydrous sodium sulfate, filtered and concentrated in *vacuo.* A solution of glacial acetic acid (30 mL) and conc. sulfuric acid (6 mL) was added and the mixture was refluxed for 12 hours. The reaction mixture was cooled in an ice bath and made alkaline with sodium hydroxide, the product extracted with dichloromethane before purification by flash chromatography (0-50% ethyl acetate in hexane) to give the title compound (3.79 g, 21.2 mmol, 77%).

¹H NMR (400 MHz, CDCl₃) δ ppm 8.03 (d, 1H, *J=* 8.4 Hz, CH Ar), 7.38 (m, 1H, CH Ar), 7.18 (m, 1H, CH Ar), 2.54 (s, 1H, CH₃CO), 2.48 (s, 1H, CH₃).

### 1-(5-(Bromomethyl)-2-nitrophenyl)ethan-1-one

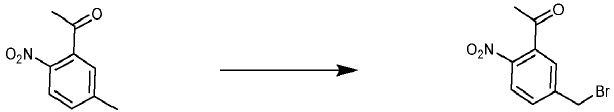

To a solution of 1-(5-methyl-2-nitrophenyl)ethan-1-one (3.79 g, 21.2 mmol) in anhydrous acetonitrile (25 mL) was added *N*-bromosuccinimide (4.14 g, 23.3 mmol) and 1,1'-Azobis(cyclohexanecarbonitrile) (0.52 g, 2.12 mmol) and the mixture refluxed for 12 hours. After cooling to room temperature, the solvent was removed and the residue dissolved in toluene (25 mL) and filtered. The filtrate was concentrated and purified by flash chromatography (0-50% ethyl acetate in hexane) to give the title compound (3.74 g, 14.5 mmol, 68%).

¹H NMR (400 MHz, CDCl₃) δ ppm 8.09 (d, 1H, *J=* 8.4 Hz, CH Ar), 7.62 (dd, 1H, *J* = 8.5, 2.0 Hz, CH Ar), 7.43 (d, 1H, *J* = 2.0 Hz, CH Ar), 4.50 (s, 2H, CH₂Br), 2.57 (s, 1H, CH₃CO).

### 1-(5-(Bromomethyl)-2-nitrophenyl)ethan-1-ol

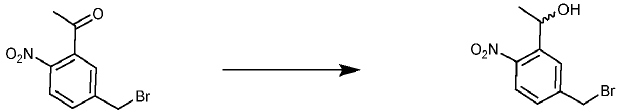

1-(5-(bromomethyl)-2-nitrophenyl)ethan-1-one (3.74 g, 14.5 mmol) was dissolved in mixture of anhydrous methanol (38 mL) and anhydrous dioxane (25 mL), whereupon sodium borohydride (0.822 g, 21.7 mmol) was added slowly at 0 °C, before allowing it to warm to room temperature over 12 hours. Water (60 mL) and 1M hydrochloric acid (15 mL) were added and the suspension extracted with ethyl acetate, dried over anhydrous sodium sulfate, filtered and concentrated before purification by flash chromatography (0-50% ethyl acetate in hexane) to give the title compound (3.38 g, 13.0 mmol, 90%).

¹H NMR (400 MHz, CDCl₃) δ ppm 7.91 (d, 1H, *J=* 8.4 Hz, CH Ar), 7.87 ppm (d, 1H, *J=* 2.0 Hz, CH Ar), 7.45 (m, 1H, CH Ar), 5.46 (q, 1H, *J=* 6.4 Hz, CH₃CH), 4.51 (s, 1H, CH₂Br), 2.27 (br. s, 1H, OH), 1.58 (d, 3H, *J=* 6.4 Hz, CH₃CH)

### 1-(5-(Azidomethyl)-2-nitrophenyl)ethan-1-ol

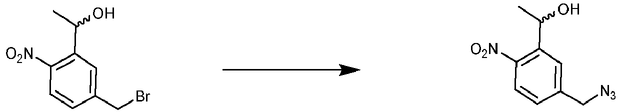

To a solution of 1-(5-(bromomethyl)-2-nitrophenyl)ethan-1-ol (2.49 g, 9.57 mmol) in anhydrous dimethyl formamide (30 mL) was added sodium azide (0.93 g, 14.4 mmol), and the mixture stirred for 3 hours at room temperature. The mixture was concentrated and then extracted from ethyl acetate with water, dried over anhydrous sodium sulfate, filtered and concentrated before purification by flash chromatography (0-50% ethyl acetate in hexane) to give the title compound (1.95 g, 8.77 mmol, 92%).

¹H NMR (400 MHz, CDCl₃) δ ppm 7.95 (d, 1H, *J=* 8.4 Hz, CH Ar), 7.81 (d, 1H, *J* = 2.0 Hz, CH Ar), 7.39 (dd, 1H, *J* = 8.4, 2.0 Hz, CH Ar), 5.48 (q, 1H, *J=* 6.4 Hz, CH₃CH), 4.49 (s, 2H, CH₂N₃), 2.17 (br. s, 1H, OH), 1.59 (d, 3H, *J=* 6.4 Hz, CH₃CH)

### 1-(5-(4-(Hexyl)-1h-1,2,3-triazol-1-ylmethyl)-2-nitrophenyl)ethan-1-ol

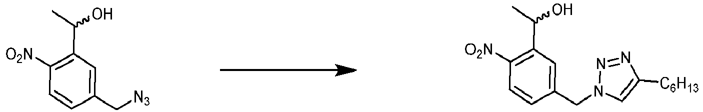

1-(5-(azidomethyl)-2-nitrophenyl)ethan-1-ol ( 0.95 g, 4.3 mmol) and 1-octyne (0.96 mL, 6.5 mmol) were added to a 3:1:1 mixture of tetrahydrofuran/*t*ert-butanol/water (54mL/ 18 mL/ 18 mL). A 7.5% solution of copper(II) sulfate in water (8.8 mL) and a 1M solution of (+)-sodium L-ascorbate in water (9.7 mL) were added and the reaction stirred at room temperature for 2 hours. The mixture was diluted with ethyl acetate and the organic phase washed with saturated sodium bicarbonate and brine (diluted with water by 10%), dried over anhydrous sodium sulfate filtered and concentrated. The crude was purified by flash chromatography (0-100% ethyl acetate in hexane ) to give the title compound (1 g, 3 mmol, 70%).

¹H NMR (400 MHz, CDCl₃) δ ppm 7.9 (d, 1H, *J=* 8.3 Hz, CH Ar), 7.78 (d, 1H, *J* = 2.0 Hz, CH Ar), 7.29 (br s, 1H, CH triazole), 7.2 (dd, 1H, *J=* 8.3 Hz, 2.0 Hz, CH Ar), 5.59 (s, 2H, CH₂N), 5.46 (q, 1H, *J=* 6.4 Hz, CHCH₃), 2.7 (t, 2H, J= 7.7 Hz, triazole CH₂CH₂), 1.71-1.62 (m, 2H, triazole CH₂CH₂), 1.56 (d, 3H, *J=* 6.4 Hz, CHCH₃), 1.40-1.25 (m, 6H, CH₂CH₂CH₂CH₃), 0.91-0.84 (m, 3H, CH₂CH₂CH₂CH₃)

### 2-Cyanoethyl (1-(5-((4-hexyl-1H-1,2,3-triazol-1-yl)methyl)-2-nitrophenyl)ethyl) diisopropylphosphoramidite

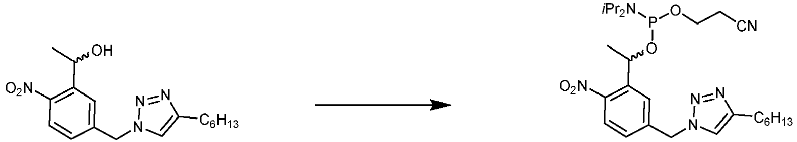

A solution of 1-(5-(4-(hexyl)-1h-1,2,3-triazol-1-ylmethyl)-2-nitrophenyl)ethan-1-ol (0.43 g, 1.3 mmol) in anhydrous dichloromethane (10 mL) was degassed under argon for 5 minutes before addition of anhydrous diisopropylethylamine (0.68 mL, 3.9 mmol). 2-Cyanoethoxy-*N*;*N*-diisopropylaminochlorophosphine (0.35 mL, 1.55 mmol) was added drop-wise and the reaction stirred at rt for 1 hour. The reaction mixture was diluted with anhydrous dichloromethane (2 x 20 mL) and washed with degassed saturated potassium chloride (30 mL), passed through anhydrous sodium sulfate, concentrated and dried under high vacuum. The crude product was purified by flash chromatography (80% ethyl acetate in (0.2% pyridine in hexane) under argon to obtain the title compound (0.6 g, 1.13 mmol, 87%) as a colourless oil, which was dissolved in degassed anhydrous acetonitrile and filtered through a 0.45 µm syringe filter before concentrating and aliquoting.

¹H NMR (400 MHz, CD₃CN) δ ppm 7.89 (t, 1H, *J* = 8.5 Hz, CH Ar), 7.72 (d, 0.5H, *J=* 2.0 Hz, CH Ar), 7.67 (d, 0.5H, *J=* 2.1 Hz, CH Ar), 7.57 (s, 0.5H, CH triazole), 7.55 (s, 0.5H, CH triazole), 7.33 (dd, 1H, J= 8.4Hz, 2.0 Hz, CH Ar), 5.62-5.58 (m, 2H, CH₂N), 5.48-5.36 (m, 1H, CHCH₃), 3.85-3.40 (m, 4H, CH₂CH₂CN, 2 x CH(CH₃)₂), 2.69-2.63 (m, 3H, CH₂CH₂CN, triazole CH₂CH₂), 2.53 (t, 1H, *J* = 5.8 Hz, triazole CH₂CH₂), 1.67-1.58 (m, triazole CH₂CH₂), 1.52 (t, 3H, *J* = 6.4 Hz, CHCH₃), 1.37-1.26 (m, 6H CH₂CH₂CH₂CH₃), 1.17-1.09 (m, 9H, 2 x CH(CH₃)₂), 0.9-0.85 (m, 3H, CH₂CH₂CH₂CH₃), 0.83 (d, 3H, *J* = 6.7 Hz, 2 x CH(CH₃)₂).

³¹P {¹H} NMR (162 MHz, CD₃CN) δ ppm 148.5, 148.3

### Example 2: Synthesis of 5'-PC C3

This example describes the synthesis of a compound of Formula (I) which is a compound of formula (II-2).

### ((1-(5-(4-(Hexyl)-1h-1,2,3-triazol-1-ylmethyl)-2-nitrophenyl)ethoxy)methyl) (methyl)sulfane

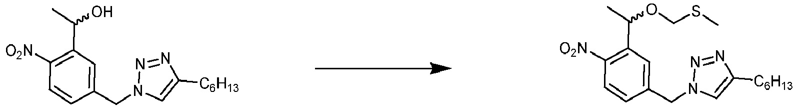

To a solution of 1-(5-(4-(hexyl)-1h-1,2,3-triazol-1-ylmethyl)-2-nitrophenyl)ethan-1-ol (0.5 g, 1.5 mmol) in dimethylsulfoxide (8 mL) was added acetic anhydride (12 mL) and glacial acetic acid (8 mL) and the reaction mixture was stirred at room temperature for 20 hours. The reaction mixture was poured into ice-cooled saturated aqueous sodium carbonate and stirred for a further 30 minutes and subsequently extracted with ethyl acetate. The combined organic layers were washed with saturated aqueous sodium carbonate, water and brine before drying over anhydrous sodium sulfate and concentrating. The crude was purified by flash chromatography (0-30% ethyl acetate in hexane), to give the title compound (0.36 g, 0.92 mmol, 61%).

¹H NMR (400 MHz, CDCl₃) 7.92 (d, 1H, *J=* 8.3 Hz, CH Ar), 7.65 (d, 1H, *J=* 2.1 Hz, CH Ar), 7.27 (br. s, 1H, CH triazole), 7.23 (dd, 1H, *J=* 8.4 Hz, 2.1 Hz, CH Ar), 5.65-5.55 (m, 2H, CH₂N), 5.41 (q, 1H, *J=* 6.3 Hz, CHCH₃), 4.6 (d, 1H, *J* = 11.5 Hz, CH₂SCH₃), 4.3 (d, 1H, *J* = 11.5 Hz, CH₂SCH₃), 2.75-2.7 (m, 2H, triazole CH₂CH₂), 2.1 (s, 3H, CH₂SCH₃), 1.72-1.62 (m, 2H, triazole CH₂CH₂), 1.52 (d, 3H, *J=* 6.4 Hz, CHCH₃), 1.40-1.27 (m, 6H, CH₂CH₂CH₂CH₃), 0.91-0.85 (m, 3H, CH₂CH₂CH₂CH₃)

### ((1-(5-(4-(Hexyl)-1h-1,2,3-triazol-1-ylmethyl)-2-nitrophenyl)ethoxy)methoxy)propan-1-ol

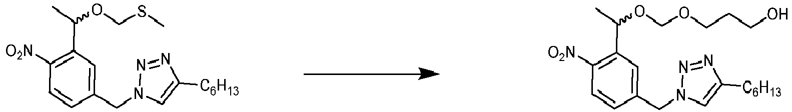

To a solution of ((1-(5-(4-(hexyl)-1h-1,2,3-triazol-1-ylmethyl)-2-nitrophenyl)ethoxy)methyl)(methyl)sulfane (0.16 g, 0.4 mmol) and 1,3-propanediol (0.57 mL, 8 mmol) in anhydrous tetrahydrofuran (2 mL) at -40 °C was added *N*-iodosuccinimide (0.09 g, 0.4 mmol) and 4Å molecular sieves, followed by triflic acid (0.035 mL, 0.4 mmol) and the mixture stirred for 30 minutes. The reaction was quenched with triethylamine and filtered through celite before concentrating in *vacuo.* The residue was dissolved in ethyl acetate and washed with saturated aqueous sodium thiosulfate. The combined organic layers were washed with saturated sodium carbonate, water and brine, drying with anhydrous sodium sulfate, filtering and concentrating in *vacuo* before purification by flash chromatography (0-100% ethyl acetate in hexane), to give the title compound (0.08 g, 0.19 mmol, 48%).

¹H NMR (400 MHz, CDCl₃) 7.92 (d, 1H, *J=* 8.4 Hz, CHAr), 7.67 (d, 1H, *J=* 1.8 Hz, CHAr), 7.34 (br. s, 1H, CH triazole), 7.22 (dd, 1H, *J=* 8.4 Hz, 2.0 Hz, CHAr), 5.65-5.57 (m, 2H, CH₂N), 5.33-5.27 (m, 1H, CHCH₃), 4.67 (d, 1H, *J=* 6.7 Hz, CH₂OCH₂), 4.55 (d, 1H, *J* = 6.7 Hz, CH₂OCH₂), 3.71-3.64 (m, 3H, CH₂CH₂CH₂OH), 3.55-3.47 (m, 1H, CH₂CH₂CH₂OH), 2.77-2.70 (m, 2H, triazole CH₂CH₂), 1.75-1.62 (m, 4H, CH₂CH₂CH₂OH, triazole CH₂CH₂), 1.52 (d, 3H, *J* = 6.4 Hz, CHCH₃), 1.39-1.24 (m, 6H, CH₂CH₂CH₂CH₃), 0.91-0.86 (m, 3H, CH₂CH₂CH₂CH₃)

### 2-Cyanoethyl (((1-(5-(4-(hexyl)-1h-1,2,3-triazol-1-ylmethyl)-2-nitrophenyl)ethoxy)methoxy)propyl) diisopropylphosphoramidite

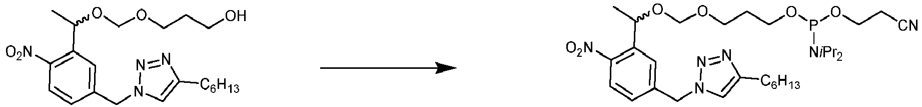

A solution of ((1-(5-(4-(hexyl)-1h-1,2,3-triazol-1-ylmethyl)-2-nitrophenyl)ethoxy)methoxy)propan-1-ol (0.16 g, 0.38 mmol) in anhydrous dichloromethane (2 mL) was degassed under argon for 5 minutes before addition of anhydrous diisopropylethylamine (0.199 mL, 1.14 mmol). 2-Cyanoethoxy-*N*,*N-*diisopropylaminochlorophosphine (0.12 mL, 0.49 mmol) was added drop-wise and the reaction stirred at rt for 1 hour. The reaction mixture was diluted with anhydrous dichloromethane (2 x 10 mL) and washed with degassed saturated potassium chloride (30 mL), passed through anhydrous sodium sulfate, concentrated and dried under high vacuum. The crude product was purified by flash chromatography (100% 0.2% pyridine in ethyl acetate) under argon to obtain the title compound (0.16 g, 0.26 mmol, 68%) as a colourless oil, which was dissolved in degassed anhydrous acetonitrile and filtered through a 0.45 µm syringe filter before concentrating and aliquoting.

¹H NMR (400 MHz, CD₃CN) δ ppm 7.92-7.88 (m, 1H, CHAr), 7.64-7.57 (m, 2H, CHAr, CH triazole), 7.34-7.28 (m, 1H, CHAr), 5.67-5.57 (m, 2H, CH₂N), 5.23 (q, 1H, *J* = 6.4 Hz, CHCH₃), 4.65-4.59 (m, 1H, CH₂OCH₂), 4.48-4.44 (m, 1H, CH₂OCH₂), 3.85-3.70 (m, 2H, 2 x CH(CH₃)₂), 3.69-3.50 (m, 5H, CH₂CH₂CN, CH₂CH₂CH₂OH), 3.41-3.32 (m, 1H,CH₂CH₂CH₂OH), 2.72-2.63 (m, 4H, CH₂CH₂CN, triazole CH₂CH₂), 1.74-1.59 (m, 4H, triazole CH₂CH₂, CH₂CH₂CH₂OH), 1.48 (d, 3H, *J=* 6.5Hz, CHCH₃), 1.38-1.27 (m, 6H, CH₂CH₂CH₂CH₃), 1.22-1.13 (m, 12H, 2 x CH(CH₃)₂), 0.94-0.86 (m, 3H, CH₂CH₂CH₂CH₃)

³¹P {¹H} NMR (162 MHz, CD₃CN) δ ppm 148.6, 148.5

### Example 3: Synthesis of 5'-PC dT

This example describes the synthesis of a compound of Formula (I) which is a compound of formula (II-2).

### 5'-O-(4,4'-Dimethoxytrityl)-3'-O-(tert-butyldimethylsilyl) thymidine

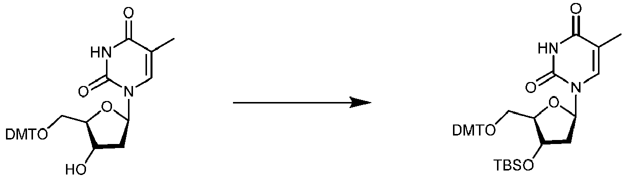

To a solution of 5'-O-(4,4'-dimethoxytrityl) thymidine (3.5 g, 6.43 mmol) and imidiazole (1.9 g, 28.2 mmol) in anhydrous dichloromethane (10 mL) was added *tert*butyldimethylsilyl chloride (4.2 g, 27.87 mmol). The reaction mixture was stirred at room temperature for 20 hours and subsequently quenched with methanol (2 mL). The solution was diluted with ethyl acetate, washed with 5% aqueous sodium bicarbonate, water and brine before drying over anhydrous sodium sulfate, filtering and concentrating. The product (3.8 g, 5.8 mmol, 90%) was taken forward without further purification.

### 3'-O-(tert-Butyldimethylsilyl) thymidine

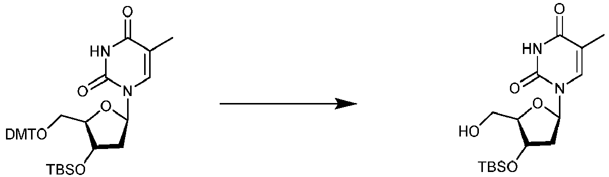

A solution of 5'-O-(4,4'-dimethoxytrityl)-3'-O-(*tert*-butyldimethylsilyl) thymidine (3.8 g, 5.8 mmol) in 80% acetic acid (45 mL) was stirred at 50 °C for 30 minutes. The reaction mixture was subsequently diluted with dichloromethane, washed with water and saturated sodium bicarbonate before drying over anhydrous sodium sulfate, filtering and concentrating. The crude was purified by flash chromatography (0-4% methanol in dichloromethane) to give the title compound (1.96 g, 5.51 mmol, 95%).

¹H NMR (400 MHz, CDCl₃) 8.59 (s, 1H, NH), 7.36 (s, 1H, CH⁶), 6.16-6.12 (m, 1H, CH^{1'}), 4.52-4.48 (m, 1H, CH^{4'}), 3.96-3.91 (m, 2H, CH^{3'}, CH^{5'}), 3.79-3.74 (m, 1H, CH^{5'}), 2.41-2.32 (m, 1H, CH^{2'}), 2.26-2.19 (m, 1H, CH^{2'}), 1.92 (d, 3H, J = 1.22 Hz, CH₃), 0.9 (s, 9H, (CH₃)₃CSi), 0.09 (s, 6H, (CH₃)₂Si)

### ((1-(5-(Azidomethyl)-2-nitrophenyl)ethoxy)methyl)(methyl)sulfane

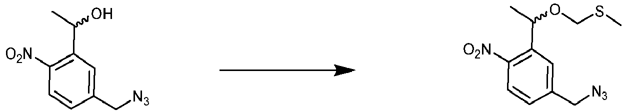

To a solution of 1-(5-(azidomethyl)-2-nitrophenyl)ethan-1-ol (1.65 g, 7.4 mmol) in dimethylsulfoxide (38 mL) was added acetic anhydride (57 mL) and glacial acetic acid (38 mL) and the reaction mixture was stirred at room temperature for 20 hours. The reaction mixture was poured into ice-cooled saturated aqueous sodium carbonate and stirred for a further 30 minutes and subsequently extracted with ethyl acetate. The combined organic layers were washed with saturated aqueous sodium carbonate, water and brine before drying over anhydrous sodium sulfate and concentrating. The crude was purified by flash chromatography (0-30% ethyl acetate in hexane), to give the title compound (1.7 g, 6 mmol, 81%).

¹H NMR (400 MHz, CDCl₃) δ ppm 7.96 (d, 1H, *J=* 8.4 Hz, CH Ar), 7.72 (d, 1H, *J* = 2.0 Hz, CH Ar), 7.39 (dd, 1H, *J* = 8.4, 2.0 Hz, CH Ar), 5.45 (q, 1H, *J=* 6.4 Hz, CH₃CH), 4.64 (d, 1H, *J=* 11.5 Hz, CH₂SCH₃), 4.49 (s, 2H, CH₂N₃), 4.35 (d, 1H, *J=* 11.5 Hz, CH₂SCH₃), 2.13 (s, 3H, CH₂SCH₃), 1.56 (d, 3H, *J* = 6.5 Hz, CH₃CH).

### 5'-O-((1-(5-(Azidomethyl)-2-nitrophenyl)ethoxy)methyl) thymidine

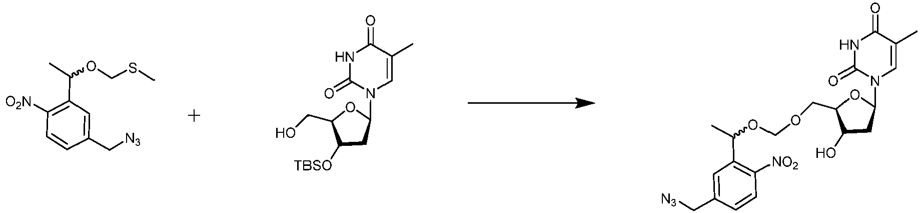

To a solution of ((1-(5-(azidomethyl)-2-nitrophenyl)ethoxy)methyl)(methyl)sulfane (0.45 g, 1.6 mmol) and 3'-O-(tert-butyldimethylsilyl) thymidine (0.54 g, 1.5 mmol) in anhydrous tetrahydrofuran (5 mL) at -40 °C was added *N*-iodosuccinimide (0.36 g, 1.6 mmol) and 4Å molecular sieves followed by triflic acid (0.14 mL, 1.6 mmol) and the mixture stirred for 30 minutes. The reaction was quenched with triethylamine and filtered through celite before concentrating in *vacuo.* The residue was dissolved in ethyl acetate and washed with saturated aqueous sodium thiosulfate. The combined organic layers were washed with saturated sodium carbonate, water and brine before drying with anhydrous sodium sulfate, filtering and concentrating in *vacuo.* The residue was dissolved in anhydrous methanol (15 mL), ammonium fluoride (0.56 g, 15.2 mmol) was added and the mixture refluxed for 2 hours before cooling to room temperature for 12 hours. The reaction mixture was concentrated in *vacuo* and purified by flash chromatography (0-100% ethyl acetate in hexane), to give the title compound (0.35 g, 0.74 mmol, 46%, mix of diastereomers).

¹H NMR (400 MHz, CDCl₃) δ ppm 8.60 (s, 1H, NH), 7.96-7.91 (m, 1H, CH Ar), 7.76-7.70 (m, 1H, CH Ar), 7.43-7.37 (m, 2H, CH Ar, CH⁶), 6.35-6.26 (m, 1H, CH^{1'}), 5.41-5.30 (m, 1H, CHCH₃), 4.86-4.81 (m, 0.5H, CH₂OCH₂), 4.76-4.65 (m, 1H, CH₂OCH₂), 4.62-4.58 (m, 0.5H, CH₂OCH₂), 4.53-4.49 (m, 2H, CH₂N₃), 4.47-4.42 (m, 0.5H, CH^{3'}), 4.17-4.12 (m, 0.5H, CH^{3'}), 4.04 (q, 1H, *J*= 3.3 Hz, CH^{4'}), 3.96-3.88 (m, 1H, CH^{4'}, CH^{5'}), 3.78-3.60 (m, 1.5H, CH^{5'}), 2.39-2.27 (m, 1H, CH^{2'}), 2.19-2.05 (m, 1H, CH^{2'}), 1.93-1.91 (m, 1.5H, CH₃), 1.85-1.83 (m, 1.5H, CH₃), 1.60-1.56 (m, 3H, CHCH₃).

### 5'-O-((1-(5-((4-(Hexyl)-1H-1,2,3-triazol-1-yl)methyl)-2-nitrophenyl)ethoxy)methyl) thymidine

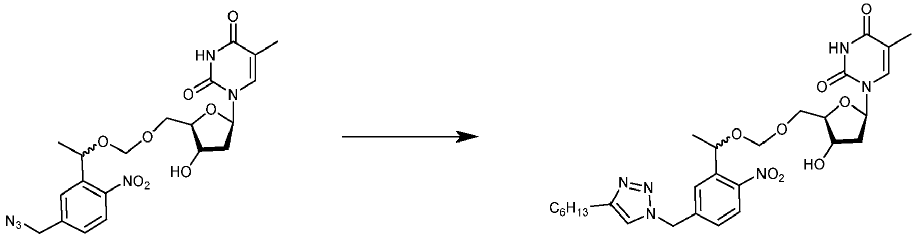

5'-O-((1-(5-(azidomethyl)-2-nitrophenyl)ethoxy)methyl) thymidine (0.35 g, 0.74 mmol) and 1-octyne (0.16 mL, 1.1 mmol) were added to a 3:1:1 mixture of tetrahydrofuran/*t*ert-butanol/water (10 mL/3.5 mL/3.5 mL). A 7.5% solution of copper(II) sulfate in water (1.63 mL) and a 1M solution of (+)-sodium L-ascorbate in water (1.85 mL) were added and the reaction stirred at room temperature for 2 hours. The mixture was diluted with ethyl acetate and the organic phase washed with saturated sodium bicarbonate and brine (diluted with water by 10%), dried over anhydrous sodium sulfate filtered and concentrated. The crude was purified by flash chromatography (0-3% methanol in (0.1% triethylamine in dichloromethane)) to give the title compound (0.27 g, 0.46 mmol, 62%, mix of diastereomers).

¹H NMR (400 MHz, CDCl₃) δ ppm 9.47-9.35 (m, 1H, NH), 7.90-7.85 (m, 1H, CH Ar), 7.65-7.61 (m, 1H, CH Ar), 7.43 (s, 0.5H, CH⁶), 7.39-7.33 (m, 1.5H, CH⁶, CH triazole), 7.29-7.22 (m, 1H, CH Ar), 6.30-6.23 (m, 1H, CH^{1'}), 5.64-5.55 (m, 2H, CH₂N), 5.27 (q, 0.5H, *J* = 6.4 Hz, CHCH₃), 5.21 (q, 0.5H, *J* = 6.3 Hz, CHCH₃), 4.79-4.76 (m, 0.5H, CH₂OCH₂), 4.70-4.66 (m, 1H, CH₂OCH₂), 4.65-4.61 (m, 0.5H, CH₂OCH₂), 4.42-4.31 (m, 1H, CH^{3'}, OH), 4.16-4.11 (m, 0.5H, OH), 3.95 (q, 0.5H, *J* = 3.1 Hz, CH^{4'}), 3.91 (q, 0.5H, *J*= 3.9 Hz, CH^{4'}), 3.84-3.78 (m, 0.5H, CH^{3'}), 3.75-3.69 (m, 0.5H, CH^{5'}), 3.67-3.59 (m, 1H, CH^{5'}), 3.53-3.47 (m, 0.5H, CH^{5'}), 2.70 (t, 2H, triazole CH₂CH₂), 2.39-2.31 (m, 0.5H, CH^{2'}), 2.28-2.20 (m, 0.5H, CH^{2'}), 2.18-2.10 (m, 0.5H, CH^{2'}), 1.99-1.91 (m, 0.5H, CH^{2'}), 1.87-1.82 (m, 3H, CH₃), 1.69-1.59 (m, 2H, triazole CH₂CH₂), 1.52-1.47 (m, 3H, CHCH₃), 1.38-1.23 (m, 6H, CH₂CH₂CH₂CH₃), 0.90-0.82 (m, 3H, CH₂CH₂CH₂CH₃)

### 5'-O-((1-(5-((4-(Hexyl)-1H-1,2,3-triazol-1-yl)methyl)-2-nitrophenyl)ethoxy)methyl)thymidine-3'-O-(2-cyanoethyldiisopropylphosphoramidite)

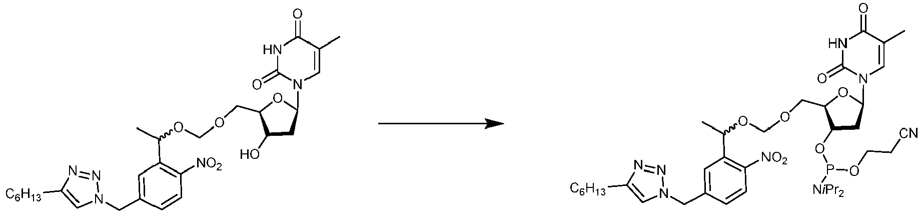

A solution of 5'-O-((1-(5-((4-(hexyl)-1H-1,2,3-triazol-1-yl)methyl)-2-nitrophenyl)ethoxy)methyl) thymidine (0.27 g, 0.46 mmol) in anhydrous dichloromethane (3 mL) was degassed under argon for 5 minutes before addition of anhydrous diisopropylethylamine (0.24 mL, 1.38 mmol). 2-Cyanoethoxy-*N*;*N-*diisopropylaminochlorophosphine (0.123 mL, 0.552 mmol) was added drop-wise and the reaction stirred at rt for 1 hour. The reaction mixture was diluted with anhydrous dichloromethane (2 x 10 mL) and washed with degassed saturated potassium chloride (30 mL), passed through anhydrous sodium sulfate, concentrated and dried under high vacuum. The crude product was purified by flash chromatography (100% 0.2% pyridine in ethyl acetate) under argon to obtain the title compound (0.25 g, 0.32 mmol, 70%, mix of diastereomers) as a colourless oil, which was dissolved in degassed anhydrous acetonitrile and filtered through a 0.45 µm syringe filter before concentrating and aliquoting.

¹H NMR (400 MHz, CDCl₃) δ ppm 8.89 (br. s, 1H, NH), 7.91-7.86 (m, 1H, CH Ar), 7.62-7.56 (m, 2H, CH Ar, CH triazole), 7.40-7.36 (s, 1H, CH⁶), 7.32-7.27 (m, 1H, CH Ar), 6.21-6.16 (m, 1H, CH^{1'}), 5.62-5.59 (m, 2H, CH₂N), 5.30-5.22 (m, 1H, CHCH₃), 4.76-4.72 (m, 0.5H, CH₂OCH₂), 4.68-4.64 (m, 0.5H, CH₂OCH₂), 4.60-4.52 (m, 1H, CH₂OCH₂), 4.48-4.40 (m, 0.5H, CH^{3'}),4.31-4.22 (m, 0.5H, CH^{3'}), 4.1-3.93 (m, 1H, CH^{4'}), 3.86-3.42 (m, 6H, 2 x CH(CH₃)₂, CH₂CH₂CN, CH^{5'}), 2.68-2.61 (m, 4H, CH₂CH₂CN, triazole CH₂CH₂), 2.35-2.09 (m, 2H, CH2'), 1.81-1.78 (m, 1.5H, CH₃), 1.81-1.78 (m, 1.5H, CH₃), 1.76-1.74 (m, 1.5H, CH₃), 1.66-1.57 (m, 2H, triazole CH₂CH₂), 1.50-1.44 (m, 3H, CHCH₃), 1.37-1.25 (m, 6H, CH₂CH₂CH₂CH₃), 1.21-1.11 (2 x CH(CH₃)₂), 0.91-0.84 (m, 3H, CH₂CH₂CH₂CH₃)

³¹P {¹H} NMR (162 MHz, CD₃CN) δ ppm 149.9, 149.8, 149.7, 149.6

### Example 4: Synthesis of 5'-PC MePhos

This example describes the synthesis of a compound of Formula (I) which is a compound of formula (II-3).

### 1-(5-Oct-1-ynyl-2-nitrophenyl)ethan-1-one

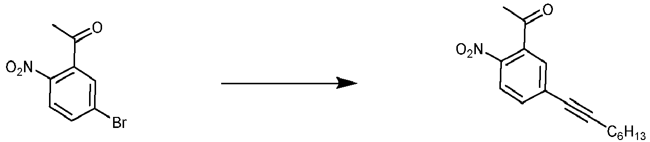

To a solution of 1-octyne (3.025 mL, 20.5 mmol), anhydrous triethylamine (30 mL, 41 mmol) in anhydrous dimethyl formamide (75 mL) was added 1-(5-bromo-2-nitrophenyl)ethenone (2.5g, 10.25 mmol) and the mixture degassed under argon for 5 minutes. Subsequently, tetrakis(triphenylphosphine)palladium(0) (1.21 g, 1.05 mmol) and copper(I) iodide (0.4 g, 2.05 mmol) were added and the reaction heated at 80 °C for 1 hour. The reaction mixture was concentrated in *vacuo* and purified by flash chromatography (0-20% ethyl acetate in hexane) to give the title compound as a colourless oil (1.2 g, 4.4 mmol, 43%).

¹H NMR (400 MHz, CDCl₃) δ ppm 8.04 (d, 1H, *J=* 8.6 Hz, CH Ar), 7.54 (dd, 1H, *J=* 8.6 Hz, 1.8 Hz, CH Ar), 7.37 (d, 1H, *J=* 1.7 Hz, CH Ar), 2.55 (s, 3H, CH₃), 2.45 (t, 2H, *J=* 7.15 Hz, CCH₂CH₂), 1.67-1.58 (m, 2H, CCH₂CH₂), 1.50-1.27 (m, 6H, CH₂CH₂CH₂CH₃), 0.94-0.89 (m, 3H, CH₂CH₂CH₂CH₃),

### 2-Methyl(5-oct-1-ynyl-2-nitrophenyl)-1,3-dioxolane

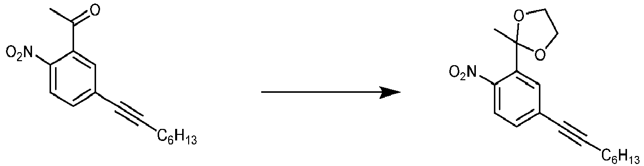

A mixture of 1-(5-oct-1-ynyl-2-nitrophenyl)ethan-1-one (1.2 g, 4.6 mmol), anhydrous ethylene glycol (3.9 mL, 23 mmol) and *p*-toluenesulfonic acid (0.045 g, 0.23 mmol) in anhydrous toluene (40 mL) was refluxed in a Dean-Stark apparatus for 48 hours. The reaction mixture was washed with saturated sodium bicarbonate, brine, dried over anhydrous sodium sulfate and concentrated. The crude was purified by flash chromatography (0-11% ethyl acetate in hexane) to give the title compound as a colourless oil (1.45 g, 4.6 mmol, 100%).

¹H NMR (400 MHz, CDCl₃) δ ppm 7.66 (d, 1H, *J* = 1.6 Hz, CH Ar), 7.41-7.33 (m, 2H, 2 x CH Ar), 4.06-3.97 (m, 2H, OCH₂CH₂O), 3.71-3.62 (m, 2H, OCH₂CH₂O), 2.55 (s, 3H, CH₃), 2.43 (t, 2H, *J* = 7.15 Hz, CCH₂CH₂), 1.86 (s, 3H, CH₃), 1.67-1.58 (m, 2H, CCH₂CH₂), 1.51-1.26 (m, 6H, CH₂CH₂CH₂CH₃), 0.95-0.88 (m, 3H, CH₂CH₂CH₂CH₃),

### 2-(1-(5-Oct-1-ynyl-2-nitrophenyl)ethoxy)ethan-1-ol

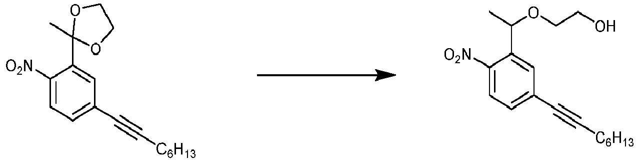

To a solution of 2-methyl(5-oct-1-ynyl-2-nitrophenyl)-1,3-dioxolane (0.85 g, 2.7 mmol) in anhydrous acetonitrile (20 mL) at 0 °C was added titanium tetrachloride (0.4 mL, 3.5 mmol) followed by sodium cyanoborohydride (0.2 g, 3.24 mmol). The subsequent yellow suspension was stirred at room temperature for 2 hours before being neutralised with saturated sodium bicarbonate, extracted with dichloromethane, dried over anhydrous sodium sulfate, filtered and concentrated. The crude was purified by flash chromatography (0-43% ethyl acetate in hexane) to give the title compound as a colourless oil (0.56 g, 1.75 mmol, 65%).

¹H NMR (400 MHz, CDCl₃) δ ppm 7.87 (d, 1H, *J=* 8.4 Hz, CH Ar), 7.74 (d, 1H, *J* = 1.8 Hz, CH Ar), 7.39 (dd, 1H, J = 8.5 Hz, 1.9 Hz, CH Ar), 5.07 (q, 1H, J = 6.3 Hz, CHCH₃), 3.78-3.71 (m, 2H, CH₂CH₂OH), 3.49-3.39 (m, 2H, CH₂CH₂OH), 2.45 (t, 2H, *J=* 7.1 Hz, CCH₂CH₂), 1.97-1.90 (m, 1H, CH₂CH₂OH), 1.67-1.56 (m, 2H, CCH₂CH₂), 1.56-1.53 (m, 3H, CHCH₃), 1.51-1.26 (m, 6H, CH₂CH₂CH₂CH₃), 0.95-0.89 (m, 3H, CH₂CH₂CH₂CH₃)

### Bis(diisopropylamino)(methyl)phosphine

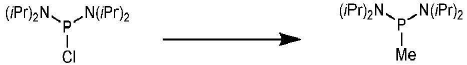

To a solution of bis(diisopropylamino)chlorophosphine (22 g, 82.5 mmol) in anhydrous diethyl ether (350 mL) at 0 °C was added drop-wise a 3 molar solution of methylmagnesium bromide in diethyl ether (33 mL) and the mixture stirred for 1 hour. The reaction mixture was warmed to room temperature and filtered under argon before concentrating. The crude was distilled under reduced pressure (70-75 °C at 0.07 mmHg) to give a colourless oil (19 g, 77 mmol, 93%).

¹H NMR (400 MHz, CDCl₃) δ ppm 3.46-3.33 (m, 4H, 4 x CH(CH₃)₂), 1.22 (s, 1.5H, PCH₃), 1.21.1.16 (m, 13.5H, PCH₃, 2 x CH(CH₃)₂), 1.09 (d, 12H, *J* = 6.7 Hz, 2 x CH(CH₃)₂)

³¹P {¹H} NMR (162 MHz, CD₃CN) δ ppm 39.7

### 2-(1-(5-Oct-1-ynyl-2-nitrophenyl)ethoxy)ethoxy[(diisopropylamino)(methyl)phosphine]

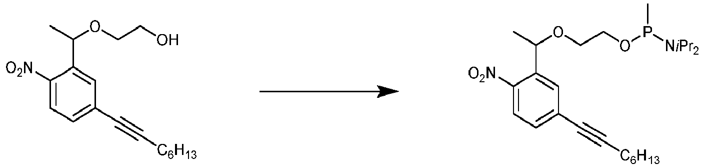

To a solution of 2-(1-(5-Oct-1-ynyl-2-nitrophenyl)ethoxy)ethan-1-ol (0.12 g, 0.38 mmol) and 4,5-dicyanoimidazole (0.04 g, 0.34 mmol) in degassed anhydrous dichloromethane (5 mL) was added dropwise a mixture of bis(diisopropylamino)(methyl)phosphine (0.128 g, 0.38 mmol) in degassed anhydrous dichloromethane (2 mL) and the mixture stirred for 40 minutes. The reaction was neutralised with a small amount of anhydrous triethylamine and whole mixture purified by flash chromatography (25% ethyl acetate in (0.2% triethylamine in hexane) under argon to obtain the title compound (0.13 g, 0.28 mmol, 74%) as a colourless oil.

¹H NMR (400 MHz, CDCl₃) δ ppm 7.91-7.86 (m, 1H, CH Ar), 7.80-7.77 (m, 1H, CH Ar), 7.42 (dd, 1H, CH Ar), 5.04-4.97 (m, 1H, CHCH₃), 3.71-3.41 (m, 5H, CH₂CH₂OP, CH(CH₃)₂), 3.36-3.24 (m, 1H, CH(CH₃)₂), 2.45 (t, 2H, *J* = 7.1 Hz, CCH₂CH₂), 1.64-1.56 (m, 2H, CCH₂CH₂), 1.50-1.30 (m, 9H, PCH₃, CH₂CH₂CH₂CH₃), 1.20-1.06 (m, 12H, 2 x CH(CH₃)₂), 0.93-0.88 (m, 3H, CH₂CH₂CH₂CH₃)

³¹P {¹H} NMR (162 MHz, CD₃CN) δ ppm 122.9, 122.2

### Example 5: Oligonucleotide synthesis and purification methods

Standard DNA phosphoramidites, solid supports and reagents were purchased from Link Technologies and Applied Biosystems. Small scale automated solid phase synthesis of oligonucleotides was performed on a K&A H-8 SE DNA/RNA synthesiser. Synthesis was performed on 0.2 µmole or 1.0 µmole scale involving cycles of acid-catalyzed detritylation, coupling, capping, and iodine oxidation. Standard DNA phosphoramidites were coupled for 60's. Coupling efficiencies and overall synthesis yields were determined by the inbuilt automated trityl cation conductivity monitoring facility and were ≥98.0% in all cases. The oligonucleotides were treated with 20% diethylamine then cleaved from the solid support on an Applied Biosystems 394 synthesiser with concentrated ammonium hydroxide for 60 minutes at room temperature followed by heating in a sealed tube for 5 h at 55° C to remove protecting groups from the nucleobase and backbone.

Large scale automated solid phase synthesis of oligonucleotides was performed on an Akta oligopilot synthesiser. Synthesis was performed on 15 µmole or 30 µmole scale involving cycles of acid-catalyzed detritylation, coupling, capping, and iodine oxidation. Standard DNA phosphoramidites were coupled for 60's. Coupling efficiencies and overall synthesis yields were determined by the inbuilt automated trityl cation conductivity monitoring facility and were ≥98.0% in all cases. The oligonucleotides were treated with 20% diethylamine for 10 minutes then cleaved from the solid support manually with concentrated ammonium hydroxide by heating in a sealed tube for 5 h at 55° C.

Methyl phosphonamidites were coupled for 10 minutes. Oligonucleotides containing methyl phosphonates were cleaved and deprotected by treatment with a solution of 45:45:10 acetonitrile/ethanol/concentrated ammonium hydroxide for 30 minutes at room temperature followed by addition of an equal amount of ethylenediamine for 6 hours.

RPHPLC purification of oligonucleotides was performed using a Gilson HPLC system on a Luna 10 µm C8(2) 100 Å LC column with pH 7.5 0.1M triethylammonium acetate (TEAA) buffer and desalted using pre-packed sephadex G-25 columns.

Mass spectra of oligonucleotides were recorded using a XEVO G2-QTOF MS instrument in ES⁻ mode. UV absorbance was performed on an Agilent Technologies Cary 60 UV-Vis in water at 260 nm.

UV cleavage was performed using a handheld Analytik Jena UVP UVGL-25 4W UV lamp positioned roughly 2 cm from the sample. Irradiation at 365 nm for 50 minutes to a sample in milli-Q water resulted in near quantitative cleavage. Low molecular weight cleavage products were separated from oligonucleotides using pre-packed sephadex G-25 columns or by organic extraction. Methyl phosphonates were further treated with concentrated ammonium hydroxide at room temperature for 2 hours.

Ultra-performance liquid chromatography-mass spectrometry (UPLC-MS) analysis of oligonucleotides was performed on a Waters Acquity H Class system coupled to a Waters Xevo G2-XS QToF mass spectrometer. Chromatography was carried out on a Waters Acquity Premier BEH C18 oligonucleotide column 2.1 x 100 mm at 60°C (1.7 µm particle size, 130 Å) at a flow rate of 0.25 mL/min using a gradient of buffers A and B: buffer A, 400 mM 1,1,1,3,3,3-hexafluoroisopropanol, 15 mM triethylamine); buffer B, 50% 400 mM 1,1,1,3,3,3-hexafluoroisopropanol, 15 mM triethylamine 50% methanol. The eluent was directly injected into the mass spectrometer, and data acquired in the negative-ion mode. This data was analysed and deconvoluted using the MaxEnt1 algorithm (UNIFI, Waters).

Capillary electrophoresis (CE) analysis of oligonucleotides was performed on a Sciex P/ACE MDQ Plus using a ssDNA 100-R Kit.

### Example 6: Modification and purification of oligonucleotides using the compound of example 2

A 92mer oligonucleotide mixed sequence was synthesised under standard conditions on a 15 µmol scale, with the terminal 5' addition of spacer C3 phosphoramidite replaced with 5'-PC C3 (2-Cyanoethyl (((1-(5-(4-(hexyl)-1h-1,2,3-triazol-1-ylmethyl)-2-nitrophenyl)ethoxy)methoxy)propyl) diisopropylphosphoramidite). After cleavage and deprotection the crude oligonucleotide was analysed by mass spectrometry. Mass calculated 26846.3. Mass found 26848.1.

1 µmol of crude oligonucleotide was purified by RPHPLC using pH 7.5 0.1M TEAA on a Luna 10 µm C8(2) 100 Å LC column with a 30 minute 20-60% gradient. Collected oligonucleotide was desalted using pre-packed sephadex G-25 columns. A sample was analysed by mass spectrometry. Mass calculated 26846.3. Mass found 26847.6.

The purified oligonucleotide was taken up in 1 mL of milli-Q water and irradiated at 365 nm for 50 minutes before passing through a pre-packed sephadex G-25 column. A sample was analysed by mass spectrometry. Mass calculated 26501.9. Mass found 26503.1. Photocleavage proceeded quantitatively.

The oligonucleotide was repurified by RPHPLC using pH 7.5 0.1M TEAA on a Luna 10 µm C8(2) 100 Å LC column with a 20 minute 5-45% gradient. Collected oligonucleotide was desalted using pre-packed sephadex G-25 columns. A sample was analysed by mass spectrometry. Mass calculated 26501.9. Mass found 26502.9.

A sample was analysed for purity by UPLC-MS (Figure 1A) and was found to be 90.05% pure.

For comparison, an identical 92 mer oligonucleotide was synthesised under standard conditions on a 1 µmol scale, with the terminal 5' addition replaced with standard spacer C3 phosphoramidite.

The crude oligonucleotide was purified by RPHPLC using pH 7.5 0.1M TEAA on a Luna 10 µm C8(2) 100 Å LC column with a 30 minute 20-60% gradient. Collected oligonucleotide was desalted using pre-packed sephadex G-25 columns.

The oligonucleotide was repurified by RPHPLC using pH 7.5 0.1M TEAA on a Luna 10 µm C8(2) 100 Å LC column with a 20 minute 5-45% gradient. Collected oligonucleotide was desalted using pre-packed sephadex G-25 columns.

A sample was analysed for purity by UPLC-MS (Figure 1B) and was found to be 62.31% pure.

This example shows that the compound of formula (I) can be incorporated into a polynucleotide; can be used to improve purification e.g. by HPCL; and can be successfully photocleaved to yield the desired product.

### Example 7: Modification and purification of oligonucleotides using the compound of example 1

Two oligonucleotides were synthesised with and without terminal 5'-PC PO₃ (the compound of example 1; i.e. 2-cyanoethyl (1-(5-((4-hexyl-1H-1,2,3-triazol-1-yl)methyl)-2-nitrophenyl)ethyl) diisopropylphosphoramidite) modifications under standard conditions on a 1 µmol scale. Sequences are shown below. SEQ ID NO: 3 corresponds to SEQ ID NO: 1 with additional 5' PC PO3. SEQ ID NO: 4 corresponds to SEQ ID NO: 2 with additional 5' PC PO3.
SEQ ID NO: 1: TTGCAGCTCCTTCTCTTGTTCCGTGGAGCAAGCCTTCTTAA
SEQ ID NO: 2: GCCTATTGTAGTGCGGAAGAGAATCGGTCTAAGCTTCCTAATTC
SEQ ID NO: 3: XTTGCAGCTCCTTCTCTTGTTCCGTGGAGCAAGCCTTCTTAA
SEQ ID NO: 4: XGCCTATTGTAGTGCGGAAGAGAATCGGTCTAAGCTTCCTAATTC [X = 5'-PC PO₃]

After cleavage and deprotection the oligonucleotides were purified by RPHPLC using pH 7.5 0.1M TEAA on a Luna 10 µm C8(2) 100 Å LC column, with Seq. 1 and Seq. 2 using a 20 minute 10-40% gradient and Seq. 3 and Seq. 4 using a 20 minute 15-60% gradient. Collected oligonucleotide was desalted using pre-packed sephadex G-25 columns. A sample was analysed by mass spectrometry.
SEQ ID NO: 1: Mass calculated 12484.1, mass found 12484.4
SEQ ID NO: 2: Mass calculated 13561.8, mass found 13562.1
SEQ ID NO: 3: Mass calculated 12878.5, mass found 12878.7
SEQ ID NO: 4: Mass calculated 13956.2, mass count 13956.7

The purified oligonucleotides SEQ ID NO: 3 and SEQ ID NO: 4 were taken up in 1 mL of milli-Q water and irradiated at 365 nm for 50 minutes before passing through a pre-packed sephadex G-25 column.. A sample was analysed by mass spectrometry. Photocleavage proceeded quantitatively.
SEQ ID NO: 3: Mass calculated 12562.1, mass found 12564.5
SEQ ID NO: 4: Mass calculated 13639.8, mass found 13642.4

The oligonucleotides were analysed by CE for purity. Results are shown in Figure 2 (A: SEQ ID NO: 1; B: SEQ ID NO: 2; C: SEQ ID NO: 3; D: SEQ ID NO: 4) and calculated purity levels are summarised in the table below:

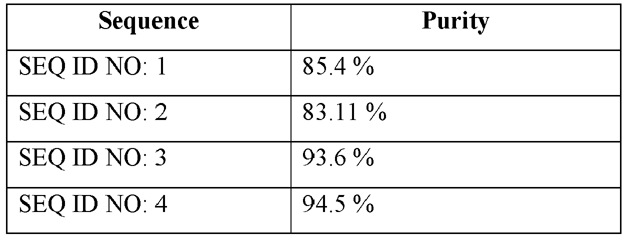

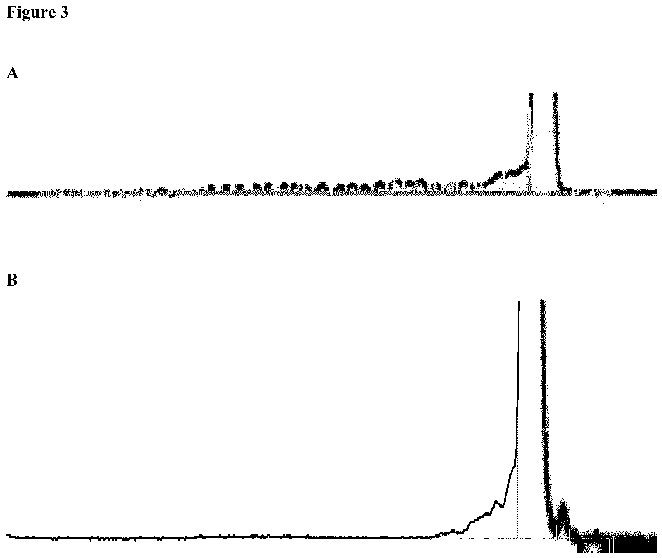

As summarised above and visible in Figure 2, oligonucleotides synthesised with 5'-PC PO₃ showed a significantly higher purity with fewer truncated sequences than those synthesised traditionally. Figure 3 compares expanded regions of the electropheregrams in Figure 2 for SEQ ID NO: 2 (A) vs SEQ ID NO: 4 (B). As can clearly be seen, the trace for SEQ ID NO: 4 is far cleaner than the trace for SEQ ID NO: 2 indicating fewer impurities.

This example shows that the compound of formula (I) can be incorporated into a polynucleotide; can be used to improve purification e.g. by HPCL; and can be successfully photocleaved to yield the desired product; and that the purity of the resulting polynucleotide is higher than can be achieved using conventional methods.

### Example 8: Modification and purification of oligonucleotides using the compound of example 3

An oligonucleotide was synthesised with a terminal 5'-PC dT (the compound of example 3; i.e. 5'-O-((1-(5-((4-(Hexyl)-1H-1,2,3-triazol-1-yl)methyl)-2-nitrophenyl)ethoxy)methyl)thymidine-3'-O-(2-cyanoethyldiisopropylphosphoramidite)) modification under standard conditions on a 1 µmol scale.
SEQ ID NO: 5: XTAATACGACTCACTATAG
X = 5'-PC dT]

After cleavage and deprotection the oligonucleotide was purified by RPHPLC using pH 7.5 0.1M TEAA on a Luna 10 µm C8(2) 100 Å LC column, using a 20 minute 5-45% gradient. Collected oligonucleotide was desalted using pre-packed sephadex G-25 columns. A sample was analysed by mass spectrometry. Mass calculated 6115.2, mass found 6116.0.

The purified oligonucleotide was taken up in 1 mL of milli-Q water and irradiated at 365 nm for 50 minutes before passing through a pre-packed sephadex G-25 column.. A sample was analysed by mass spectrometry. Mass calculated 5770.8. Mass found 5770.3 Photocleavage proceeded quantitatively.

This example shows that the compound of formula (I) can be incorporated into a polynucleotide and can be successfully photocleaved to yield the desired product.

### Example 9: Modification and purification of oligonucleotides using the compound of example 4

An oligonucleotide was synthesised with a terminal 5'-PC MePhos (the compound of example 4; i.e. 2-(1-(5-Oct-1-ynyl-2-nitrophenyl)ethoxy)ethoxy[(diisopropylamino)(methyl)phosphine]) modification under standard conditions on a 1 µmol scale.
SEQ ID NO: 6: XTTTTTTTTTT
X = 5'-PC MePhos]

After cleavage and deprotection the oligonucleotide were purified by RPHPLC using pH 7.5 0.1M TEAA on a Luna 10 µm C8(2) 100 Å LC column, using a 20 minute 5-45% gradient. Collected oligonucleotide was desalted using pre-packed sephadex G-25 columns. A sample was analysed by mass spectrometry. Mass calculated 3360.14, mass found 3363.3.

The purified oligonucleotide was taken up in 1 mL of milli-Q water and irradiated at 365 nm for 50 minutes before passing through a pre-packed sephadex G-25 column.. A sample was analysed by mass spectrometry. Mass calculated 3103.0. Mass found 3102.2 Photocleavage proceeded quantitatively to give the 5'-hydroxyethyl methylphosphonate oligonucleotide.

The oligonucleotide was hydrolyised with concentrated ammonium hydroxide for 2 hours at room temperature, concentrated and desalted using a pre-packed sephadex G-25 column. A sample was analysed by mass spectrometry. Mass calculated 2979.98, mass found 2980.3. Hydrolysis proceeded quantitatively.

This example shows that the compound of formula (I) can be incorporated into a polynucleotide and can be successfully photocleaved to yield the desired product.

## Claims

1. A method for modifying a polynucleotide or an analogue or derivative thereof, comprising reacting the polynucleotide or analogue or derivative thereof with a compound of formula (I): 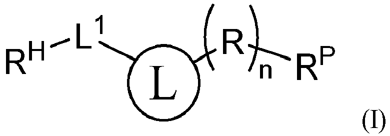 wherein:
- R^{H} is a hydrophobic group;
- L¹ is a linking group;
- Ⓛ is a photolabile group;
- R is a modifying group;
- n is an integer selected from 0 and 1; and
- R^{P} is a phosphorous-based group;
under conditions such that a reactive functional group of the polynucleotide or analogue or derivative thereof reacts with the phosphorous-based group R^{P}, thereby connecting the hydrophobic group R^{H} to the polynucleotide or analogue or derivative thereof.

2. The method of claim 1, comprising:
(i) reacting a free hydroxyl group at the 5' position of the polynucleotide or analogue or derivative thereof with the phosphorous-based group R^{P}; or
(ii) reacting a free hydroxyl group at the 3' position of the polynucleotide or analogue or derivative thereof with the phosphorous-based group R^{P}.

3. The method of claim 1 or claim 2, wherein:
- R^{H} is selected from C₁ to C₂₀ alkyl, C₂ to C₂₀ alkenyl, C₂ to C₂₀ alkynyl, C₅ to C₁₀ carbocyclyl, C₆ to C₁₈ aryl, 5- to 10-membered heteroaryl and 5- to 10-membered heterocyclyl, wherein R^{H} is optionally substituted; and/or
- L¹ is selected from: (i) a chemical bond; and (ii) a linker comprising a C₁ to C₂₀ alkylene group, a C₂ to C₂₀ alkenylene group and/or a C₂ to C₂₀ alkynylene group, wherein said alkylene, alkenylene or alkynylene group is optionally interrupted by and/or terminated in one or more groups selected from a heteroatom, a phosphite group, a phosphate group, a carbonyl group, a C₆ to C₁₀ aryl group, a C₅ to C₁₀ carbocyclyl group, a 5- to 10-membered heteroaryl group and a 5- to 10-membered saturated or partially unsaturated heterocyclic group, wherein the linker is optionally further substituted; and/or
- R^{P} is a phosphoramidite, a phosphoramidate, an alkyl phosphonamidite or an alkyl phosphonamidate; and/or
- R is selected from:
(i) a nucleoside or a derivative or analogue thereof;
(ii) a group comprising a C₁ to C₂₀ alkylene group, a C₂ to C₂₀ alkenylene group and/or a C₂ to C₂₀ alkynylene group, wherein said alkylene, alkenylene or alkynylene group is optionally interrupted by and/or terminated in one or more groups selected from: a heteroatom; a phosphite group; a phosphate group; a carbonyl group; a C₆ to C₁₀ aryl group; a C₅ to C₁₀ carbocyclyl group; a 5- to 10-membered heteroaryl group; and a 5- to 10-membered heterocyclic group; and wherein R is optionally further substituted; and
(iii) a second polynucleotide or a derivative or analogue thereof.

4. The method of any one of the preceding claims, wherein:
- R^{H} is selected from C₄ to C₁₆ alkyl, C₄ to C₁₆ alkenyl, C₄ to C₁₆ alkynyl, C₅ to C₁₀ carbocyclyl and C₆ to C₁₀ aryl, preferably C₅ to C₁₂ alkyl; wherein R^{H} is optionally substituted and is preferably unsubstituted; and/or
- L¹ is a linker comprising a C₁ to C₆ alkylene group, a C₂ to C₆ alkenylene group and/or a C₂ to C₆ alkynylene group, wherein said alkylene, alkenylene or alkynylene group is optionally interrupted by and/or terminated in one or more groups selected from a heteroatom, a carbonyl group, a phenyl group, a cyclopentyl or cyclohexyl group, a 5- to 6-membered heteroaryl group and a 5-to 6-membered saturated or partially unsaturated heterocyclic group, wherein the linker is optionally further substituted; and/or
- R^{P} is a phosphoramidite or an alkyl phosphonamidite, preferably a phosphoramidite or a methyl phosphonamidite, more preferably 2-cyanoethyl N,N-diisopropyl phosphoramidite or N,N-diisopropyl methylphosphonamidite; and/or
- R is selected from:
(i) a nucleoside or a derivative or analogue thereof;
(ii) a group comprising a C₂ to C₆ alkylene group, a C₂ to C₆ alkenylene group and/or a C₂ to C₆ alkynylene group, wherein said alkylene, alkenylene or alkynylene group is optionally interrupted by and/or terminated in one or more groups selected from a heteroatom, a carbonyl group, a phenyl group, a cyclopentyl or cyclohexyl group, a 5- to 6-membered heteroaryl group and a 5-to 6-membered saturated or partially unsaturated heterocyclic group, wherein R is optionally further substituted; and
(iii) a second polynucleotide or a derivative or analogue thereof.

5. The method of any one of the preceding claims, wherein the photolabile group Ⓛ is a moiety of formula -Ⓐ-CHR³-W-, such that the compound of formula (I) is a compound of formula (II): 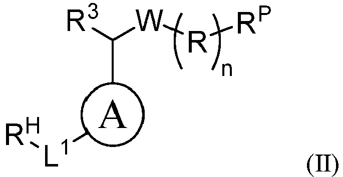 wherein R^{H}, L¹, and R are as defined in any one of claims 1 to 4, and wherein:
- Ⓐ is a 2-nitrobenzyl group, wherein said 2-nitrobenzyl group is optionally substituted with 1, 2 or 3 groups independently selected from halogen, optionally substituted C₁ to C4 alkyl, -OR^{a}, -SR^{a}, -NR^{a}R^{a}, -C(O)OR^{a}, - C(O)NR^{a}R^{a}, -C(O)R^{b}, -OC(O)R^{b} and -NHC(O)R^{b}, wherein each R^{a} is independently selected from hydrogen, optionally substituted C₁ to C₂ alkyl and optionally substituted C₁ to C₂ alkoxyl and each R^{b} is independently selected from hydrogen and an optionally substituted C₁ to C₄ alkyl group;
- R³ is methyl, ethyl or C₁ to C₂ haloalkyl; and
- W is selected from a group of formula (W-2); an oxygen atom; and a group of formula (W-1): 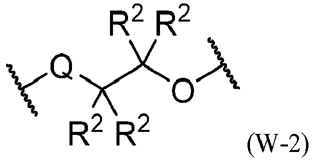 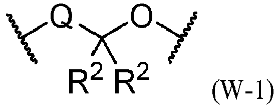 wherein:
- when n is 1, R^{P} is a phosphoramidite, a phosphoramidate, an alkyl phosphonamidite or an alkyl phosphonamidate; and when n is 0 R^{P} together with the oxygen atom to which it is attached forms a phosphoramidite, a phosphoramidate, an alkyl phosphonamidite or an alkyl phosphonamidate;
wherein said phosphoramidite, a phosphoramidate, an alkyl phosphonamidite or an alkyl phosphonamidate is preferably a phosphoramidite or a methyl phosphonamidite, more preferably 2-cyanoethyl N,N-diisopropyl phosphoramidite or N,N-diisopropyl methylphosphonamidite;
- Q is an oxygen atom or a sulphur atom; and
- each R² is independently selected from hydrogen, methyl, ethyl, C₁ to C₂ haloalkyl and a halogen group.

6. The method of claim 5, wherein W is a group of formula (W-2) and n is 0, such that the compound of formula (II) is a compound of formula (II-3): 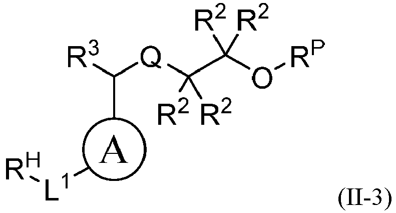 wherein R^{H}, L¹, Ⓐ, R², R³ and Q are as defined in any one of claims 1 to 5 and R^{P} together with the oxygen atom to which it is attached forms an alkyl phosphonamidite, preferably a methyl phosphonamidite, and more preferably N,N-diisopropyl methylphosphonamidite.

7. The method of claim 5, wherein W is an oxygen atom and n is 0, such that the compound of formula (II) is a compound of formula (II-1): 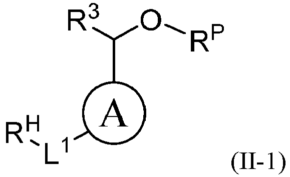 wherein R^{H}, L¹, Ⓐ, and R³ are as defined in any one of claims 1 to 5, and wherein R^{P} together with the oxygen atom to which it is attached forms a phosphoramidite, a phosphoramidate, an alkyl phosphonamidite or an alkyl phosphonamidate; preferably a phosphoramidite, more preferably 2-cyanoethyl N,N-diisopropyl phosphoramidite.

8. The method of claim 5, wherein W is a group of formula (W-1) and n is 1, such that the compound of formula (II) is a compound of formula (II-2): 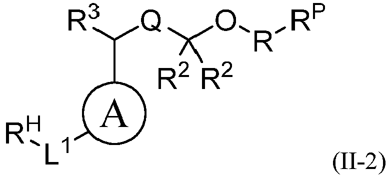 wherein R^{H}, L¹, Ⓐ, R², R³, Q, and R are as defined in any one of claims 1 to 5, and wherein R^{P} is a phosphoramidite, a phosphoramidate, an alkyl phosphonamidite or an alkyl phosphonamidate; preferably a phosphoramidite, more preferably 2-cyanoethyl N,N-diisopropyl phosphoramidite.

9. The method of any one of claims 5 to 8, wherein Ⓐ, L¹ and R^{H} are together represented by formula (A-1) or formula (A-2): 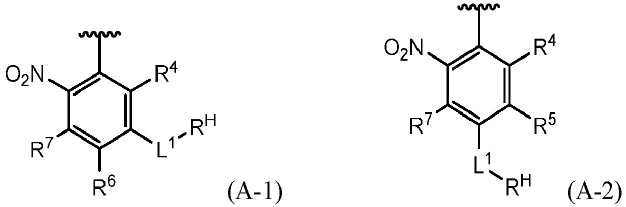 wherein L¹ and R^{H} are as defined in any one of claims 1 to 4, and wherein:
- R⁴, R⁵, R⁶ and R⁷ are each independently selected from hydrogen, halogen, optionally substituted C₁ to C4 alkyl, -OR^{a}, -SR^{a}, -NR^{a}R^{a}, -C(O)OR^{a}, -C(O)NR^{a}R^{a}, -C(O)R^{b}, -OC(O)R^{b} and -NHC(O)R^{b}, preferably R⁴, R⁵, R⁶ and R⁷ are each independently selected from hydrogen, fluorine, optionally substituted C₁ to C4 alkyl, -OR^{a}, -SR^{a}, -NR^{a}R^{a}, -OC(O)R^{b} and -NHC(O)R^{b}, more preferably R⁴, R⁵, R⁶ and R⁷ are each hydrogen or methoxy; wherein R^{a} and R^{b} are as defined in claim 5.

10. A method for purifying a polynucleotide or an analogue or derivative thereof, comprising:
(i) modifying a polynucleotide or analogue or derivative thereof in a reaction mixture according to the method of any one of claims 1 to 9, thereby increasing the hydrophobicity of the polynucleotide or analogue or derivative thereof;
(ii) separating the modified polynucleotide or analogue or derivative thereof from other components in the reaction mixture according to the hydrophobicity of the modified polynucleotide or analogue or derivative thereof; and
(iii) optionally removing the hydrophobic group from the modified polynucleotide or analogue or derivative thereof by photo-illuminating the modified polynucleotide, analogue or derivative thereof, preferably by photo-illuminating the modified polynucleotide, analogue or derivative thereof using UV light at a wavelength of about 300 to about 500 nm.

11. The method of claim 10, wherein step (ii) comprises separating the modified polynucleotide or analogue or derivative thereof from other components in the reaction mixture using a chromatographic purification technique, preferably using high performance liquid chromatography, more preferably using reverse-phase high performance liquid chromatography.

12. A compound of formula (I): 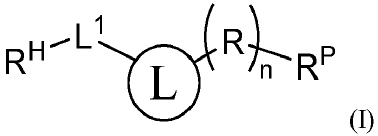 wherein R^{H}, L¹, Ⓛ, R, n and R^{P} are as defined in any one of claims 1 to 9.

13. A modified polynucleotide or a derivative or analogue thereof, comprising a group of formula (I^{∗}): 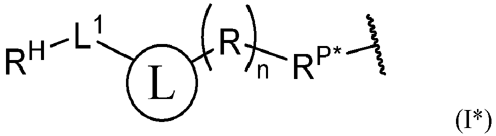 wherein R^{H}, L¹, Ⓛ, R and n are as defined in any one of claims 1 to 9, and wherein:
- R^{P∗} is a phosphorous-based linkage; and
- the wavy line indicates the point of attachment to the polynucleotide or derivative or analogue thereof.

14. The modified polynucleotide or derivative or analogue thereof of claim 13, wherein:
- R^{P∗} is a phosphodiester linkage, a phosphotriester linkage, a phosphite-triester linkage, a phosphite-diester linkage, a phosphorothioate linkage, a phosphorodithioate linkage, an alkylphosphonate linkage, or an alkylphosphonite linkage, preferably a phosphite-triester linkage or an alkylphosphonite linkage, and more preferably a phosphite-triester linkage or a methylphosphonite linkage; and/or
- the group of formula (I^{∗}) is attached at the 3' position or the 5' position of the polynucleotide or analogue or derivative thereof, preferably at the 5' position of the polynucleotide or analogue or derivative thereof.

15. Use of a compound of formula (I) as defined in any one of claims 1 to 9, for: (i) modifying a polynucleotide or a derivative or analogue thereof, optionally thereby increasing the hydrophobicity of the polynucleotide or a derivative or analogue thereof; or (ii) purifying a polynucleotide or a derivative or analogue thereof.

16. A polynucleotide or a derivative or analogue thereof, obtainable by a method as defined in any one of claims 1 to 11.
